# EUROPEAN PATENT SPECIFICATION

(11) **EP 4 142 775 B1**
(45) Date of publication and mention of the grant of the patent: **25.03.2026**
(21) Application number: 21723704.9
(22) Date of filing: 03.05.2021
(51) Int. Cl.: A61K 38/55, A61P 31/12

(54) **TREATMENT AND/OR PREVENTION OF A DISEASE OR A SYNDROME RELATED TO A VIRUS INFECTION**
BEHANDLUNG UND/ODER PRÄVENTION EINER ERKRANKUNG ODER EINES SYNDROMS IM ZUSAMMENHANG MIT EINER VIRUSINFEKTION
TRAITEMENT ET/OU PRÉVENTION D'UNE MALADIE OU D'UN SYNDROME LIÉ À UNE INFECTION VIRALE

(30) Priority: 01.05.2020 EP 20172564; 20.08.2020 EP 20192016
(43) Date of publication of application: 08.03.2023
(73) Proprietor: Ageronix SA, 1227 Carouge GE (CH)
(72) Inventor: ZHUKOVSKY, Nikolay, 1292 Chambesy (CH)
(74) Representative: Vossius & Partner Patentanwälte Rechtsanwälte mbB
(86) International application number: PCT/EP2021/061597
(87) International publication number: WO 2021/219896

(56) References cited:
- ZHOU XUEYUAN ET AL: "Alpha-1-antitrypsin interacts with gp41 to block HIV-1 entry into CD4+ T lymphocytes", BMC MICROBIOLOGY, vol. 16, no. 1, 1 December 2016 (2016-12-01), XP055826486, Retrieved from the Internet <URL:https://bmcmicrobiol.biomedcentral.com/track/pdf/10.1186/s12866-016-0751-2.pdf> DOI: 10.1186/s12866-016-0751-2
- SABINA JANCIAUSKIENE ET AL: "Well-Known and Less Well-Known Functions of Alpha-1 Antitrypsin. Its Role in Chronic Obstructive Pulmonary Disease and Other Disease Developments", ANNALS OF THE AMERICAN THORACIC SOCIETY, vol. 13, no. Supplement_4, 1 August 2016 (2016-08-01), pages S280 - S288, XP055552391, ISSN: 2329-6933, DOI: 10.1513/AnnalsATS.201507-468KV
- HOFFMANN MARKUS ET AL: "SARS-CoV-2 Cell Entry Depends on ACE2 and TMPRSS2 and Is Blocked by a Clinically Proven Protease Inhibitor", CELL, ELSEVIER, AMSTERDAM NL, vol. 181, no. 2, 5 March 2020 (2020-03-05), pages 271, XP086136225, ISSN: 0092-8674, [retrieved on 20200305], DOI: 10.1016/J.CELL.2020.02.052

## Description

### FIELD OF THE INVENTION

The present invention provides compositions and methods for the treatment and/or prevention of disease or syndrome caused by a coronavirus infection. The invention further relates to methods for determining the susceptibility of a subject for such a treatment as well as a method for determining the amount of the composition required for an effective treatment.

### BACKGROUND OF THE INVENTION

Viruses, such as for example Coronaviruses, cause diseases in animals and humans around the world. Coronaviruses are RNA viruses.

Human coronaviruses (HCoV) are mainly known to cause infections of the upper and lower respiratory tract. Examples for human coronaviruses are; a beta coronavirus that causes Middle East Respiratory Syndrome (named MERS-CoV), a beta coronavirus that causes severe acute respiratory syndrome (named SARS-CoV, or SARS-CoV-1), a novel coronavirus that causes coronavirus disease 2019 or COVID-19 (named SARS-CoV-2), alpha coronavirus 299E, alpha coronavirus NL63, beta coronavirus OC43, and beta coronavirus HKU1.

The disease or syndrome caused by a SARS-CoV-2 infection is also referred to as COVID-19. A SARS-CoV-2 infection can be either asymptomatic or lead to a disease or syndrome related to mild or severe symptoms. The most common symptoms of a disease or syndrome related to a SARS-CoV-2 (also referred to as SARS-CoV-19) infection are fever and cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste. Further symptoms include lack of appetite, loss of weight, stomach pain, conjunctivitis, skin rash, lymphoma, apathy, and somnolence.

Patients with severe symptoms can develop pneumonia. A significant number of patients with pneumonia require passive oxygen therapy. Non-invasive ventilation and high-flow nasal oxygen therapy can be applied in mild and moderate non-hypercapnia pneumonia cases. A lung-saving ventilation strategy must be implemented in severe acute respiratory syndrome or acute respiratory distress syndrome (SARS/ARDS) and mechanically ventilated patients.

While the principal complication of coronavirus disease 2019 (COVID-19) is respiratory failure, a considerable number of patients have been reported with neurological symptoms affecting both the peripheral and central nervous systems (Niazkar, Zibaee et al. 2020 Neurol Sci 41(7): 1667-1671; Nordvig, Fong et al. 2021, Neurol Clin Pract 11(2): e135-e146). The hematogenic pathway, retro-/antero-grade transport along peripheral nerves as well as rare direct invasion, are considered as potential neuroinvasion mechanisms of neurotropic virus including SARS-CoV-2 (Barrantes 2021 Brain Behav Immun Health 14: 100251; Tavcar, Potokar et al. 2021, Front Cell Neurosci 15: 662578). Severe cases of SARS-CoV-2 often exhibit disproportionate and abnormal inflammatory responses, including systemic upregulation of cytokines, chemokines, and pro-inflammatory cues (Najjar, Najjar et al. 2020, J Neuroinflammation 17(1): 231). Such systemic hyper-inflammation could impair the neurovascular endothelial function, damage the blood brain barrier ultimately activating CNS immune system and contributing to CNS complications (Amruta, Chastain et al., 2021, Cytokine Growth Factor Rev 58: 1-15). Despite the attention that the COVID-19 pandemic has recently taken, several other viruses are associated with major brain disorders like Alzheimer's, Parkinson's and multiple sclerosis' disease. The diseases or syndromes related to virus infections further include a wide range of diseases or syndromes such as inflammatory diseases and are a major burden for society. Therefore, there is a need to improve the treatment and/or prevention of diseases or syndromes related to viruses.

The above technical problem is solved by the aspects disclosed herein and as defined in the claims.

### SUMMARY OF THE INVENTION

The present invention provides a composition for use in the treatment and/or prevention of disease or syndrome caused by a coronavirus infection, preferably a SARS-CoV-2 infection in a subject in need thereof, the composition comprising a therapeutically effective amount of an Alpha1-Antitrypsin protein, a variant, an isoform and/or a fragment thereof as defined in the claims. The coronavirus infection is preferably a SARS-CoV-2 infection. The subject in need of a treatment and/or prevention of a disease or syndrome caused by a coronavirus infection, preferably a SARS-CoV-2 infection may have at least i) a lower level of endogenous alpha-antitrypsin (AAT), ii) a higher level of at least one spike protein priming protease, iii) a higher level of an angiotensin converting enzyme 2 (ACE2) receptor, and/or iv) a higher level of interferon-gamma (IFN-γ) compared to at least one subject who is asymptomatic or has mild symptoms during a a coronavirus infection, preferably a SARS-CoV-2 infection.

The invention also relates to a method for determining the susceptibility of a subject of interest for treatment and/or prevention of a disease or syndrome caused by a coronavirus infection, more preferably a SARS-CoV-2 infection using the composition for use of the present invention as defined in the claims.

The invention further provides a method for determining the therapeutically effective amount of alphal-antitrypsin (AAT) for an effective treatment and/or prevention of a disease or syndrome caused by a coronavirus infection, preferably a SARS-CoV-2 infection using the composition for use of the present invention as defined in the claims.

Disclosed is a method of treatment and/or prevention of a disease or syndrome related to a virus infection, preferably a coronavirus infection, more preferably a SARS-CoV-2 infection in a subject in need thereof, the method comprising administering a therapeutically effective amount of an Alphal -Antitrypsin protein, a variant, an isoform and/or a fragment thereof. The alphal-antitrypsin (AAT) protein, a variant, an isoform and/or a fragment thereof can be a plasma-extracted AAT, a variant, an isoform and/or a fragment thereof, in particular a human plasma-extracted AAT, a variant, an isoform and/or a fragment thereof; or a recombinant alphal-antitrypsin (rhAAT) protein, a variant, an isoform and/or a fragment thereof, preferably the alphal-antitrypsin (AAT) protein, a variant, an isoform and/or a fragment thereof is a recombinant alphal-antitrypsin (rhAAT) protein, a variant, an isoform and/or a fragment thereof.

### DESCRIPTION OF THE INVENTION

Although methods and materials similar or equivalent to those described herein can be used in the practice or testing of the present invention, suitable methods and materials are described below.

The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof", or "patient"/"patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult, child and newborn subjects, whether male or female, are intended to be covered. Preferably, the subject is a human. Most preferably a human suffering from a disease or syndrome related to a virus infection, preferably a coronavirus infection, more preferably a SARS-CoV-2 infection.

As used herein, the terms "peptide", "protein", "polypeptide", "polypeptidic" and "peptidic" are used interchangeably to designate a series of amino acid residues connected to the other by peptide bonds between the alpha-amino and carboxy groups of adjacent residues.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) of the invention and, preferably, is designed for transfer between different host cells. The terms "expression vector", "gene delivery vector" and "gene therapy vector" refer to any vector that is effective to incorporate and express one or more nucleic acid(s) of the invention, in a cell, preferably under the regulation of a promoter. A cloning or expression vector may comprise additional elements, for example, regulatory and/or post-transcriptional regulatory elements in addition to a promoter.

The term "about," particularly in reference to a given quantity, is meant to encompass deviations of plus or minus ten (10) percent, preferably 5 percent, even more preferably 2 percent and most preferably 1 percent.

The present invention relates to a composition for use in the treatment and/or prevention of disease or syndrome related to any virus infection in a subject in need thereof, the composition comprising a therapeutically effective amount of an Alpha1-Antitrypsin protein, a variant, an isoform and/or a fragment thereof.

The alphal-antitrypsin (AAT) protein, a variant, an isoform and/or a fragment thereof can be a plasma-extracted AAT, a variant, an isoform and/or a fragment thereof, in particular a human plasma-extracted AAT, a variant, an isoform and/or a fragment thereof; or a recombinant alphal-antitrypsin (rhAAT) protein, a variant, an isoform and/or a fragment thereof, preferably the alpha1-antitrypsin (AAT) protein, a variant, an isoform and/or a fragment thereof is a recombinant alphal-antitrypsin (rhAAT) protein, a variant, an isoform and/or a fragment thereof. The virus infection can be due to a DNA virus (double or single stranded), an RNA virus (single or double stranded, whether positive of negative), a reverse transcribing virus or any emerging virus, whether enveloped or non-enveloped.

In some aspects of the invention, the composition is used in the treatment and/or prevention of disease or syndrome related to a respiratory virus infection in a subject in need thereof. In some aspects, the respiratory virus described herein is a virus selected from the group of Rhinovirus, RSV, Parainfluenza, Metapneumovirus, Coronavirus, Enterovirus, Adenovirus, Bocavirus, Polyomavirus, Herpes simplex virus, and Cytomegalovirus.

In some aspects of the invention, the composition is used in the treatment and/or prevention of disease or syndrome related to a DNA virus infection in a subject in need thereof.

In some aspects, the DNA virus described herein is selected from the group consisting of Adenovirus, Rhinovirus, RSV, Influenza virus, Parainfluenza virus, Metapneumovirus, Coronavirus, Enterovirus, Adenovirus, Bocavirus, Polyomavirus, Herpes simplex virus, Cytomegalovirus, Bocavirus, Polyomavirus, and Cytomegalovirus.

In some aspects of the invention, the composition is used in the treatment and/or prevention of disease or syndrome related to an RNA virus infection in a subject in need thereof. The RNA virus may be an enveloped or coated virus or a nonenveloped or naked RNA virus. The RNA virus may be single stranded RNA (ssRNA) virus or a double stranded RNA (dsRNA) virus. The single stranded RNA virus may be a positive sense ssRNA virus or a negative sense ssRNA virus.

In some aspects, the RNA virus described herein is selected from the group consisting of Rhinovirus, RSV, Influenza virus, Parainfluenza virus, Metapneumovirus, Coronavirus, Enterovirus Adenovirus, Bocavirus, Polyomavirus, Herpes simplex virus, and Cytomegalovirus. In some aspects of the invention, the composition is used in the treatment and/or prevention of disease or syndrome related to an Coronavirus infection in a subject in need thereof. In some aspects, the Coronavirus described herein is a Coronavirus from the genus selected from the group of α-CoV, β-CoV, γ-CoV or δ-CoV. In another specific aspect, the Coronavirus described herein is of the genus α-CoV or β-CoV. In some aspects, the Coronavirus described herein is selected from the group consisting of Human coronavirus OC43 (HCoV-OC43), Human coronavirus HKU1 (HCoV- HKU1), Human coronavirus 229E (HCoV-229E), Human coronavirus NL63 (HCoV-NL63, New Haven corona virus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019"). Preferably, the virus infection is an RNA virus infection, most preferably a coronavirus infection due to a coronavirus selected from the non-limiting group comprising MERS-CoV, SARS-CoV and SARS-CoV-2. Most preferably the virus infection is a SARS-CoV-2 infection.

It is to be understood that the disclosure includes all disease or syndrome related a virus infection, preferably a coronavirus infection, more preferably a SARS-CoV-2 infection. The disease or syndrome related to a SARS-CoV-2 - infection is also referred to as COVID-19. In one aspect, the disease or syndrome related a virus infection is an inflammation, e.g. of blood vessels throughout the body (e.g. Kawasaki disease), an immune disease (e.g. Grave's disease) and/or a respiratory syndrome, in particular a severe acute respiratory syndrome. The disease or syndrome can be related a virus infection in that the virus infection is associated with the disease or syndrome, prior to the disease or syndrome, contributes to the disease or syndrome and/or causes the disease or syndrome. For example the virus infection may induce inflammation that directly or indirectly induces a disease or syndrome. The inflammation related to the virus infection may be acute or chronic inflammation. The inflammation related to the virus infection may subsequently persist systemically and/or in a specific organ e.g. the brain and/or induce lasting damages. In some aspects of the invention, the composition is used in the treatment and/or prevention of an inflammatory disease or syndrome of the nervous system related to a virus infection in a subject in need thereof.

The term "inflammatory disease or syndrome of the nervous system", as used herein, refers to a disease, a syndrome and/or a condition that is characterized by increased inflammation in the nervous system compared to a healthy reference subject. The disease or syndrome described herein including disease or syndrome of the nervous system is related to a virus. Inflammation is characterized by a dysregulation of inflammation markers and/or increased immune cell infiltration, activation, proliferation, and/or differentiation in the blood and/or the brain. An inflammation marker is a marker that is indicative for inflammation in a subject. In certain aspects the inflammatory marker described herein is a marker selected from the group of CRP, erythrocyte sedimentation rate (ESR), and procalcitonin (PCT), Interleukin (e.g., IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-33, IL-32, IL-33, IL-35 or IL-36) Tumor necrosis factor (e.g., TNF alpha, TNF beta) , Interferon (e.g., interferon gamma) MIP-I, MCP-I, RANTES, other chemokines and/or other cytokines. An inflammatory marker may also be detectable indirectly, e.g., by detection of an inhibitory factor of an inflammatory marker (e.g., binding factor and/or antagonist). In some aspects, the inflammatory marker is measured in cells involved in inflammation, in cells affected by cells involved in inflammation, in the cerebrospinal fluid, and/or in the blood. In some aspects, the inflammation marker is indicative for immune cell infiltration, activation, proliferation and/or differentiation. Detection of the inflammation marker or the ratio of two or more inflammation markers is detected outside the normal range. The normal range of inflammation markers and whether a marker(ratio) has to be below or above a threshold to be indicative for inflammation is known to the person skilled in the art. In some aspects, the gene expression level, the RNA transcript level, the protein expression level, the protein activity level and/or the enzymatic activity level of at least one inflammation marker is detected. In some aspects at least one inflammation marker is detected quantitatively and/or qualitatively to determine the inflammatory disease or syndrome of the nervous system in a subject in need of treatment and/or prevention.

In some aspects, the inflammatory disease or syndrome of the nervous system described herein is characterized by acute inflammation, that is the duration of inflammation symptoms typically takes from about a few minutes (e.g., 2, 5, 10, 15, 30, 45 minutes) to a few days (e.g., 2, 3, 5, 7, 10 or 14 days). Acute inflammation typically occurs as a direct result of a stimulus such as virus infection. In some aspects, the inflammatory disease or syndrome of the nervous system is characterized by chronic inflammation, that is the duration of symptoms of inflammation typically take at least about a few days (e.g., 2, 3, 5, 7, 10 or 14 days) or the symptoms of inflammation reoccur at least once (e.g., once or more times, twice or more times or three or more times). In some aspects, the inflammatory disease or syndrome of the nervous system is characterized by chronic low-grade inflammation. Chronic low-grade inflammation can occur in the absence of clinical symptoms.

In certain aspects, the subject in need of treatment and/or prevention has a history of a virus infection. Therefore the subject in need of treatment and/or prevention was infected with the virus at least once. In certain aspects the subject in need of treatment and/or prevention was infected with the virus at least once. In certain aspects the subject in need of treatment and/or prevention was infected with the virus at least once during childhood. In certain aspects the subject in need of treatment and/or prevention was infected with the virus at least once. In certain aspects the subject in need of treatment and/or prevention was infected with the virus at least once during the last 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, or 0.5 year(s). In certain aspects, the virus infection is active (e.g. detectable) at the timepoint of diagnosis of the inflammatory disease or syndrome of the nervous system in the subject in need of treatment and/or prevention.

Methods for detecting virus infections are known to the person skilled in the art. In some aspects, the invention relates to a method for detecting a virus selected from the group of virus isolation, nucleic acid based methods, microscopy based methods, host antibody detection, electron microscopy and host cell phenotype.

In some aspects, the virus infection described herein is detected in a sample such as in a sample selected from the group of nasopharyngeal swab, blood, tissue (e.g. skin), sputum, gargles, bronchial washings, urine, semen, faeces, cerebrospinal fluid, dried blood spots, nasal mucus. In some aspects, the virus infection described herein is obtained as an information retrieved from the patient history.

Examples for detection of a (previous) SARS-CoV-2 infection include Human IFN-γ SARS-CoV-2 ELISpot^{PLUS} kit (ALP), strips (Mabtech, 3420-4AST-P1-1) or determination of a T-cell response (Zuo, J., Dowell, A.C., Pearce, H. et al., 2021, Nat Immunol).

In some aspects, the inflammatory disease or syndrome of the nervous system described herein is a inflammatory disease or syndrome of the sympathetic nervous system. In some aspects, the inflammatory disease or syndrome of the nervous system described herein is a inflammatory disease or syndrome of the parasympathetic nervous system. In some aspects, the inflammatory disease or syndrome of the nervous system described herein is a inflammatory disease or syndrome of the central nervous system. In some aspects, the inflammatory disease or syndrome of the nervous system described herein is a inflammatory disease or syndrome of the peripheral nervous system.

In certain aspects, the inflammatory disease or syndrome of the nervous system related to a virus is selected from the group of multiple sclerosis, amyotrophic lateral sclerosis, Alzheimer's disease, Parkinson's disease and Huntington's disease.

The term "multiple sclerosis", as used herein, refers to a disease or disorder characterized by inflammation, demyelination, oligodendrocyte death, membrane damage and axonal death. In some aspects, the multiple sclerosis described herein refers to relapsing/remitting multiple sclerosis or progressive multiple sclerosis. In some aspects, multiple sclerosis is at least one of the four main multiple sclerosis varieties as defined in an international survey of neurologists (Lublin and Reingold, 1996, Neurology 46(4):907-11), which are namely, relapsing/remitting multiple sclerosis, secondary progressive multiple sclerosis, progressive/relapsing multiple sclerosis, or primary progressive multiple sclerosis (PPMS).

In some aspects, the multiple sclerosis described herein refers to symptoms of multiple sclerosis which comprise vision problems, dizziness, vertigo, sensory dysfunction, weakness, problems with coordination, loss of balance, fatigue, pain, neurocognitive deficits, mental health deficits, bladder dysfunction, bowel dysfunction, sexual dysfunction, heat sensitivity.

The term "Huntington's disease", as used herein, refers to a neurodegenerative disease caused by a tri-nucleotide repeat expansion (e.g., CAG, which is translated into a poly-Glutamine, or PolyQ, tract) in the HTT gene that results in production of pathogenic mutant huntingtin protein (HTT, or mHTT). In some aspects, mutant huntingtin protein accelerates the rate of neuronal cell death in certain regions of the brain. In some aspects, the Huntington's disease described herein refers to symptoms of Huntington's disease which comprise impaired motorfunction, cognitive impairment, depression, anxiety, movement disturbances, chorea, rigidity, muscle contracture (dystonia), slow eye movements or abnormal eye movements, impaired gait, altered posture, impaired balance, unintended weight loss, sleep rhythm disturbances, circadian rhythm disturbances and autonomic nervous system dysfunction.

The term "amyotrophic lateral sclerosis", as used herein, refers to a progressive neurodegenerative disease that affects upper motor neurons (motor neurons in the brain) and/or lower motor neurons (motor neurons in the spinal cord) and results in motor neuron death. In some aspects, amyotrophic lateral sclerosis includes all of the classifications of amyotrophic lateral sclerosis known in the art, including, but not limited to classical amyotrophic lateral sclerosis (typically affecting both lower and upper motor neurons), Primary Lateral Sclerosis (PLS, typically affecting only the upper motor neurons), Progressive Bulbar Palsy (PBP or Bulbar Onset, a version of amyotrophic lateral sclerosis that typically begins with difficulties swallowing, chewing and speaking), Progressive Muscular Atrophy (PMA, typically affecting only the lower motor neurons) and familial amyotrophic lateral sclerosis (a genetic version of amyotrophic lateral sclerosis).

In some aspects, the term "amyotrophic lateral sclerosis" refers to symptoms of amyotrophic lateral sclerosis, which include, without limitation, progressive weakness, atrophy, fasciculation, hyperreflexia, dysarthria, dysphagia and/or paralysis of respiratory function. The term "Alzheimer's disease" (AD), as used herein, refers to mental deterioration associated with a specific degenerative brain disease that is characterized by senile plaques, neuritic tangles and progressive neuronal loss which manifests clinically in progressive memory deficits, confusion, behavioral problems, inability to care for oneself and/or gradual physical deterioration.

In some aspects, subjects suffering Alzheimer's disease are identified using the NINCDS-ADRDA (National Institute of Neurological and Communicative Disorders and the Alzheimer's Disease and Related Disorders Association) criteria:
1) Clinical Dementia Rating (CDR) = 1; Mini Mental State Examination (MMSE) between 16 and 24 points and Medial temporal atrophy (determined by Magnetic Resonance Imaging, MRI) >3 points in Scheltens scale. In some aspects, the term Alzheimer's disease includes all the stages of the disease, including the following stages defined by NINCDS-ADRDA Alzheimer's Criteria for diagnosis in 1984.
2) Definite Alzheimer's disease: The patient meets the criteria for probable Alzheimer's disease and has histopathologic evidence of AD via autopsy or biopsy.
   Probable or prodromal Alzheimer's disease: Dementia has been established by clinical and neuropsychological examination. Cognitive impairments also have to be progressive and be present in two or more areas of cognition. The onset of the deficits has been between the ages of 40 and 90 years and finally there must be an absence of other diseases capable of producing a dementia syndrome.
3) Possible or non-prodromal Alzheimer's disease: There is a dementia syndrome with an atypical onset, presentation; and without a known etiology; but no co-morbid diseases capable of producing dementia are believed to be in the origin of it. In some aspects, the term Alzheimer's disease refers one stage of Alzheimer's disease. In some aspects, the term Alzheimer's disease refers to two stages of Alzheimer's disease. In some aspects, the term "Alzheimer's disease" refers to symptoms of Alzheimer's disease, which include without limitation, loss of memory, confusion, difficulty thinking, changes in language, changes in behavior, and/or changes in personality.

The term "Parkinson's disease", as used herein, refers to a neurological syndrome characterized by a dopamine deficiency, resulting from degenerative, vascular, or inflammatory changes in the basal ganglia of the substantia nigra. Symptoms of Parkinson's disease include, without limitation, the following: rest tremor, cogwheel rigidity, bradykinesia, postural reflex impairment, good response to 1-dopa treatment, the absence of prominent oculomotor palsy, cerebellar or pyramidal signs, amyotrophy, dyspraxia, and/or dysphasia. In a specific aspect, the present invention is utilized for the treatment of a dopaminergic dysfunction-related syndrome. In some aspects, Parkinson's disease includes any stage of Parkinson's disease. In some aspects, the term Parkinson's disease includes the early stage of Parkinson's disease, which refers broadly to the first stages in Parkinson's disease, wherein a person suffering from the disease exhibits mild symptoms that are not disabling, such as an episodic tremor of a single limb (e.g., the hand), and which affect only one side of the body.

In some aspects, the term Parkinson's disease includes the advanced stage of Parkinson's disease, which refers to a more progressive stage in Parkinson's disease, wherein a person suffering from the disease exhibits symptoms which are typically severe and which may lead to some disability (e.g., tremors encompassing both sides of the body, balance problems, etc.). Symptoms associated with advanced-stage Parkinson's disease may vary significantly in individuals and may take several years to manifest after the initial appearance of the disease. In some apects, the term "Parkinson's disease" refers to symptoms of Parkinson's disease, which include without limitation, tremors (e.g., tremor which is most pronounced during rest), shaking (e.g. trembling of hands, arms, legs, jaw and face), muscular rigidity, lack of postural reflexes, slowing of the voluntary movements, retropulsion, mask-like facial expression, stooped posture, poor balance, poor coordination, bradykinesia, postural instability, and/or gait abnormalities.

Examples of established links of inflammatory disease or syndrome of the nervous system and virus infections:

| Brain disorder | Virus involved | Virus family | Reference |
|---|---|---|---|
| Parkinson's Disease (PD) | Hepatitis C | Flaviviridae | Tsai, Liou et al. 2016 Neurology 86(9): 840-846 |
| | H5N1 | Orthomyxoviridae | Jang, Boltz et al. 2012 J Neurosci 32(5): 1545-1559 |
| | SARS-CoV-2 | Coronaviridae | (Sulzer, Antonini et al. 2020 NPJ Parkinsons Dis 6: 18.) |
| | HIV | Retroviridae | Tse, Cersosimo et al. 2004 Parkinsonism Relat Disord 10(6): 323-334. |
| Alzheimer's Disease (AD) | Herpes | Herpesviridae | Abbott 2020 Nature 587(7832): 22-25. |
| Multiple Sclerosis (MS) | Epstein-Barr | Herpesviridae | Soldan and Lieberman, 2020, Drug Discov Today Dis Models 32(Pt A): 35-52 |
| | Herpes | Herpesviridae | Virtanen and Jacobson, 2012, CNS Neurol Disord Drug Targets 11(5): 528-544. |

The disease or syndrome is preferably related to a coronavirus infection, and more preferably the disease or syndrome is related to a SARS-CoV-2 infection. The disease of syndrome related to a SARS-CoV-2 disease or syndrome is preferably at least one selected form the group consisting of fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste, lack of appetite, loss of weight, stomach pain, conjunctivits, skin rash, lymphoma, apathy, and somnolence, preferably fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste.

The present invention relates to a composition for use in the treatment and/or prevention of disease or syndrome caused by a coronavirus infection in a subject in need thereof, the composition comprising a therapeutically effective amount of an Alpha1-Antitrypsin (AAT) protein, a variant, an isoform and/or a fragment thereof as defined in the claims. The coronavirus is preferably SARS-CoV-2.

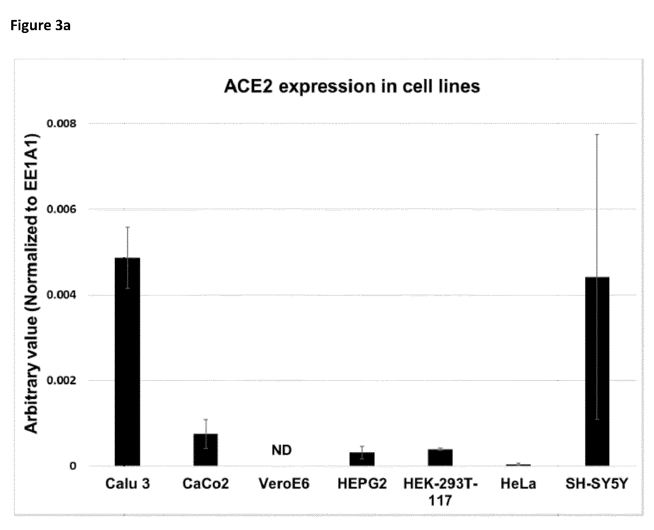

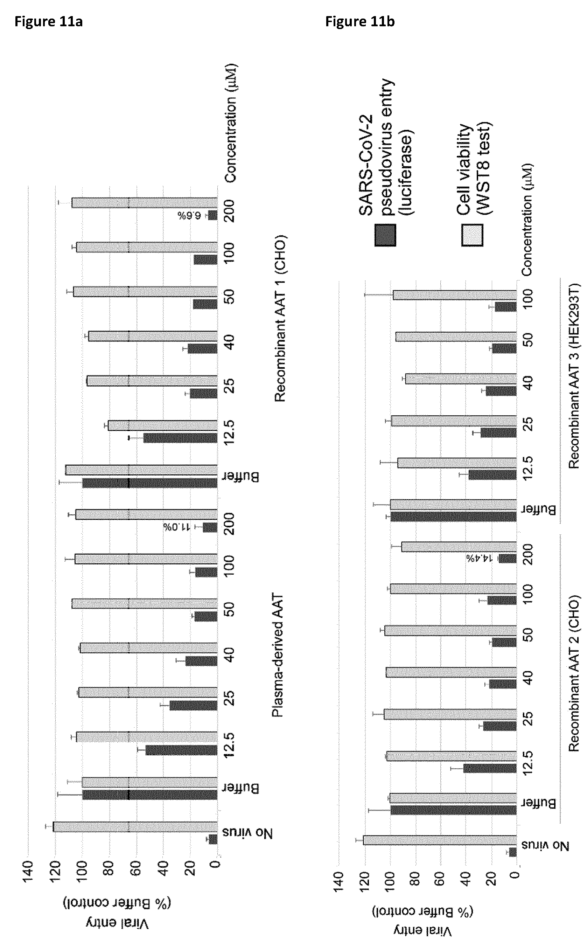

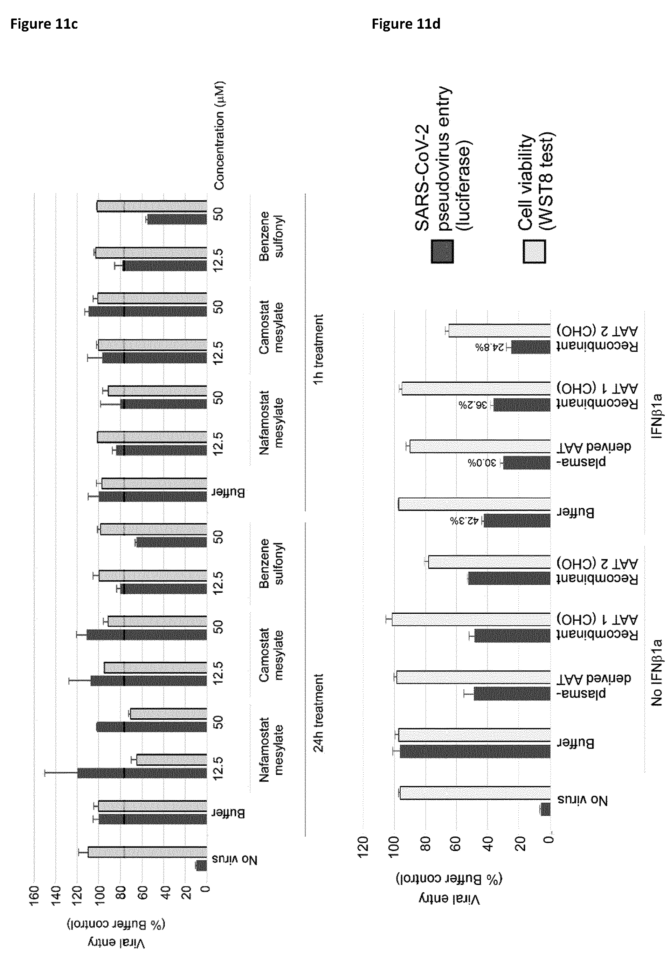

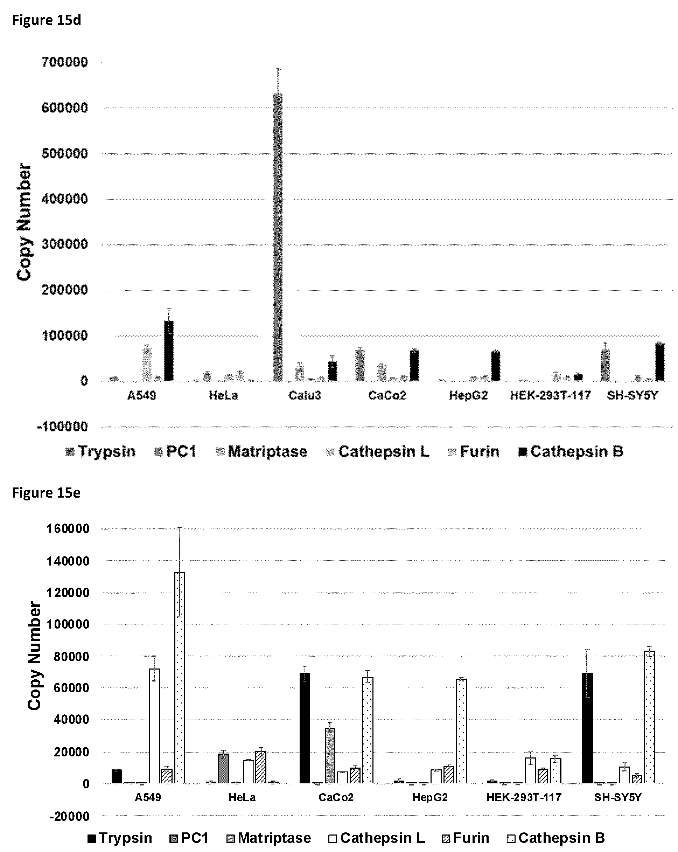

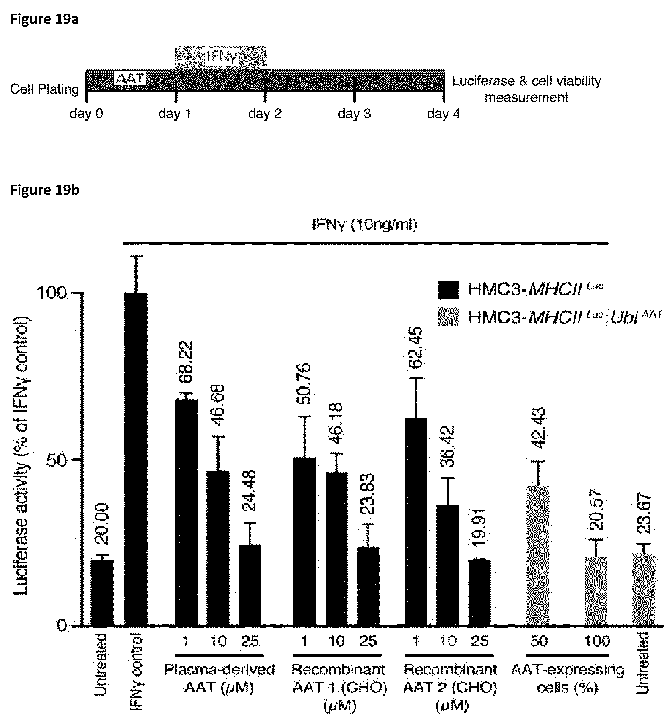

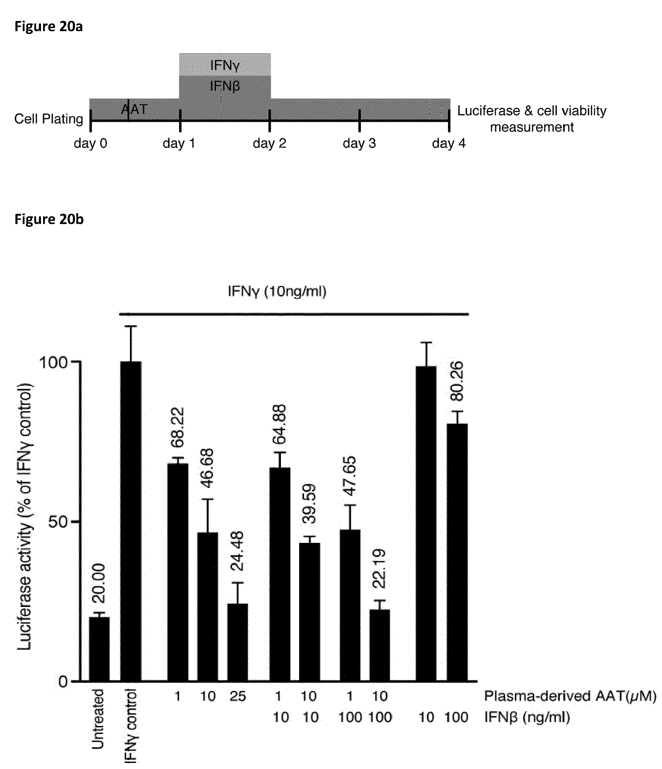

The inventor(s) found that AAT and rhAAT inhibits viral entry of several viruses (see Figure 11, 21, 22) and reduces inflammation, in particular in microglia of the nervous system (Figure 19, 20). Furthermore, SH-SY5Y cells that are of neuronal origin and are frequently used to study neurodegenerative disease, including Parkinsons's disease (Xicoy, H., Wieringa, B. & Martens, G.J. The SH-SY5Y cell line in Parkinson's disease research: a systematic review. Mol Neurodegeneration 12, 10, 2017) display a realtively high copy number of spike protein priming proteases mRNA, namely trypsin and cathepsin B (Figure 15e). Coupled with a relatively high level of ACE2 expression in SH-SY5Y (Figure 3a), these neural tissue (Bielarz V, Willemart K, Avalosse N, et al. Susceptibility of neuroblastoma and glioblastoma cell lines to SARS-CoV-2 infection. Brain Res. 2021 May 1) and cells of similar origin, becomesusceptible to SARS-CoV-2 viral infection..

Various strategies can be used to deliver the composition for use of the invention to the nervous system, including the brain and to pass the blood brain barrier (Salameh, T. S., & Banks, W. A., 2014, Advances in pharmacology, 71, 277-299; Tashima, T., 2020, Receptor-Mediated Transcytosis. Chemical and Pharmaceutical Bulletin, 68(4), 316-32; Pardridge, W. M., 2020, Frontiers in aging neuroscience, 11, 373; Upadhyay, R. K., 2014, BioMed research international).

In some aspects, the composition for use of the invention is fused to a blood-brain barrier enhancing protein. In some aspects the blood-brain barrier enhancing protein described herein is at least one full protein, variant, isoform and/or fragment of the protein selected from the group of transferrin, insulin, insulin-like growth factor, low density lipoprotein.

Trojan horse strategies may also be used (see e.g. Pardridge, W.M., 2017, BioDrugs 31, 503-519). In some aspects, the composition for use of the invention is linked to an antibody or a fragment thereof that binds to an endogenous BBB receptor transporter, such as the insulin receptor or transferrin receptor.

The composition for use of the invention may also be altered to increase lipophilicity and subsequently improve BBB crossing properties (see e.g. Upadhyay, R. K., 2014,. BioMed research international, Article ID 869269, 37 pages). In some aspects, the composition for use of the invention comprises modifications that increase the lipophilicity. In some aspects the modifications that increase the lipophilicity described herein comprise the addition of at least one hypdrophilic peptide, replacement of sequence parts with at least one hypdrophilic peptide, the addition of lipid moieties, and/or replacement of non-lipid moieties with lipid moieties. Accordingly, the invention is at least in part based on the broad effect of AAT on diseases or syndromes related to virus infections.

Particular subjects can be particularly suitableto treatment and/or prevention with AAT and/or rhAAT protein.

Therefore, the present invention also relates a composition for use according to the present invention, wherein the subject in need thereof has at least one altered level selected from i) endogenous alpha-antitrypsin (AAT), at least one spike protein priming protease, angiotensin converting enzyme 2 (ACE2 receptor) and interferon-gamma (IFN-γ) compared to at least one reference subject. The alphal-antitrypsin (AAT) protein, a variant, an isoform and/or a fragment thereof can be a plasma-extracted AAT, a variant, an isoform and/or a fragment thereof, in particular a human plasma-extracted AAT, a variant, an isoform and/or a fragment thereof; or a recombinant alphal-antitrypsin (rhAAT) protein, a variant, an isoform and/or a fragment thereof, preferably the alphal-antitrypsin (AAT) protein, a variant, an isoform and/or a fragment thereof is a recombinant alpha1-antitrypsin (rhAAT) protein, a variant, an isoform and/or a fragment thereof.

The "subject in need thereof" is also referred to as a subject of interest. The subject in need thereof is a subject can be a subject during or with a virus infection, preferably a coronavirus infection, more preferably a SARS-CoV-2 infection (i.e. an infected subject) having a disease or syndrome related to a virus infection, preferably a coronavirus infection, more preferably a SARS-CoV-2 infection. An infected subject can require treatment and/or prevention of a disease or syndrome related to a virus infection, preferably a coronavirus infection, more preferably a SARS-CoV-2 infection. The treatment with AAT and/or rhAAT can inhibit or reduce the entry of the virus infection, preferably the coronavirus infection, more preferably the SARS-CoV-2 virus into the cells by inhibiting the spike protein priming protease, reduce the propagation of the virus infection, preferably the coronavirus infection, more preferably the SARS-CoV-2 virus in the body and/or reduce inflammation as a response to the virus infection, preferably the coronavirus infection, more preferably the SARS-CoV-2 infection. A subject in need of a treatment and/or prevention can have a respiratory syndrome, more preferably an acute respiratory syndrome, even more preferably a severe acute respiratory syndrome. The subject in need of a treatment and/or prevention may have at least one symptom selected from the group of consisting of fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste, lack of appetite, loss of weight, stomach pain, conjunctivits, skin rash, lymphoma, apathy, and somnolence, preferably from the group consisting of fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste. A subject in need of a treatment and/or prevention may require intensive care and/or artificial ventilation. A subject in need of a treatment and/or prevention can be defined by one of the above definitions or any combination thereof.

A subject in need thereof can also be a subject prior to a virus infection, preferably a coronavirus infection, more preferably a SARS-CoV-2 infection, which is particularly susceptible to delevop a disease or syndrome after a virus infection, preferably a coronavirus infection, more preferably a SARS-CoV-2 infection. Such a subject may require prevention of a disease or syndrome related to a virus infection, preferably a coronavirus infection, more preferably a SARS-CoV-2 infection prior to infection.

The at least one reference subject can be a group of reference subjects. Preferably, the reference (reference) subject(s) is/are a subjects with or during a virus infection, preferably a coronavirus infection, more preferably a SARS-CoV-2 infection (i.e. an infected subject) which is/are asymptomatic or has/have mild symptoms, more preferably subject(s) is/are subjects with or during a virus infection, preferably a coronavirus infection, more preferably a SARS-CoV-2 infection, which is/are asymptomatic.

Asymptomatic means that the (reference) subject has no symptoms, preferably has no symptoms selected from the group of consisting of no fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste, lack of appetite, loss of weight, stomach pain, conjunctivits, skin rash, lymphoma, apathy, and somnolence, more preferably has no fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste. A asymptomatic (reference) subject preferably have no respiratory syndrome, more preferably no acute respiratory syndrome, even more preferably no severe acute respiratory syndrome. A asymptomatic (reference) subject does not require intensive care and/or artificial ventilation. A asymptomatic (reference) subject can be defined by one of the above definitions or any combination thereof.

A (reference) subject with mild symptoms preferably have no respiratory syndrome, more preferably no acute respiratory syndrome, even more preferably no severe acute respiratory syndrome. A (reference) subject with mild symptoms preferably does not require intensive care and/or artificial ventilation. A (reference) subject with mild symptoms can have at least one symptom selected from the group consisting of fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste, lack of appetite, loss of weight, stomach pain, conjunctivits, skin rash, lymphoma, apathy, and somnolence, more preferably has no fever, cough, fatigue, difficulty breathing, chills, joint or muscle pain, expectoration, sputum production, dyspnea, myalgia, arthralgia or sore throat, headache, nausea, vomiting, diarrhea, sinus pain, stuffy nose, reduced or altered sense of smell or taste, wherein the (reference) subject with mild symptoms does not require intensive care and/or artificial ventilation. A (reference) subject with mild symptoms can be defined by one of the above definitions or any combination thereof.

A reference subject can be a child, in particular a child having an age of less than 10 years, preferably less than 5 years. A reference subject can have an age of between 1 to 10 years, preferably 2 to 5 years.

A (reference) subject during or with a virus infection, particularly a coronavirus infection, more particularly a SARS-CoV-2 infection is a subject, which is preferably a (reference) subject infected with a virus, particularly a coronavirus, more particularly SARS-CoV-2. An infected (reference) subject means that the virus, particularly the coronavirus, more particularly the SARS-CoV-2 virus has entered the cells of the body of the (reference) subject, in is preferably proliferating in the cells of the body of the (reference) subject.

After infection with a virus, particularly a coronavirus, more particularly SARS-CoV-2, the interferon-gamma levels in the infected subject increase. The increased interferon-gamma levels in turn lead to an increase in the level of the angiotensin converting enzyme 2 (ACE2 receptor). Increased levels of the angiotensin converting enzyme 2 (ACE2 receptor) stimulate increased activity and priming of the spike protein by proteases. Then, the endogenous levels of AAT decrease in response to the increased activity level(s) of at least one spike protein priming protease. Afterwards, endogenous level of AAT deplete further as AAT binds (and inhibits) active spike protein priming proteases. Subjects having at least one selected from the group of i) a lower level of endogenous alpha-antitrypsin (AAT), ii) a higher level of at least one spike protein priming protease, iii) a higher level of angiotensin converting enzyme 2 (ACE2 receptor) and iv) a higher level of interferon-gamma (IFN-γ) compared to at least one reference subject, are particularly susceptible to develop a disease or syndrome in response to the virus, particularly the coronavirus, more particularly the SARS-CoV-2 infection. Therefore, these subjects of interest are particularly relevant and suited to receive treatment and/or prevention using a composition comprising a therapeutically effective amount of an alpha1-antitrypsin (AAT) protein, a variant, an isoform and/or a fragment thereof. The alpha1-antitrypsin (AAT) protein, a variant, an isoform and/or a fragment thereof can be a plasma-extracted AAT, a variant, an isoform and/or a fragment thereof, in particular a human plasma-extracted AAT, a variant, an isoform and/or a fragment thereof; or a recombinant alpha1-antitrypsin (rhAAT) protein, a variant, an isoform and/or a fragment thereof, preferably the alphal-antitrypsin (AAT) protein, a variant, an isoform and/or a fragment thereof is a recombinant alphal-antitrypsin (rhAAT) protein, a variant, an isoform and/or a fragment thereof. Most preferably, the AAT protein is recombinant alphal-antitrypsin (rhAAT) protein, produced in a Chinese Hamster Ovary (CHO) cell and/or in a Human Embryonic Kidney (HEK) cell.

The present invention also relates to a composition for use according to the present invention, wherein the subject in need thereof has at least one selected from the group consisting of:
1. a lower level of endogenous alpha-antitrypsin (AAT) prior to a virus infection, preferably a coronavirus infection, more preferably a SARS-CoV-2 infection or during a SARS-CoV-2 infection compared to at least one subject during a virus infection, preferably a coronavirus infection, more preferably a SARS-CoV-2 infection, which is asymptomatic or has mild symptoms,
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
3. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
4. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

The at least one subject, which is asymptomatic or has mild symptoms is also referred to as at least one reference subject. The reference subject is defined as described herein.

The "subject in need thereof "is also referred to as a subject of interest. The in need of a treatment and/or prevention of a disease syndrome related to a virus infection, preferably a coronavirus infection, more preferably a SARS-CoV-2 infection is defined as described herein.

The levels as defined in i) to iv) can be protein levels and/or mRNA levels, preferably a protein or mRNA level. Protein level(s) are measured using antibody-based assays such as enzyme-linked immunosorbent assay (ELISA), and/or bio-layer interferometry (BLI) based on fiber optic biosensors (ForteBio Octet).

Level of spike protein priming protease can be determined by measuring the activity of the spike protein protease using fluorogenic peptides derived from SARS-CoV-2 spike protein. The method is described in Jaimes et. al (Javier A. Jaimes, Jean K. Millet, Gary R. Whittaker Proteolytic Cleavage of the SARS-CoV-2 Spike Protein and the Role of the Novel S1/S2 Site, CELL, iScience 23, 101212, June 26, 2020). Transparent Methods Peptides: Fluorogenic peptides derived from SARS-CoV-2 spike (S) S1/S2 sites composed of the sequences HTVSLLRSTSQ (SEQ ID NO: 11) and TNSPRRARSVA (SEQ ID NO: 12) sequences, respectively, and harboring the (7- methoxycoumarin-4-yl)acetyl/2,4-dinitrophenyl (MCA/DNP) FRET pair were synthesized by Biomatik (Wilmington, DE, USA). Recombinant furin can be purchased from New England Biolabs (Ipswich, MA, USA). Recombinant L-1-Tosylamide-2-phenylethyl chloromethyl ketone (TPCK)-treated trypsin can be obtained from Sigma-Aldrich (St Louis, MO, USA). Recombinant PC1, matriptase, cathepsin B, and cathepsin L can be purchased from R&D Systems (Minneapolis, MN, USA). Fluorogenic peptide assay: For each fluorogenic peptide, a reaction is performed in a 100 µL volume with buffer composed of 100 mM Hepes, 0.5% Triton X-100, 1 mM CaCl2 and 1 mM 2-mercaptoethanol pH 7.5 for furin (diluted to 10 U/mL); 25 mM MES, 5 mM CaCl2, 1% (w/v) Brij-35, pH 6.0 for PC1 (diluted to 2.2 ng/µL); PBS for trypsin (diluted to 8 nM); 50 mM Tris, 50 mM NaCl, 0.01% (v/v) Tween^{®} 20, pH 9.0 for matriptase (diluted to 2.2 ng/µL); 25 mM MES, pH 5.0 for cathepsin B (diluted to 2.2 ng/µL); 50 mM MES, 5 mM DTT, 1 mM EDTA, 0.005% (w/v) Brij-35, pH 6.0 for cathepsin L (diluted to 2.2 ng/µL) and with the peptide diluted to 50 µM. Reactions are performed at 30 °C in triplicates, and fluorescence emission is measured every minute for 45 min using a SpectraMax fluorometer (Molecular Devices, Sunnyvale, CA, USA), with λex 330 nm and λem 390 nm wavelengths setting, enabling tracking of fluorescence intensity over time and calculation of Vmax of reactions. Assays should be performed in triplicates with results representing averages of Vmax from three independent experiments.

Level of IFN-γ protein could be measured using a flow cytometry, particle-based immunoassay. The method can be adopted from Huang et. al., (Huang KJ, Su IJ, Theron M, et al. An interferon-gamma-related cytokine storm in SARS patients. J Med Virol. 2005;75(2):185-194. doi:10.1002/jmv.20255) BD Human Th1/Th2 Cytokine or Chemokine Bead Array (CBA) Kit. The BD Human Th1/Th2 Cytokine CBA Kit (BD PharMingen, San Diego, CA) to measure IFN-γ levels by flow cytometry in a particle-based immunoassay. This kit allowed simultaneous measurement of six cytokines from 50 ml of patient serum sample. The limits of detection of these immunoassays is 7.1 pg/ml for IFN-γ.

Preferably, the endogenous level of AAT described herein is a protein level. The level of the at least one spike protein protease described herein is preferably mRNA level. The level of the ACE2 receptor described herein is preferably a mRNA level. The level of IFN-γ described herein is preferably a protein level. The protein and/or mRNA levels can be measured in blood, urine or saliva, preferably in blood, more preferably in blood plasma, most preferably in human blood plasma.

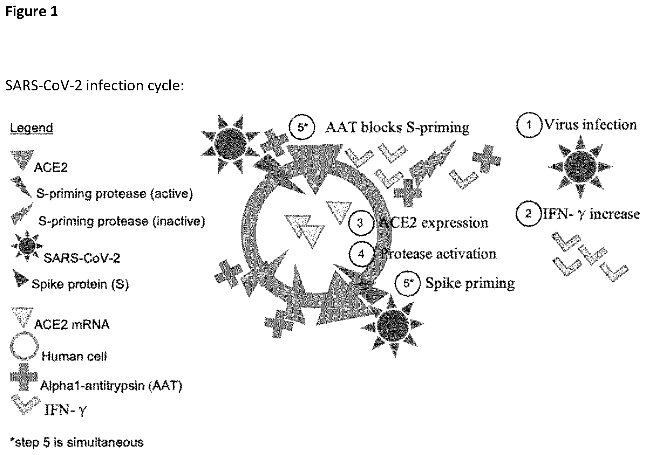

Several factors of the entry of viruses, particularly coronaviruses, more particularly the SARS-CoV-2 virus into cells and propagation in cells are already understood, while others are still subject of investigation. Entry of the SARS-CoV-2 virus is mediated by the spike protein, spike protein priming protease(s) and an ACE2 receptor. The SARS-CoV-2 spike protein is also referred to as spike protein S. The spike protein priming protease cleaves the spike protein of SARS-CoV-2 thereby priming the SARS-CoV-2 for entry into the cell. SARS-CoV-2 enters the cell by interaction of the primed spike protein with the ACE receptor. Thus, if at least one or more priming protease(s) is present in the subject of interest, the easier and faster the entry of the SARS-CoV-2 virus into the cells. Also, the more ACE2 receptor(s) is present, the easier and faster the entry of the SARS-CoV-2 into the cells. The infection with SARS-CoV-2 leads to inflammation and thus elevated IFN-γ expression. IFN-γ expression in response to a SARS-CoV-2 infection can in turn lead to an increased expression of the ACE2 receptor. During proliferation of SARS-CoV-2, IFN-γ levels increase further, stimulating the increase in ACE2 interaction with the spike protein and subsequently the priming of the spike protein, AAT levels decrease and upon viral entry into the cells, inflammation increases, which leads to even higher levels of IFN-γ. After infection with SARS-CoV-2, first, the IFN-γ levels increase, second, the AAT levels decrease and the levels of cathepsin L increase and/or other spike protein priming (S-priming) proteases increase. See Figure 1.

Therefore, the invention is at least in part based on the discovery, that AAT as well as rhAAT simultaneously reduces viral entry (see e.g. Figure 6, 7, 8, 11, 12, 21, 22) and virus associated inflammation (see e.g. Figure 19, 20), in particularly virus associated inflammation due to high IFN-γ levels. This combined effect is particularly useful in the patient populations described herein.

In certain aspects the invention relates to the composition for use according to the invention, wherein the spike protein priming protease is at least one selected from the group consisting of transmembrane protease serine subtype 2 (TMPRSS2), transmembrane protease subtype 6 (TMPRSS6), cathepsin L, cathepsin B, proprotein convertase 1 (PC1), trypsin, elastase, neutrophil elastase, matriptase and furin.

In certain aspects the invention relates to the composition for use according to the invention, wherein the spike protein priming protease is cathepsin L and/or furin.

AAT is endogenously expressed in the human body. AAT is also referred to as alpha-1-proteinase inhibitor. AAT is capable of inhibiting proteases, specifically spike protein proteases, such as transmembrane protease serine subtype 2 (TMPRSS2), transmembrane protease subtype 6 (TMPRSS6 / matriptase-2), cathepsin L, cathepsin B, proprotein convertase 1 (PC1), trypsin, elastase, neutrophil elastase, matriptase and furin. If the levels of the four players of the present invention (AAT, spike protein priming protease(s), ACE2 receptor and IFN-γ) are altered in an infected subject of interest compared to a reference subject, the infected subject of interest may benefit particularly form a treatment and/or prevention of a diseases or syndrome related with a SARS-CoV-2 infection. Low endogenous AAT levels can lead to a higher susceptibility of a subject of interest to develop a disease or syndrome related to SARS-CoV-2, in particular to develop COVID-19.

In certain aspects the invention relates to the composition for use according to the invention, wherein the lower level of endogenous AAT prior to a virus infection or during a virus infection is caused by AAT-deficiency.

Alphal-Antitrypsin (hereafter "AAT") is a protein that naturally occurs in the human body and is produced in the liver, preferably in hepatocytes. According to Janciauskiene et. al., (Janciauskiene SM, Bals R, Koczulla R, Vogehneier C, Kõhnlein T, Welte T. The discovery of α1-antitrypsin and its role in health and disease. Respir Med. 2011;105(8):1129-1139. doi:10.1016/j.rmed.2011.02.002) the normal plasma concentration of AAT ranges from 0.9 to 1.75 g/L. Considering a MW of 52,000 (Brantly M, Nukiwa T, Crystal RG. Molecular basis of alpha-1-antitrypsin deficiency. Am J Med. 1988;84(6A):13-31. doi:10.1016/0002-9343(88)90154-4), this corresponds to 16 to 32 µM normal blood plasma concentrations. Crystal 1990 (Crystal RG. Alpha 1-antitrypsin deficiency, emphysema, and liver disease. Genetic basis and strategies for therapy. J Clin Invest. 1990 May;85(5):1343-52. doi: 10.1172/JCI114578. PMID: 2185272; PMCID: PMC296579) describes that 11 µM is the threshold level for the clinical manifestation of AAT-deficiency. For most healthy individuals, 2g daily expression of AAT in the liver is enough to reach this critical serum level of 11 µM, the endogenous AAT level is then sufficient to protect the lower respiratory tract from destruction by neutrophil elastase (NE) and inhibiting the progressive destruction of the alveoli, which culminates in emphysema. Crystal 1990 further notes that normal endogenous levels of AAT in healthy individuals vary between 20-53 µM. Endogenous levels of AAT protein in the blood plasma of healthy human subjects prior to a virus infection, preferably a coronavirus infection, more preferably a SARS-CoV-2 infection range between 5 and 60 µM, preferably between 10 and 40 µM, more preferably between 25 to 30 µM, even more preferably between 16 and 32 µM. Alternatively the Endogenous levels of AAT protein in the blood plasma of healthy human subjects prior to a virus infection, preferably a coronavirus infection, more preferably a SARS-CoV-2 infection is preferably higher than 30 µM, more preferably higher than 40 µM, most preferably higher than 50 µM. AAT protein can be plasma AAT protein or recombinant AAT (rhAAT) protein. In some aspects, Plasma AAT protein is derived from blood plasma. In some aspects, Recombinant AAT protein is produced recombinantly, for example in HEK cells, CHO cells or E. coli cells. Pharmaceutical companies worldwide derive AAT protein from human blood plasma for the treatment of AAT-deficiency, a hereditary disorder. Plasma derived AAT is approved in the US and the EU, by the Food and Drug Administration (FDA) and the European Medicines Agency (EMA), respectively. Preferably, the AAT protein of the invention has the human amino acid sequence, most preferably as set forth in SEQ ID NO: 1 (see Table 1).

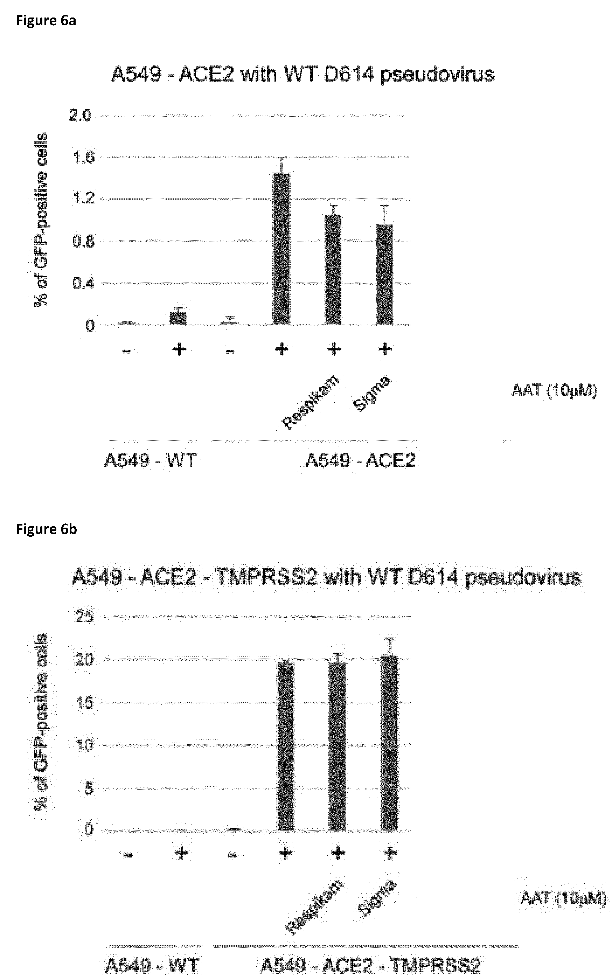

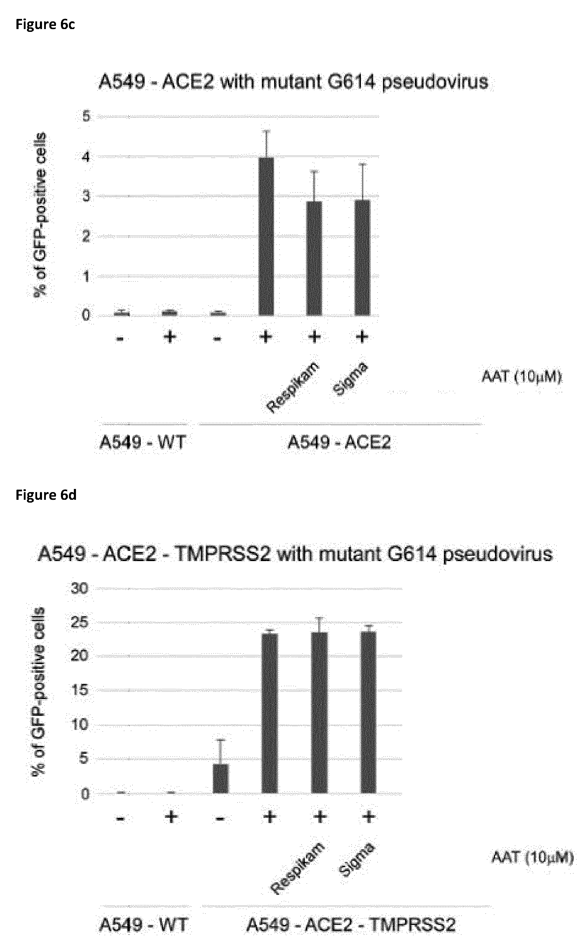

As shown in Figure 6a and 6c of the present invention, 10 µM of AAT reduces pseudoviral entry by 20-30% in A549 cells, which overexpress the ACE2 receptor.

According to Azouz et. al. (Nurit P. Azouz, Andrea M. Klingler and Marc E. Rothenberg, Alphal-Antitrypsin (AAT) is an Inhibitor of the SARS-CoV-2-Priming Protease TMPRSS2, (bioRxiv preprint online, https://doi.org/10.1101/2020.05.04.077826, posted on May 5, 2020) concentrations of 1-100 µM AAT achieve dose-dependent inhibition of TMPRSS2 proteolytic activity.

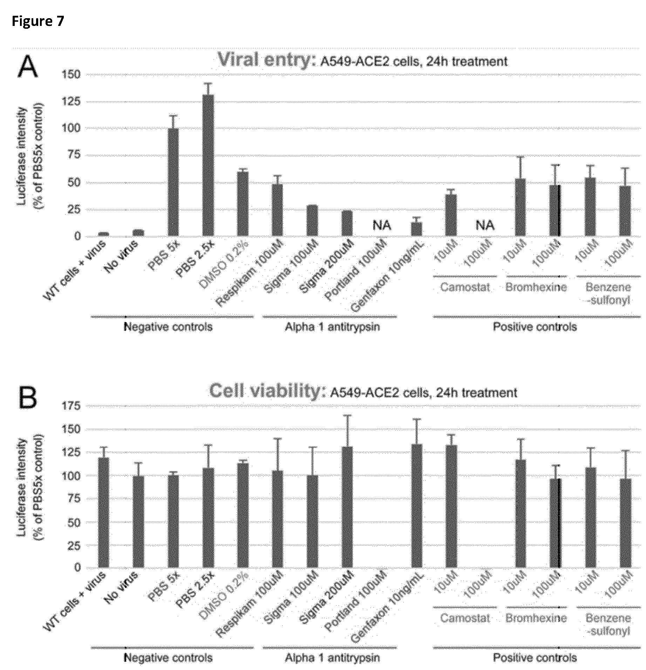

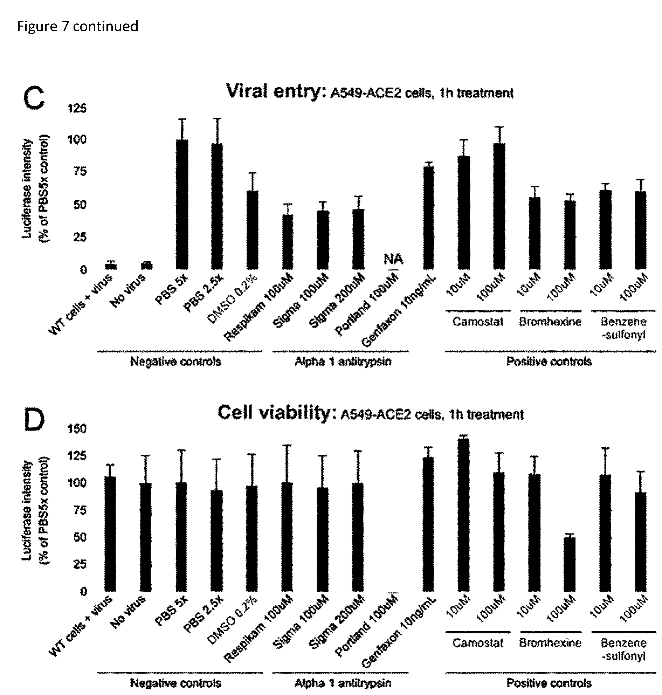

As shown in Figure 7 of the present invention, 100 µM of AAT reduces pseudoviral entry by 50-75% in A549 cells, which overexpress the ACE2 receptor only.

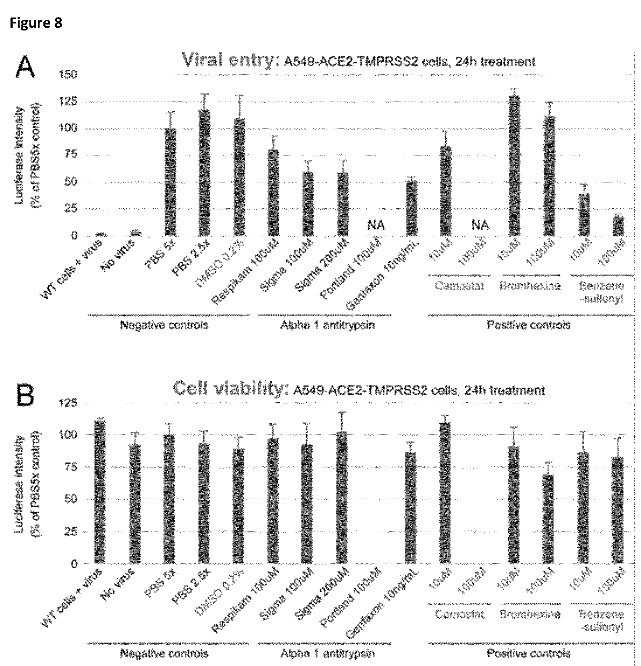

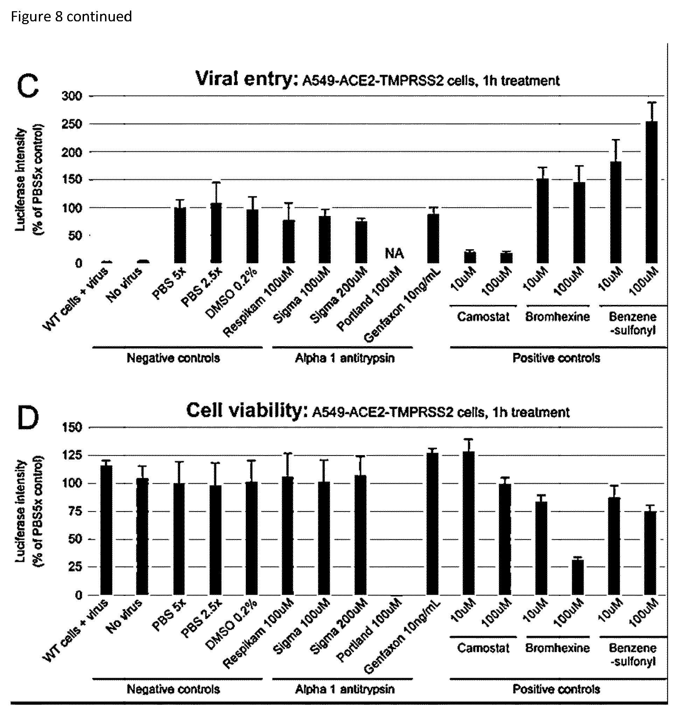

As shown in Figure 8 of the present invention, 100 µM of AAT reduces pseudoviral entry by up to 45% only in A549 cells that overexpress both the ACE2 receptor and the spike protein priming protease TMPRSS2.

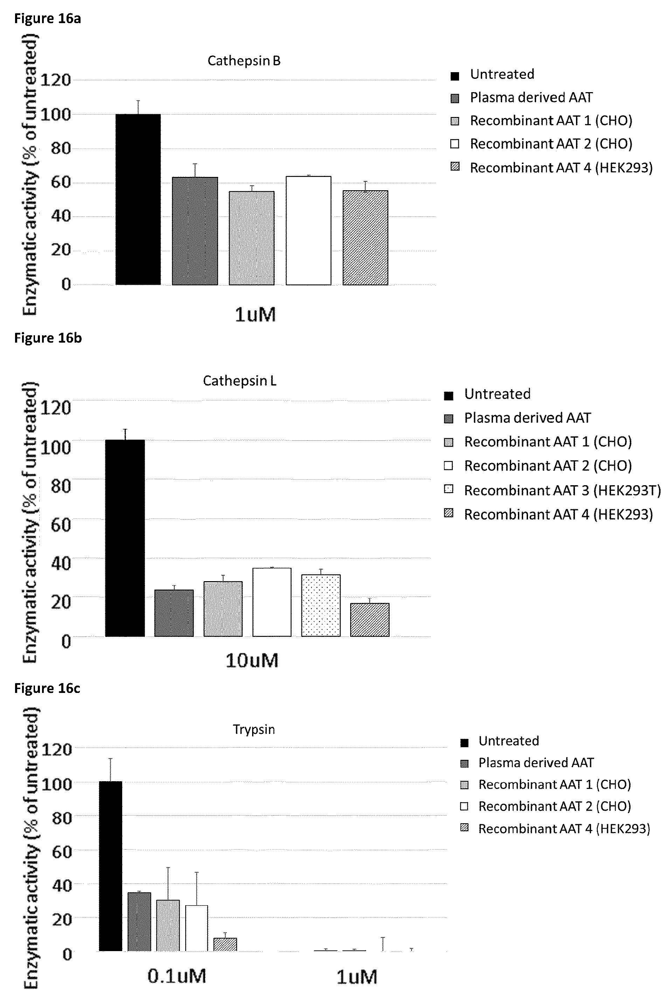

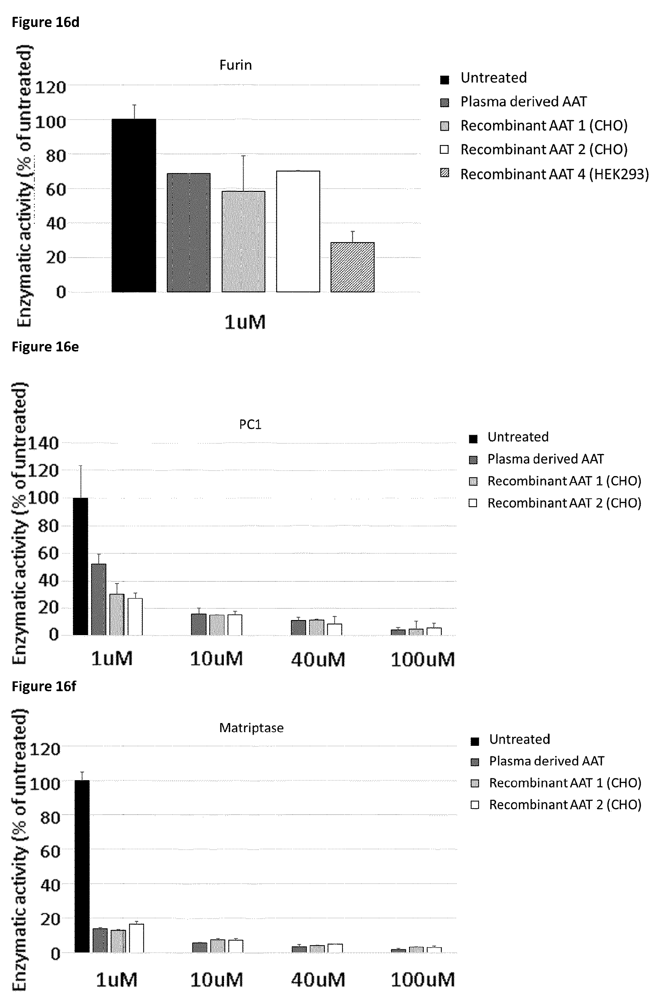

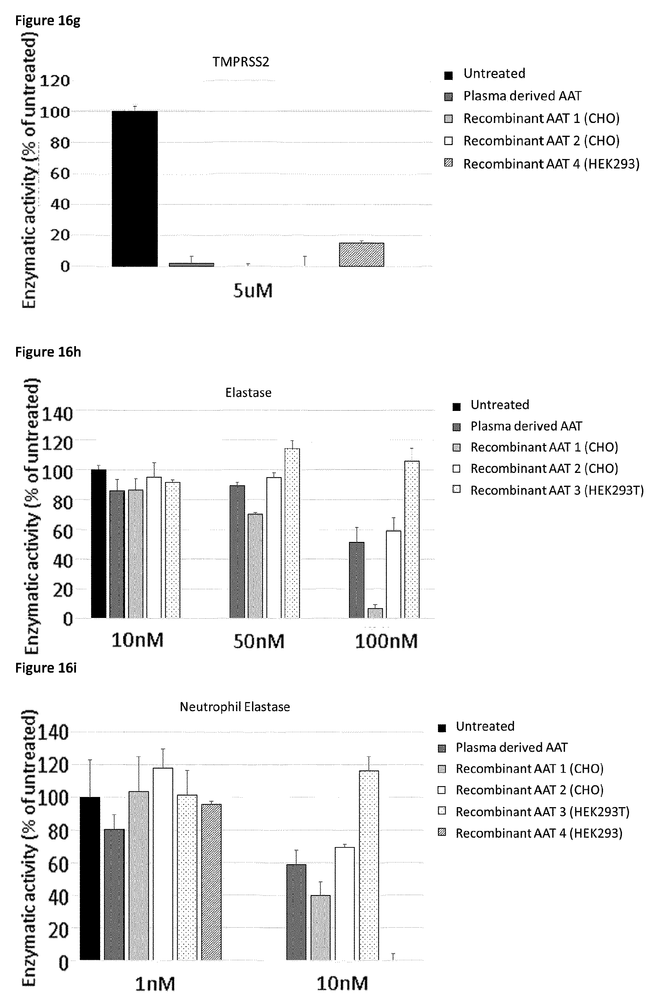

It is important to note that viral entry is observed independently of TMPRSS2 in both Figure 6 and 7 of the current invention. Demonstrating that priming proteases such as furin and/or cathepsin L are able to replace TMPRSS2, probably among others. In this regard, AAT as well as rhAAT reduces the activity of the proteases cathepsin B, cathepsin L, trypsin, furin, PC1, Matriptase, elastase and neutrophil elastase (Figure 16).

Therefore, the inhibitory effect of AAT as well as rhAAT on viral entry extends beyond the effect of other TMPRSS2 inhibitors (Figure 7, 8, 11, 12, 21, 22), in that AAT as well as rhAAT effectively inhibits several priming proteases (Figure 16) (e.g., proteases that can replace TMPRSS2 function) and subsequent ACE2 mediated viral entry (Figure 7, 8, 11, 12, 21, 22). Therefore, AAT as well as rhAAT reduces viral entry by reducing priming protease activity, in particular by broadly and efficiently reducing priming protease(s) activity.

Accordingly, the invention is at least in part based on the surprising finding that compositions comprising AAT and/or rhAAT, variants, isoforms and/or fragments thereof, are particularly effective in the treatment and/or prevention of a disease or syndrome related to a virus infection, preferably a coronavirus infection, more preferably a SARS-CoV-2 infection, in particular in a subject with one or more of the preconditions described herein.

In the present invention, the lower level of endogenous AAT according to i) is preferably a protein level in human blood plasma. The level of endogenous AAT protein in human blood plasma according to i) is preferably less than 200 µM, preferably less than 150 µM, 100 µM, less than 90, µM, less than 80 µM, less than 70 µM, less than 60 µM, less than 50 µM, less than 40 µM, less than 30 µM, less than 25 µM, less than 20 µM, less than 15 µM, less than 11 µM or less than 10 µM. More preferably less than 200 µM, less than 100 µM, less than 25 µM, less than 15 µM, or less than 11 µM. A low level of AAT is not sufficient to successfully inhibit the spike protein priming protease(s). A low level of endogenous AAT can therefore promote virus proliferation, in particular coronavirus proliferation, more particularly SARS-CoV-2 proliferation and/or the development of a disease of syndrome related to a virus infection, preferably a coronavirus infection, more preferably a SARS-CoV-2 infection. A lower level of endogenous AAT prior to a virus infection, preferably a coronavirus infection, more preferably a SARS-CoV-2 infection or during a virus infection, preferably a coronavirus infection, more preferably a SARS-CoV-2 infection can be caused by AAT deficiency, preferably lower level of endogenous AAT prior to a virus infection, preferably a coronavirus infection, more preferably a SARS-CoV-2 infection is caused by AAT deficiency. The AAT-deficiency is a condition that is inherited in an autosomal codominant pattern. Codominance means that two different versions of the gene may be active (expressed), and both versions contribute to the genetic trait. The most common version (allele) of the *SERPINA1* gene, called M, produces normal levels of alpha-1 antitrypsin. Most people in the general population have two copies of the M allele (MM) in each cell. Other versions of the *SERPINA1* gene lead to reduced levels of alpha-1 antitrypsin. For example, the S allele produces moderately low levels of this protein, and the Z allele produces very little alpha-1 antitrypsin. Individuals with two copies of the Z allele (ZZ) in each cell are likely to have alpha-1 antitrypsin deficiency. Those with the SZ combination have an increased risk of developing lung diseases (such as emphysema), particularly if they smoke. Worldwide, it is estimated that 161 million people have one copy of the S or Z allele and one copy of the M allele in each cell (MS or MZ). Individuals with an MS (or SS) combination usually produce enough alpha-1 antitrypsin to protect the lungs. People with MZ alleles, however, have a slightly increased risk of impaired lung or liver function. Subjects with an AAT deficiency in the present invention preferably have ZZ mutation, SZ mutation, MS mutation, MZ mutation or SS mutation of the *SERPINA1* gene, preferably a ZZ mutation. The low levels of AAT secretion in the ZZ mutation of the *SERPINA1* gene are due to misfolding of AAT and its subsequent accumulation in the endoplasmic reticulum (ER) of hepatocytes (Crystal 1990), resulting in progressive liver disease as the accumulation of misfolded AAT negatively affects hepatocytes' health leading to their ultimate demise.

A lower level of endogenous AAT during a virus infection, preferably a coronavirus infection, more preferably a SARS-CoV-2 infection can also be caused by the virus infection, the coronavirus infection, or the SARS-CoV-2 infection respectively. That is, the level of endogenous AAT in a reference subject can increase temporarily during the virus infection as healthy hepatocytes attempt to overcompensate for the drop in endogenous AAT levels, while the level of endogenous AAT in a subject with genetic AAT-deficiency is lower due to absence or incomplete virus infection-induced increase (lack of healthy hepatocytes).

A lower level of endogenous AAT may also be caused by a liver disease such as a non-alcoholic fatty liver disease, diabetes mellitus type 1 or 2 (preferably type 1), obesity and cardiovascular conditions. Fatty acid deposit build-up in the liver leads to increased stress (increased IFN-γ), tissue inflammation and subsequent damage to hepatocytes causing lower level of healthy secretion of AAT. As with other types of liver maladies, it is important to note that AAT-deficiency leads to liver disease over time as the accumulation of misfolded AAT in the ER of hepatocytes ultimately causes liver failure.

In the present invention, the spike protein priming protease can be any protease capable of priming the spike protein of a virus, preferably a coronavirus, more preferably SARS-CoV-2. Preferably, the spike protein priming protease is at least one selected from the group consisting of transmembrane protease serine subtype 2 (TMPRSS2), transmembrane protease subtype 6 (TMPRSS6), cathepsin L, cathepsin B, proprotein convertase 1 (PC1), trypsin, elastase, neutrophil elastase, matriptase and furin, more preferably TMPRSS2, cathepsin L and furin, even more preferably cathepsin L or furin. The spike protein priming protease is furin is also referred to as paired basic amino acid cleaving (PACE) enzyme.

The higher level of the at least one spike protein priming protease, preferably Cathepsin L, described herein is preferably a mRNA level in human blood plasma, or a protein level in human blood plasma. The plasma concentration of Cathepsin L in healthy subjects is 0.2 to 1 ng/mL (i.e. 10 to 50 pM given a molecular weight of about 23-24 kDa; (Kirschke 1977 https://febs.onlinelibrary.wiley.com/doi/10.1111/j.1432-1033.1977.tb11393.x). Moreover, immune cells are known to be a major source of extracellular cysteine cathepsins in inflammation, including in the brain (Hayashi et al., 2013, von Bernhardi et al., 2015, Wendt et al., 2008, Wendt et al., 2007). The level of the at least one spike protein priming protease protein described herein is preferably higher than 0.2, 0.5 or 1 ng/ml in human blood plasma. The level of the at least one spike protein priming protein described herein protease is preferably higher than 10, 20, 30, 40, 50, 75 or 100 pM, preferably higher than 10 or 50 pM, even more preferably higher than 50 pM. In this aspect the at least one spike protein priming protease(s) is preferably cathepsin L. In certain aspects, the at least one spike protein priming protease(s) described herein are at least two, at least three, at least four, at least five, at least six, at least seven, at least eight, at least nine spike protein priming proteases. In certain aspects, the at least one spike protein priming protease(s) described herein is at least one protease selected from the group consisting of TMPRSS2, cathepsin B, cathepsin L, trypsin, furin, PC1, matriptase, elastase and neutrophil elastase. In certain aspects, the at least one spike protein priming protease(s) described herein is at least one protease selected from the group consisting of TMPRSS2, cathepsin B, cathepsin L, trypsin, furin, PC1 and matriptase.

In certain aspects the invention relates to the composition for use according to the invention, wherein the higher level of at least one spike protein priming protease is caused by age and/or a genetic predisposition.

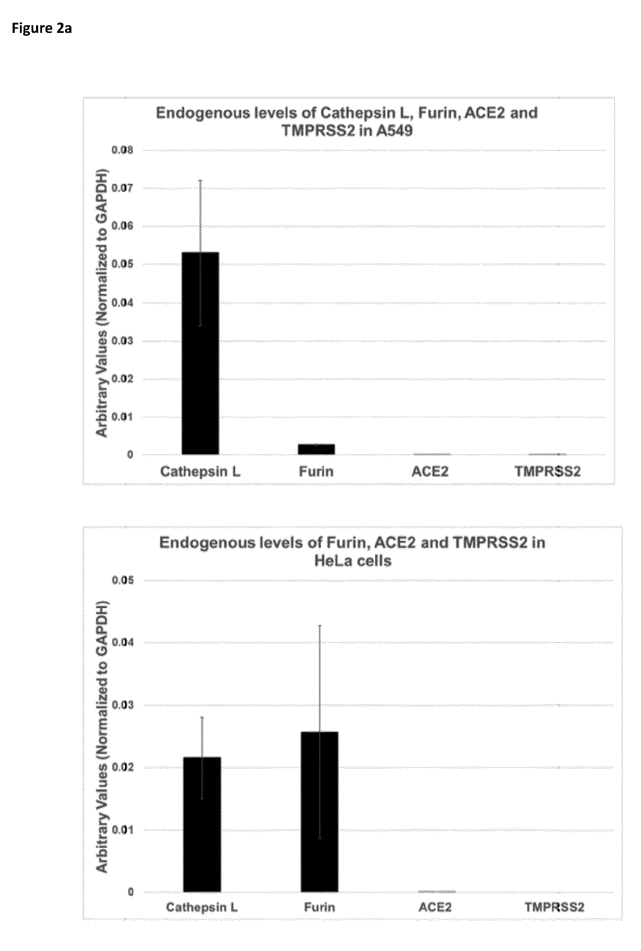

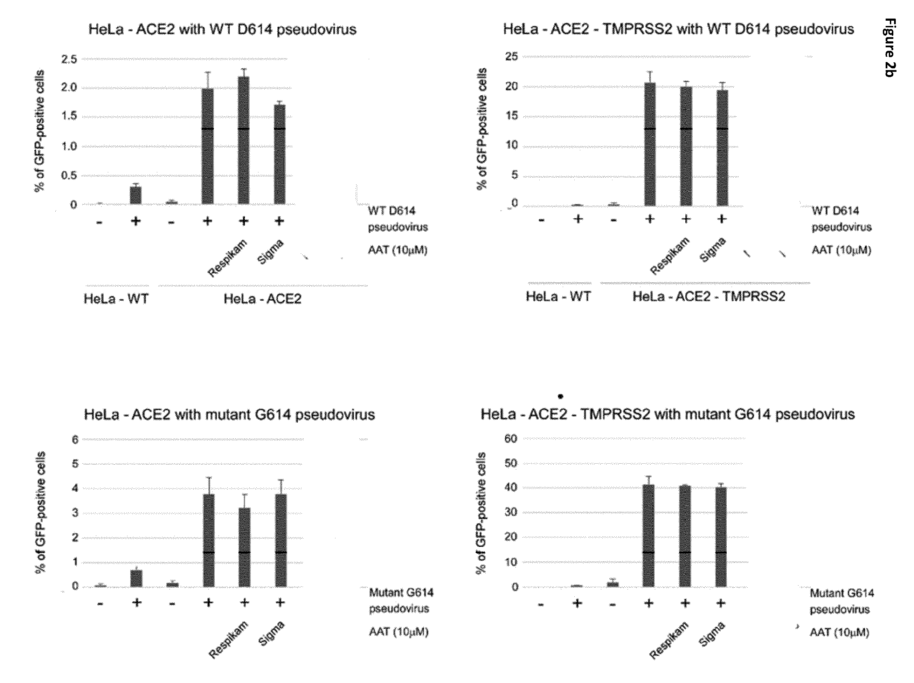

The higher level of the at least one spike protein priming protease can be caused by age and/or a genetic predisposition. In particular, higher level of cathepsin B and L, preferably of cathepsin B can be caused by age. Higher levels of spike protease protein, in particular cathepsin B and L, more preferably cathepsin B can accumulate in the lysosome. The level of spike protein priming protease is higher in subjects of interest, with an age of more than 50 years, preferably more than 60 years, more preferably more than 70 years, even more preferably more than 80 years, and most preferably more than 90 years. Subjects of Afro-American origin may have a genetic predisposition for higher levels of spike protein priming protease, in particular furin. As shown in Figure 2(a), HeLa cells differ in relative mRNA rates of furin and Cathepsin L compared to A549 cells. HeLa cells are of Afro-American origin. A549 cells are airway epithelial cells of Caucasian origin. As shown in Figure 2(b), HeLa cells are more susceptible to SARS-CoV-2 entry than A549 cells (Figure 6) and are less reactive to treatment with AAT. A genetic predisposition can be determined by genetic profiling of individual subjects of interest.

In certain aspects the invention relates to the composition for use according to the invention, wherein the higher level of ACE2 receptor is caused by at least one selected from the group of an infection, inflammation, age and a genetic predisposition.

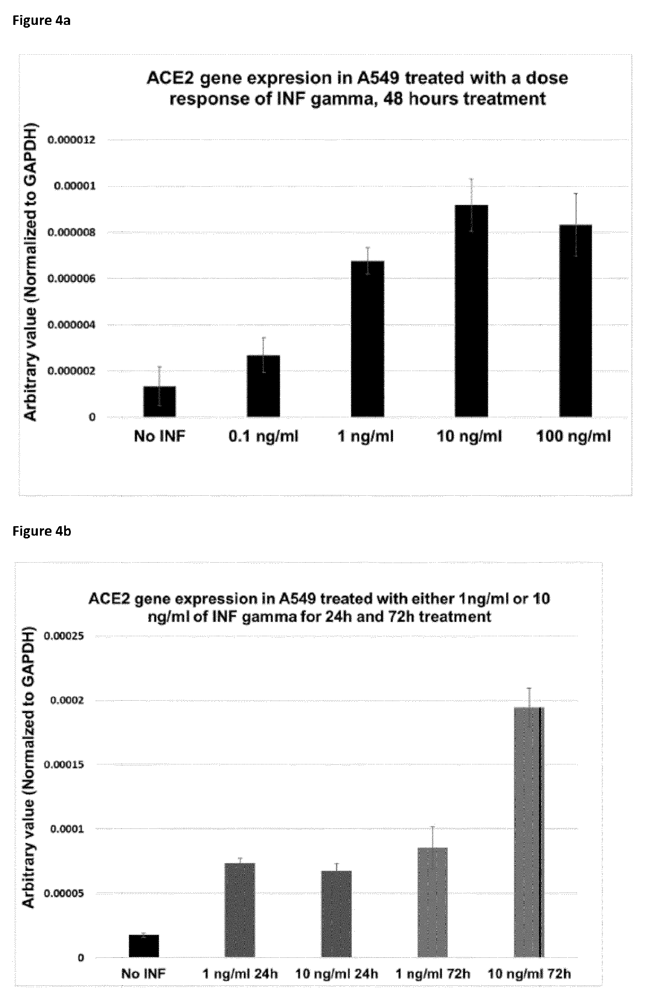

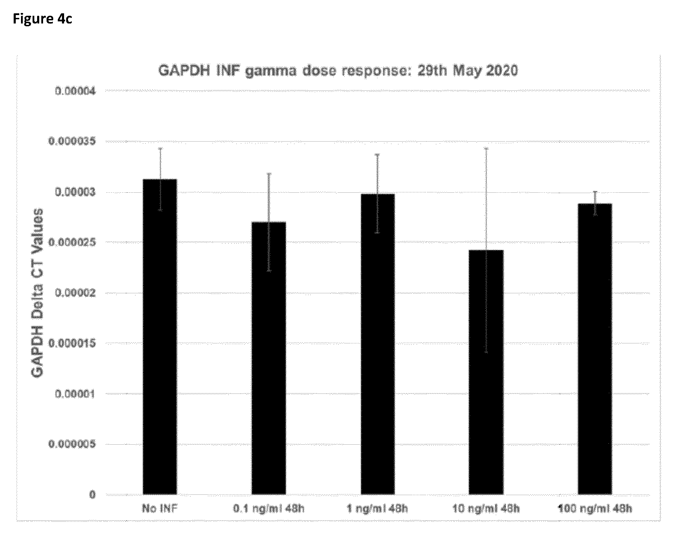

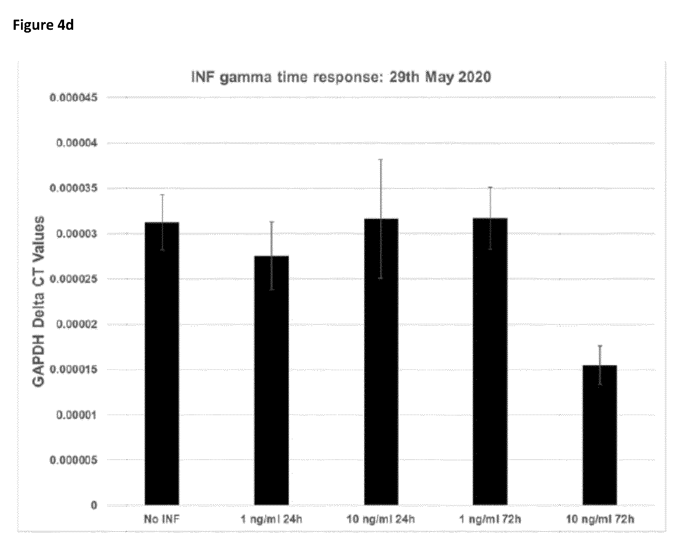

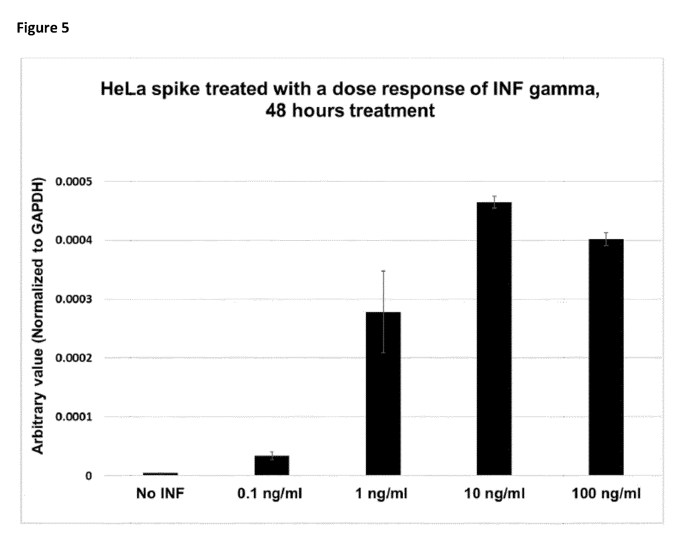

The higher level of ACE 2 receptor described herein is preferably an mRNA level in human blood plasma. The higher level of the ACE2 receptor can be caused by at least one selected from the group of an infection, inflammation (e.g. IFN-y), age and a genetic predisposition. The infection can be a viral infection and/or a bacterial infection, preferably a viral infection, more preferably a coronavirus infection, even more preferably a SARS or SARS-CoV-2 infection. A further example for infection is Leishmaniasis. As shown in Figure 4 and 5, ACE2 receptor expression is upregulated in response to higher levels of IFN-y, which levels in turn increase as an immune response to inflammation. The inflammation can be caused, for example, by a bacterial and/or viral infection, cancer, delayed type hypersensitivity; autoimmune diseases (such as autoimmune encephalomyelitis, rheumatoid arthritis, autoimmune insulitis (also referred to as type 1 diabetes mellitus), allograft rejection and graft versus host reaction, nonspecific inflammation and cytokine release. The age is preferably an age of more than 50 years, preferably more than 60 years, more preferably more than 70 years, even more preferably more than 80 years, and most preferably more than 90 years. An example of a genetic predisposition for high levels of IFN-γ is familial Mediterranean fever. A genetic predisposition can be determined by genetic profiling of individual subjects of interest. A higher level of ACE2 receptor described herein can also be present in a tissue selected from the group consisting of lung (Calu3), colon (CaCo2), liver (HEPG2), kidney (HEK-293T), and the brain (SH-SY5Y) as confirmed by qPCR and shown in Figure 3a of the current invention.

In certain aspects the invention relates to the composition for use according to the invention, wherein the higher level of IFN-γ is caused by at least one selected from the group of an infection, inflammation, age and a genetic predisposition.

The higher level of interferon-gamma (IFN-γ) according to iv) is preferably a protein or mRNA level in human blood plasma, more preferably a protein level in human blood plasma. In healthy humans, the IFN-γ level is below or around the limit of detection of the assays, e.g. less than 30 to 50 pg/mL) (Billau 1996; Kimura 2001). IFN-γ is produced almost exclusively by natural killer (NK) cells, CD4+ and some CD8+ lymphocytes. Production of IFN-γ by either NK or T cells requires cooperation of accessory cells, mostly mononuclear phagocytes, which also need to be in some state of activation (Billiau A. Interferon-gamma: biology and role in pathogenesis. Adv Immunol. 1996;62:61-130. doi:10.1016/s0065-2776(08)60428-9). Thus, inflammatory conditions leading to NK cells activation and T cells activation lead to increased IFN-γ levels. Inflammation state involving circulating NK or T cells (infection, cancer) are expected to lead to higher plasma levels.

The following Table provides values for plasma levels of IFN-γ in several conditions.

| Condition | Type of condition | IFN-γ (pg/mL) | IFN-γ-Control (pg/mL) | Reference |
|---|---|---|---|---|
| SARS | infection | 456 (acute phase) | 3.3 (control) | *Huang et. al.,* |
| | | | | Huang, K. - J., Su, I. - J., Theron, M., Wu, Y. - C., Lai, S. - K., Liu, C. - C. and Lei, H. - Y. (2005), An interferon - γ - related cytokine storm in SARS patients, J. Med. Virol., 75: 185-194. doi:10.1002/imv.20255 |
| | | 26.6 pM | 0.19 pM | |
| Leishmaniasis | infection | 118 (untreated) | 31 (treated) | *Hailu et. al.,* |
| | | | 1.8 pM | Hailu A, van der Poll T, Berhe N, Kager PA. Elevated plasma levels of interferon (IFN)-gamma, IFN-γ inducing cytokines, and IFN-γ inducible CXC chemokines in visceral leishmaniasis. Am J Trop Med Hyg. 2004;71(5):561-567. |
| | | 6.9 pM | | |
| Familial Mediterranean fever | Genetic disorder | 19.4 (acute attack) | 4.8 (attack-free) | *Köklü et. al.,* |
| | | | | Köklü S, Oztürk MA, Balci M, Yüksel O, Ertenli I, Kiraz S. Interferon-gamma levels in familial Mediterranean fever. Joint Bone Spine. 2005;72(1):38-40. doi:10.1016/j.jbspin.2004.03.01 1 |
| | | | 0.28 pM | |
| | | 1.1 pM | | |

The level of IFN-γ protein in human blood plasma according to iv) is preferably high than 1, 5, 10, 15, 20, 25, 30, 40, 50, 75, 100, 125, 150, 200, 300, 400 or 500 pg/ml. The higher level of IFN-γ is preferably caused by at least one selected from the group of an infection, inflammation, age and a genetic predisposition, more preferably by inflammation. The infection can be a viral infection and/or a bacterial infection, preferably a viral infection, more preferably a coronavirus infection, even more preferably a SARS or SARS-CoV-2 infection. A further example for infection is Leishmaniasis. The inflammation can be caused, for example, by a bacterial and/or viral infection, cancer, delayed type hypersensitivity; autoimmune diseases (such as autoimmune encephalomyelitis, rheumatoid arthritis, autoimmune insulitis (also referred to as type 1 diabetes mellitus), allograft rejection and graft versus host reaction, nonspecific inflammation and cytokine release. The age is preferably an age of more than 50 years, preferably more than 60 years, more preferably more than 70 years, even more preferably more than 80 years, and most preferably more than 90 years. An example of a genetic predisposition for high levels of IFN-γ is familial Mediterranean fever. A genetic predisposition can be determined by genetic profiling of individual subjects of interest.

Accordingly, the invention is at least in part based on the surprising finding that compositions comprising AAT as well as rhAAT, variants, isoforms and/or fragments thereof, reduce both virus proliferation and inflammation, in particular IFN-γ associated inflammation.

In one aspect, "subject in need thereof", i.e. the subject of interest has two selected from the group consisting of:
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
3. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
4. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

Thus, the "subject in need thereof" can have
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

Preferably, in this aspect, the AAT protein level in human blood plasma described herein is lower than 52 µM and the cathepsin L protein level in human blood plasma is higher than 10 pM.

In another aspect, the "subject in need thereof" can have
1. a lower level of endogenous alpha-antitrypsin (AAT) prior to a virus infection, preferably a coronavirus infection, more preferably a SARS-CoV-2 infection or during a SARS-CoV-2 infection compared to at least one subject during a virus infection, preferably a coronavirus infection, more preferably a SARS-CoV-2 infection, which is asymptomatic or has mild symptoms, and
2. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In yet a further aspect, the "subject in need thereof" can have
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
2. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In these aspects, 1. and 4. can be at least one disease or condition selected from the group consisting of AAT-deficiency, a liver disease such as a non-alcoholic fatty liver disease, diabetes, obesity and a cardiovascular condition. In some aspects the invention relates to the composition for use according to the invention, wherein the higher level of IFN-γ is caused by at least one disease or condition selected from the group consisting of AAT-deficiency, a liver disease such as a non-alcoholic fatty liver disease, diabetes, obesity and a cardiovascular condition. In some aspects the invention relates to the composition for use according to the invention, wherein the lower level of AAT is caused by at least one disease or condition selected from the group consisting of AAT-deficiency, a liver disease such as a non-alcoholic fatty liver disease, diabetes, obesity and a cardiovascular condition. Diabetes can be diabetes mellitus type 1 or type 2. Liver disease can be acetaminophen-induced liver injury, severe chronic hepatitis, alcoholic liver disease (ALD), encompassing a broad spectrum of phenotypes including simple steatosis, steatohepatitis, liver fibrosis and cirrhosis or even HCC (hepatocellular carcinoma). Cardiovascular condition can be any condition brought on by a sudden reduction or blockage of blood flow to the heart, cardiac infraction, acute coronary syndrome (ACS), also in patients with acute myocardial infarction, whereby the left ventricular ejection fraction is inversely correlated with AAT concentrations in the serum, suggesting that systolic dysfunction is associated with an inflammatory response.

Preferably, in this aspect, the AAT protein level in human blood plasma described herein is lower than 52 µM and the IFN-γ protein level in human blood plasma described herein is higher than 0.19 pM.

In a further aspect, "subject in need thereof", i.e. the subject of interest has two selected from the group consisting of:
1. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
2. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
3. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In yet a further aspect, the "subject in need thereof" can have
1. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
2. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In yet a further aspect, the "subject in need thereof" can have
1. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
2. a higher level of interferon-gamma (IFN- γ ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In yet a further aspect, the "subject in need thereof" can have
1. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
2. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In a further aspect, the "subject in need thereof", i.e. the subject of interest has three selected from the group consisting of:
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
3. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
4. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

Thus, the "subject in need thereof' can have
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
3. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In a further aspect, the "subject in need thereof" can have
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
3. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

Preferably, in this aspect, the AAT protein level in human blood plasma described herein is lower than 36 µM, the cathepsin L protein level in human blood plasma described herein is higher than 10 pM, the IFN-γ protein level in human blood plasma described herein is higher than 0.19 mM.

More preferably, in this aspect, the AAT protein level in human blood plasma described herein is lower than 52 µM, the cathepsin L protein level in human blood plasma described herein is higher than 10 pM, the IFN-γ protein level in human blood plasma described herein is higher than 0.19 mM.

In a further aspect, the "subject in need thereof" can have
1. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
2. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
3. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

In a further aspect, the "subject in need thereof", i.e. the subject of interest has
1. a lower level of endogenous alpha-antitrypsin (AAT) prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms
2. a higher level of at least one spike protein priming protease prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms,
3. a higher level of angiotensin converting enzyme 2 (ACE2 receptor) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms, and
4. a higher level of interferon-gamma (IFN-γ) in a subject prior to a virus infection, preferably a coronavirus infection, more preferably a SARS-CoV-2 infection or during a virus infection, preferably a coronavirus infection, more preferably a SARS-CoV-2 infection compared to at least one subject during a virus infection, preferably a coronavirus infection, more preferably a SARS-CoV-2 infection, which is asymptomatic or has mild symptoms.

AAT protein can be plasma AAT protein, a variant, an isoform and/or a fragment thereof; or recombinant AAT protein, a variant, an isoform and/or a fragment thereof. Preferably, the AAT protein, a variant, an isoform and/or a fragment thereof is recombinant AAT protein, a variant, an isoform and/or a fragment thereof. Plasma AAT protein is preferably derived from blood plasma AAT protein, more preferably from human blood plasma (also referred to as human plasma-extracted AAT). In certain aspects, the alphal-antitrypsin (AAT) protein, a variant, an isoform and/or a fragment thereof is recombinant alphal-antitrypsin (also referred to as rhAAT), a variant, an isoform and/or a fragment thereof. Recombinant AAT protein is produced recombinantly, for example in CHO cells, HEK cells (HEK293 and/or HEK293T) or E. coli cells. Pharmaceutical companies worldwide derive AAT protein from human blood plasma for the treatment of AAT-deficiency, a hereditary disorder. Plasma derived AAT is FDA and EMA approved. Preferably, the AAT protein of the invention has the human amino acid sequence, most preferably as set forth in SEQ ID NO: 1 (see Table 1). Recombinant AAT protein a variant, an isoform and/or a fragment thereof is preferably free from an Fc-domain and/or histidine-tag (His-tag).

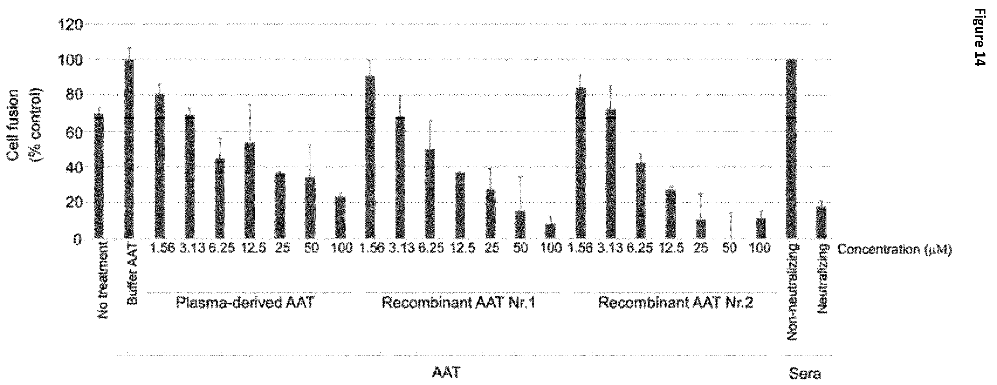

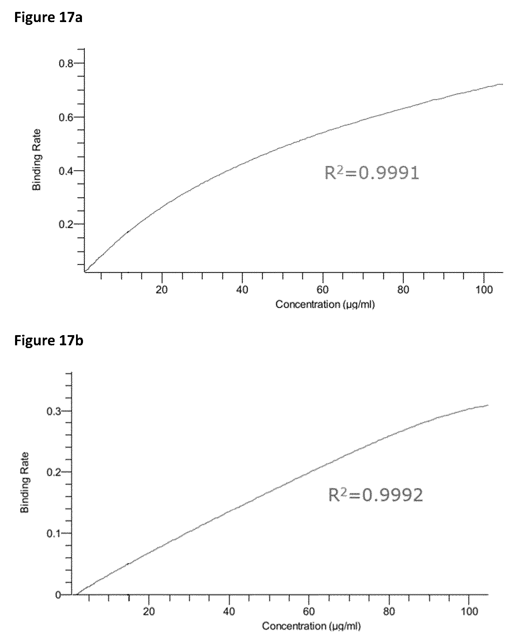

The inventor(s) found that recombinant AAT (rhAAT produced in CHO) binds to an AAT-Antibody with a different affinity (Figure 17) and has a more pronounced biologic effect than a plasma-derived AAT. Particularly, recombinant AAT (rhAAT) inhibits ACE2/Spike protein mediated cell fusion (Figure 14) more effectively than plasma derived AAT and has a different enzymatic inhibition profile (Figure 16).

Accordingly, the invention is at least in part based on the surprising finding that recombinant AAT (rhAAT) produced in CHO cells is particularly effective for use in the treatment and/or prevention of a disease or syndrome related to a virus infection, preferably a coronavirus infection, more preferably a SARS-CoV-2 infection.

In certain aspects, the invention relates to the composition for use according to the invention, wherein the alphal-antitrypsin (AAT) protein, a variant, an isoform and/or a fragment thereof is recombinant alphal-antitrypsin produced by in human cells (e.g. HEK293 or HEK293T cells, see Example 23).

The inventor(s) found that recombinant AAT produced in human cells, specifically in HEK293 (rhAAT without a His-tag), is particular effective in the reduction of enzymatic activity of Cathepsin L (Figure 16b), Trypsin (Figure 16c), Furin (Figure 16d) and Neutrophil Elastase (Figure 16i) compared to recombinant AAT (rhAAT) produced in CHO cells as well as plasma-derived AAT.

Accordingly, the invention is at least in part based on the surprising finding that recombinant AAT produced in human cells, specifically in HEK293, is particularly effective for use in the treatment and/or prevention of a disease or syndrome related to a virus infection, preferably a coronavirus infection, more preferably a SARS-CoV-2 infection.

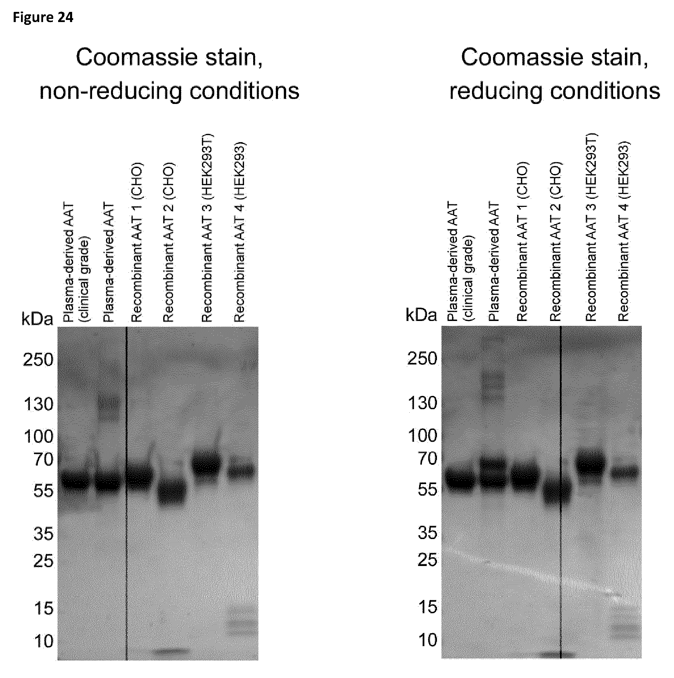

In certain aspects, the invention relates to the composition for use according to the invention, wherein the alphal-antitrypsin (AAT) protein, a variant, an isoform and/or a fragment thereof is recombinant alphal-antitrypsin (rhAAT produced in CHO) having more non-human glycan profile. In certain aspects, the non-human glycan profile described herein is a mammalian-cell-derived glycan profile and/or a glycoengineered glycan profile. Glycoengineering strategies, e.g., used to reduce fucosylation and/or enhance sialylation of glycoproteins are known to the person skilled in the art. In certain aspects, the non-human glycan profile described herein is a CHO-cell-derived glycan profile. CHO cells express a different glycosylation machinery than human cells, which results in different composition of glycans at the surface of recombinant proteins (Figure 24) (Lalonde, M. E., & Durocher, Y., 2017, Journal of biotechnology, 251, 128-140).

In certain aspects, the invention relates to the composition for use according to the invention, wherein the AAT protein, a variant, an isoform and/or a fragment thereof is recombinant AAT produced by pXC-17.4 (GS System, Lonza), a variant, an isoform and/or a fragment thereof. The inventor(s) found that the recombinant AAT produced by pXC-17.4 (GS System, Lonza) in CHO (Recombinant AAT 1) induces a more pronounced inhibitory effect on the activity of Elastase (Figure 16h) and Neutrophil Elastase (Figure 16i) than plasma-derived alpha1-proteinase inhibitor (Plasma-derived AAT) and recombinant AAT produced by PL136/PL137 (pCGS3, Merck) in CHO (Recombinant AAT 2).

Accordingly, the invention is at least in part based on the surprising finding, that certain forms of recombinant AAT (rhAAT) are particularly effective for use in the treatment and/or prevention of a disease or syndrome related to a virus infection, preferably a coronavirus infection, more preferably a SARS-CoV-2 infection.

As used herein, a "fragment" of an AAT protein, peptide or polypeptide refers to a sequence containing less amino acids in length than the AAT protein, peptide or polypeptide of the invention, in particular less amino acids than the sequence of AAT as set forth in SEQ ID NO:1. The fragment is preferably a functional fragment, e.g. a fragment with the same biological activities as the AAT protein as set forth in SEQ ID NO:1. The functional fragment preferably derived from the AAT protein as set forth in SEQ ID NO:1. Any AAT fragment can be used as long as it exhibits the same properties, i.e. is biologically active, as the native AAT sequence from which it derives.

Preferably, the (functional) fragment shares about 5 consecutive amino-acids, at least about 7 consecutive amino-acids, at least about 15 consecutive amino-acids, at least about 20 consecutive amino-acids, at least about 25 consecutive amino-acids, at least about 20 consecutive amino-acids, at least about 30 consecutive amino-acids, at least about 35 consecutive amino-acids, at least about 40 consecutive amino-acids, at least about 45 consecutive amino-acids, at least about 50 consecutive amino-acids, at least about 55 consecutive amino-acids, at least about 60 consecutive amino-acids, at least about 100 consecutive amino-acids, at least about 150 consecutive amino-acids, at least about 200 consecutive amino-acids, at least about 300 consecutive amino-acids, *etc...* or more of the native human AAT amino acid sequence as set forth in SEQ ID NO:1. In some aspects, the (functional) fragment described herein, comprises an expression optimized signal protein (e.g. Example 24)

The functional AAT fragment can comprise or consist of a C-terminal fragment of AAT as set forth in SEQ ID NO:2. The C-terminal fragment of SEQ ID NO:2 consist of amino acids 374 to 418 of SEQ ID NO:1.

More preferably, the AAT fragment is a fragment containing less amino acids in length than the C-terminal AAT sequence 374-418 (SEQ ID NO: 2). Alternatively, the AAT fragment consists essentially in SEQ ID NO: 2.

| **Table 1** | |
|---|---|
| | SE Q ID NO : |
| | 1 |
| | |
| MFLEAIPMSIPPEVKFNKPFVFLMIEQNTKSPLFMGKVVNPTQK | 2 |
| Cyclo-(CPFVFLM)-SH | 3 |
| Cyclo-(CPFVFLE)-SH | 4 |
| Cyclo-(CPFVFLR)-SH | 5 |
| Cyclo-(CPEVFLM)-SH | 6 |

The term "variant" refers to a protein, peptide or polypeptide having an amino acid sequence that differ to some extent from the AAT native sequence peptide, that is an amino acid sequence that vary from the AAT native sequence by amino acid substitutions, whereby one or more amino acids are substituted by another with same characteristics and conformational roles. Preferably, a variant of the present invention is at least 99%, 98%, 97%, 96%, 95%, 90% or 85% homologous to amino acids of SEQ ID NO: 1 or SEQ ID NO:2. A variant can also have at least 99%, 98%, 97%, 96%, 95%, 90% or 85% sequence identity to amino acids of SEQ ID NO:1 or SEQ ID NO:2. The amino acid sequence variants can have substitutions, deletions, and/or insertions at certain positions within the amino acid sequence of the native amino acid sequence, e.g. at the N- or C-terminal sequence or within the amino acid sequence. Substitutions can also be conservative, in this case, the conservative amino acid substitutions are herein defined as exchanges within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gln
IV. Large, aromatic residues: Phe, Tyr, Trp
V. Large, aliphatic, nonpolar residues: Met, Leu, Ile, Val, Cys.

"Homology" refers to the percent identity between two polynucleotide or two polypeptide moieties. Two nucleic acid, or two polypeptide sequences are "substantially homologous" to each other when the sequences exhibit at least about 50% sequence identity, preferably at least about 75% sequence identity, more preferably at least about 80% or at least about 85% sequence identity, more preferably at least about 90% sequence identity, and most preferably at least about 95%-98% sequence identity over a defined length of the molecules. As used herein, substantially homologous also refers to sequences showing complete identity to the specified sequence.

In general, "identity" refers to an exact nucleotide to nucleotide or amino acid to amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100.

Alternatively, homology can be determined by readily available computer programs or by hybridization of polynucleotides under conditions which form stable duplexes between homologous regions, followed by digestion with single stranded specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art.

In some aspects, the invention relates to the composition for use according to the invention, wherein the alphal-antitrypsin fragment is a C-terminal sequence fragment, or any combination thereof.

The peptidic variants may be linear peptides or cyclic peptides and may be selected from the group comprising short cyclic peptides derived from the C-terminal sequence as set forth in SEQ ID No. 2. Preferably, the short cyclic peptides derived from the C-terminal sequence of Alphal-Antitrypsin will be selected from the non-limiting group comprising Cyclo-(CPFVFLM)-SH, Cyclo-(CPFVFLE)-SH, Cyclo-(CPFVFLR)-SH, and Cyclo-(CPEVFLM)-SH, or any combination thereof.

As used herein, an "isoform" of an AAT protein, peptide or polypeptide of the invention refers to a splice variant resulting from alternative splicing of the AAT mRNA.

In some aspects, the amino acid sequence of AAT, the variant, isoform or fragment thereof, as described herein, is at least 80% identical to the corresponding amino acid sequence in SEQ ID NO: 1. In some aspects, the amino acid sequence of AAT, the variant, isoform or fragment thereof is 80%, 81%, 82%, 83%, 84%, 85%, 86%, 87%, 88%, 89%, 90%, 91%, 92%, 93%, 94%, 95%, 96%, 97%, 98%, or 99% identical to a corresponding amino acid sequence in SEQ ID NO: 1.

The peptide, isoform, fragment or variant thereof may preferably be conjugated to an agent that increases the accumulation of the peptide, isoform, fragment or variant thereof in the target cell, preferably a cell of the respiratory tract. Such an agent can be a compound which induces receptor mediated endocytosis such as for example the membrane transferrin receptor mediated endocytosis of transferrin conjugated to therapeutic drugs (Qian Z. M. et al., "Targeted drug delivery via the transferrin receptor-mediated endocytosis pathway" Pharmacological Reviews, 54, 561, 2002) or a cell membrane permeable carrier which can, be selected e. g. among the group of fatty acids such as decanoic acid, myristic acid and stearic acid, which have already been used for intracellular delivery of peptide inhibitors of protein kinase C (Ioannides C.G. et al., "Inhibition of IL-2 receptor induction and IL-2 production in the human leukemic cell line Jurkat by a novel peptide inhibitor of protein kinase C" Cell Immunol., 131, 242, 1990) and protein-tyrosine phosphatase (Kole H.K. et al., "A peptide-based protein-tyrosine phosphatase inhibitor specifically enhances insulin receptor function in intact cells" J. Biol. Chem. 271, 14302, 1996) or among peptides. Preferably, cell membrane permeable carriers are used. More preferably a cell membrane permeable carrier peptide is used. In case the cell membrane permeable carrier is a peptide then it will preferably be a positively charged amino acid rich peptide.

Preferably such positively charged amino acid rich peptide is an arginine rich peptide. It has been shown in Futaki et al. (Futaki S. et al., "Arginine-rich peptides. An abundant source of membrane-permeable peptides having potential as carriers for intracellular protein delivery" J. Biol. Chem., 276, 5836, 2001), that the number of arginine residues in a cell membrane permeable carrier peptide has a significant influence on the method of internalization and that there seems to be an optimal number of arginine residues for the internalization, preferably they contain more than 6 arginines, more preferably they contain 9 arginines. An arginine rich peptide comprises preferably at least 6 arginines, more preferably at least 9 arginines.

The peptide, isoform, fragment or variant thereof may be conjugated to the cell membrane permeable carrier by a spacer (e.g. two glycine residues). In this case, the cell membrane permeable carrier is preferably a peptide.

Usually arginine rich peptides are selected from the non-limiting group comprising the HIV-TAT 48-57 peptide (GRKKRRQRRR; SEQ ID NO. 7), the FHV-coat 35-49 peptide (RRRRNRTRRNRRRVR; SEQ ID NO. 8), the HTLV-II Rex 4-16 peptide (TRRQRTRRARRNR; SEQ ID NO. 9) and the BMV gag 7-25 peptide (SEQ ID NO. 10).

Any cell membrane permeable carrier can be used as determined by the skilled artisan.

Since an inherent problem with native peptides (in L-form) is degradation by natural proteases, the peptide, isoform, fragment or variant thereof as well as the cell membrane permeable peptide, of the invention may be prepared to include D-forms and/or "retro-inverso isomers" of the peptide. In this case, retro-inverso isomers of fragments and variants of the peptide, as well as of the cell membrane permeable peptide, of the invention are prepared.

The peptide, isoform, fragment or variant thereof, optionally conjugated to an agent which increases the accumulation of the peptide in a cell can be prepared by a variety of methods and techniques known in the art such as for example chemical synthesis or recombinant techniques as described in Maniatis et al. 1982, Molecular Cloning, A laboratory Manual, Cold Spring Harbor Laboratory.

The peptide, isoform, fragment or variant thereof, optionally conjugated to an agent which increases the accumulation of the peptide in a cell as described herein are preferably produced, recombinantly, in a cell expression system. A wide variety of unicellular host cells are useful in expressing the DNA sequences of this invention. These hosts may include well known eukaryotic and prokaryotic hosts, such as strains of E. coli, Pseudomonas, Bacillus, Streptomyces, fungi such as yeasts, and animal cells, such as CHO, YB/20, NSO, SP2/0, R1. 1, B-W and L-M cells, African Green Monkey kidney cells (e. g., COS 1, COS 7, BSCl, BSC40, and BMT1O), insect cells (e. g., Sf9), and human cells and plant cells in tissue culture.

By "therapeutically effective dose or amount" of an Alphal-Antitrypsin protein, a variant, an isoform and/or a fragment thereof is intended an amount that when administered brings about a positive therapeutic or prophylactic response with respect to treatment of a subject for a disease or syndrome related to a coronavirus infection.

The term "coronavirus infection" can refer to an infection caused by a coronavirus selected from group comprising MERS-CoV, SARS-CoV and SARS-CoV-2 as well as any variant thereof. In some aspects, the SARS-CoV-2 variant described herein is a SARS-CoV-2 variant selected from the group of Lineage B.1.1.207, Lineage B.1.1.7, Cluster 5, 501.V2 variant, Lineage P.1, Lineage B.1.429 / CAL.20C, Lineage B.1.427, Lineage B.1.526, Lineage B.1.525, Lineage B.1.1.317, Lineage B.1.1.318, Lineage B.1.351, Lineage B.1.617 and Lineage P.3. In some aspects, the SARS-CoV-2 variant described herein is a SARS-CoV-2 variant described by a Nextstrain clade selected from the group 19A, 20A, 20C, 20G, 20H, 20B, 20D, 20F, 20I, and 20E. In some aspects, the SARS-CoV-2 virus described herein is a SARS-CoV-2 variant comprising at least one mutation selected from the group of D614G, E484K, N501Y, S477G/N, P681H, E484Q, L452R and P614R. In some aspects, the SARS-CoV-2 variant described herein is a SARS-CoV-2 variant derived from the variants described herein. In some aspects, the SARS-CoV-2 virus described herein is a SARS-CoV-2 variant having an at least 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% sequence identity to the viral genome sequence of at last one SARS-CoV-2 variant described herein.

The coronavirus infection can cause a respiratory tract infection resulting in a disease or syndrome that is a respiratory syndrome. The respiratory syndrome can be a severe acute respiratory syndrome (SARS). In some aspects, the SARS-CoV-2 infection is at least one of the three clinical courses of infections can be distinguished: (1) mild illness with upper respiratory tract manifestations, (2) non-life-threatening pneumonia and, (3) severe condition with pneumonia, acute respiratory distress syndrome (ARDS), severe systemic inflammation, organ failures, cardiovascular complications.

The composition for use of the invention may further comprise one or more pharmaceutically acceptable diluent or carrier.

"Pharmaceutically acceptable diluent or carrier" means a carrier or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable diluent or carrier include starch, glucose, lactose, sucrose, sodium stearate, glycerol monostearate, talc, sodium chloride, dried skim milk, glycerol, propylene glycol, water, ethanol and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

Alternatively, the pharmaceutical compositions further comprises one or more additional therapeutic agent. Preferably, the one or more therapeutic agent comprises a therapeutically effective amount of one or more nucleoside analog, protease inhibitor, immune-suppressor (e.g. sarilumab or tocilizumab), chloroquine, hydroxychloroquine antibiotic, an antibody directed against structural components of the virus, or fragment thereof (e.g. passive immunotherapy), interferon beta (e.g. interferon beta-1a) and/or a vaccine.

In certain other aspects, the pharmaceutical compositions and the one or more additional therapeutic agent will be administered substantially simultaneously or concurrently. For example, a subject may be given a pharmaceutical composition for use of the invention while undergoing a course of treatment with the one or more additional therapeutic agent. In addition, it is contemplated that the subject has already or may be concurrently receiving other forms of antiviral therapy/ies.

In some aspects, the additional therapeutic agent may be useful to reduce the possible side-effect(s) associated with the administration of an antibody, or an antigen-binding fragment thereof, of the invention.

In some aspects, the additional therapeutic agent may be useful to support the effect associated with the administration of an antibody, or an antigen-binding fragment thereof, of the invention. In some aspects, administration of the additional therapeutic and an antibody, or an antigen-binding fragment thereof, of the invention results in a synergistic effect regarding desired effect and/or side effect.

In some aspects, the additional therapeutic agent described herein is at least one agent selected from the group of nucleoside analog, protease inhibitor and immune modulators such as immune-suppressors.

In some aspects, the additional therapeutic agent described herein is at least one agent selected from the group of nucleoside analog, protease inhibitor, immune-suppressor (e.g. sarilumab or tocilizumab), chloroquine, hydroxychloroquine antibiotic, an antibody directed against structural components of the virus, or fragment thereof (e.g. passive immunotherapy), interferon beta (e.g. interferon beta-1a) and/or a vaccine.

Non-limiting examples of a nucleoside analog comprise Ribavirin, Remdesivir, β-d-N4-hydroxycytidine, BCX4430, Gemcitabine hydrochloride, 6-Azauridine, Mizoribine, Acyclovir fleximer, and a combination of one or more thereof.

Non-limiting examples of protease inhibitor comprise HIV and/or HCV protease inhibitor.

In some aspects, the immune modulator described herein is interferon beta. In some aspects, the immune modulator described herein is interferon beta-1a. Non-limiting examples of immune-suppressor comprise interleukin inhibitors, such as for example IL-6 (e.g. sarilumab or tocilizumab), IL-1, IL-12, IL-18 and TNF-alpha inhibitors.

The additional therapeutic agents may improve or complement the therapeutic effect of compositions and methods described herein (see e.g. Figure 20, 11d).

Accordingly, the invention is at least in part based on the finding that certain combinations (such as IFN-beta-1 a with AAT) improve the effect of AAT on viral entry and inflammation. The present invention also contemplates a gene delivery vector and pharmaceutical compositions containing the same. Preferably, the gene delivery vector is in the form of a plasmid or a vector that comprises one or more nucleic acid encoding the AAT protein, a variant, an isoform and/or a fragment thereof of the invention. Examples of gene delivery vectors comprise e.g., viral vectors, non-viral vectors, particulate carriers, and liposomes. The gene deliver is preferably performed *in vitro* or *ex vivo.*

Accordingly, the invention is at least in part based on the finding that AAT gene delivery (gene therapy) reduce the effect of inflammation (see e.g. Figure 19b).

In an aspect, said viral vector is a vector suited for ex-vivo and in-vivo gene delivery, preferably for ex vivo gene delivery. More preferably, the viral vector is selected from the group comprising an adeno-associated virus (AAV) and a lentivirus, e.g. Lentivirus of 1st, 2nd, and 3rd generation, not excluing other viral vectors such as adenoviral vector, herpes virus vectors, etc. Other means of delivery or vehicles are known (such as yeast systems, microvesicles, gene guns/means of attaching vectors to gold nanoparticles) and are provided, in some aspects, one or more of the viral or plasmid vectors may be delivered via liposomes, nanoparticles, exosomes, microvesicles, or a gene-gun.

In other aspects, the pharmaceutical composition(s) is/are a sustained-release formulation, or a formulation that is administered using a sustained-release device. Such devices are well known in the art, and include, for example, transdermal patches, and miniature implantable pumps that can provide for drug delivery over time in a continuous, steady-state fashion at a variety of doses to achieve a sustained-release effect with a non-sustained-release pharmaceutical composition.

The pharmaceutical compositions may be administered to a subject by different routes including orally, parenterally, sublingually, transdermally, rectally, transmucosally, topically, via inhalation, via buccal administration, intrapleurally, intravenous, intraarterial, intraperitoneal, subcutaneous, intramuscular, intranasal intrathecal, and intraarticular or combinations thereof. For human use, the composition may be administered as a suitably acceptable formulation in accordance with normal human practice. The skilled artisan will readily determine the dosing regimen and route of administration that is most appropriate for a particular patient. The compositions of the invention may be administered by traditional syringes, needleless injection devices, "microprojectile bombardment gone guns", or other physical methods such as electroporation ("EP"), "hydrodynamic method", or ultrasound. The composition can also be administered by intravenous injection, intravenous infusion, infusion with a dosator pump, inhalation nasal-spray, eye-drops, skin-patches, slow release formulations, *ex vivo* gene therapy or ex vivo cell-therapy, preferably by intravenous injection.

The pharmaceutical compositions may also be delivered to the patient, by several technologies including DNA injection of nucleic acid encoding the AAT protein, a variant, an isoform and/or a fragment thereof of the invention (also referred to as DNA vaccination) with and without in vivo electroporation, liposome mediated, nanoparticle facilitated, recombinant vectors such as recombinant lentivirus, recombinant adenovirus, and recombinant adenovirus associated virus sa described herein.

The compositions may be injected intra veniously or locally injected in the lung or respiratory tract or electroporated in the tissue of interest.

Disclosed are methods of treatment and/or prevention of a disease or syndrome related to a coronavirus infection in a subject in need thereof, the method comprising administering a therapeutically effective amount of i) an Alpha1-Antitrypsin protein, a variant, an isoform and/or a fragment thereof as described herein, or of ii) a pharmaceutical composition for use as described herein.

Further provided are methods of modulating onset of coronavirus infection in a subject exposed or suspected of being exposed to coronavirus comprising, administering to the subject in need of such a treatment a therapeutically effective amount of i) an Alphal-Antitrypsin protein, a variant, an isoform and/or a fragment thereof as described herein, or of ii) a pharmaceutical composition for use as described herein.

The present invention also related to a for determining the susceptibility of a subject of interest for treatment and/or prevention of a disease or syndrome caused by a coronavirus infection, more preferably a SARS-CoV-2 infection using a composition comprising a therapeutically effective amount of an alphal -antitrypsin (AAT) protein, a variant, an isoform and/or a fragment thereof as defined in the claims, comprising the steps of:
a) determining the level of at least one of the group consisting of endogenous alpha1-antitrypsin, at least one spike protein priming protease, ACE2 receptor and interferon-gamma in the subject of interest prior to the coronavirus infection, more preferably a SARS-CoV-2 infection or during the infection, more preferably a SARS-CoV-2 infection,
b) determining the level of at least one of the group consisting of endogenous alphal-antitrypsin, at least one spike protein priming protease, ACE2 receptor and interferon-gamma in at least one reference subject during the coronavirus infection, more preferably a SARS-CoV-2 infection, wherein the reference subject is asymptomatic or has mild symptoms,
c) comparing the level of interest determined in step a) to the reference level determined in step b),
wherein the subject of interest is more susceptible for treatment and/or prevention of a disease or syndrome caused by the coronavirus infection, more preferably a SARS-CoV-2 infection if the subject of interest has at least one selected from the group consisting of:
1. a lower level of interest of endogenous alpha-antitrypsin (AAT) compared to the reference level of endogenous AAT,
2. a higher level of interest of at least one spike protein priming protease compared to the reference level of at least one spike protein priming protease,
3. a higher level of interest of angiotensin converting enzyme 2 (ACE2 receptor) compared to the reference level of the ACE2 receptor and
4. a higher level of interest of interferon-gamma (IFN-y) compared to the reference level of IFN-γ.

All definitions and combinations provided herein apply to this aspect, if applicable and unless indicated otherwise.

The present invention further relates to a method for determining the therapeutically effective amount of alpha1-antitrypsin (AAT) for an effective treatment and/or prevention of a disease or syndrome caused by a coronavirus infection, more preferably a SARS-CoV-2 infection using the composition for use of the present invention comprising the steps of:
a) determining the level of endogenous alphal-antitrypsin in a subject of interest prior to the coronavirus infection, more preferably a SARS-CoV-2 infection or during the coronavirus infection, more preferably a SARS-CoV-2 infection,
b) determining the amount of AAT in the composition, which is required to achieve a level of AAT in the subject of at least 10 µM, preferably at least 20 µM, more preferably at least 50 µM, even more preferably at least 100 µM, and most preferably at least 200 µM.

All definitions and combinations provided herein apply to this aspect, if applicable and unless indicated otherwise.

All definitions and combinations provided herein apply to these aspects, if applicable and unless indicated otherwise.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification.

The foregoing description will be more fully understood with reference to the following Examples.

### FIGURE LEGENDS

**Figure 1****:** Schematic illustration of the SARS-CoV-2 infection cycle and AAT's role in blocking viral-entry into the host's cell.
**Figure 2(a):** Endogenous mRNA levels of Cathepsin L, Furin, ACE2 and TMPRSS2 in A549 and HeLa cells, show that neither HeLa cells, nor A549 cells express relevant amounts of ACE2 mRNA nor TMPRSS2. Both cell lines express Furin and Cathepsin L (a priming protease of spike protein from SARS-CoV-1 and SARS-CoV-2).
**Figure 2(b):** SARS-CoV-2 Spike D614G pseudoviral variant shows increased infectivity. Lentiviruses expressing Luciferase and pseudotyped with SARS-CoV-2- Spike wild type (D614) or D614G mutant (G614) were used to transduce HeLa cells overexpressing or not either ACE2 and/or TMPRSS2. Results show no inhibitory effect by plasma derived AAT at 10 micromolar (10µM) concentrations on the SARS-CoV-2 pseudoviral cell-entry in HeLa cells overexpressing TMPRSS2 and up to 25% in HeLa cells expressing ACE2 only.

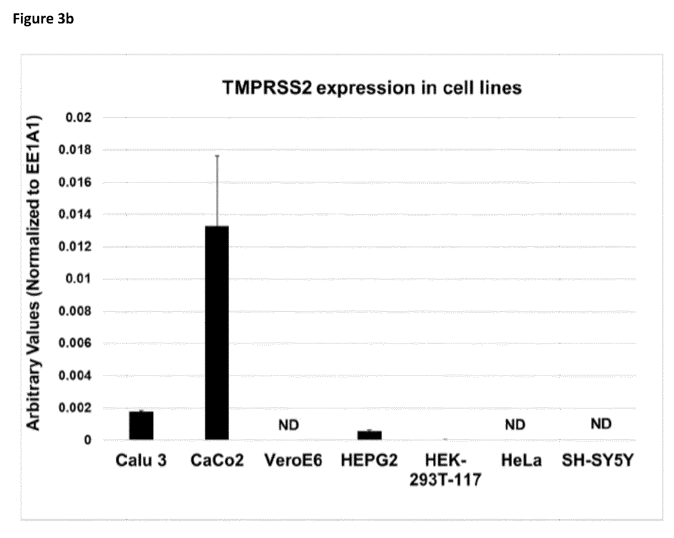

**Figure** 3: a) ACE2 gene expression in different cell lines. b) TMPRSS2 gene expression in different cell lines (tissues).
**Figure** 4: ACE2 expression in A549 in response to treatment with IFN-γ.
**Figure** 5: ACE2 expression in HeLa in response to treatment with IFN-γ.
**Figure** 6: SARS-CoV-2 pseudoviral entry assay. a) to b) A549 cells - WT pseudovirus. a) A549-ACE2 cells with WT D614 pseudovirus. b) A549 - ACE2 - TMPRSS2 cells with WT D614 pseudovirus. c to d) A549 cells - spike G416 mutated pseudovirus. c) A549 ACE2 cells with mutant G416 pseudovirus. d) A549 - ACE2 - TMPRSS2 cells with mutant G614 pseudovirus.
   Respikam = Glassia, FDA approved plasma derived AAT (clinical grade), Sigma = plasma derived AAT bought from Sigma (non-GMP grade).
Figure 7: SARS-CoV-2 pseudoviral entry is blocked by 50-75% in ACE2 enhanced A549 cells at both 100 and 200 micromola concentrations of AAT. (Respikam 100uM in PBS 5x, Sigma 100uM in PBS 5x, Sigma 200uM in PBS 2.5x, Portland 100uM in PBS 5x, Genfaxon in PBS 5x, Camostat 10uM and 100uM in DMSO 0.2%, Bromhexine 10uM and 100uM in DMSO 0.2%, Benzenesulfonyl 10uM and 100uM in DMSO 0.2%).
Figure 8: SARS-CoV-2 pseudoviral entry is blocked by up to 45% in ACE2+TMPRSS2 enhanced A549 cells at both 100 and 200 micromolar concentrations of AAT. (Respikam 100uM in PBS 5x, Sigma 100uM in PBS 5x, Sigma 200uM in PBS 2.5x, Portland 100uM in PBS 5x, Genfaxon in PBS 5x, Camostat 10uM and 100uM in DMSO 0.2%, Bromhexine 10uM and 100uM in DMSO 0.2%, Benzenesulfonyl 10uM and 100uM in DMSO 0.2%).

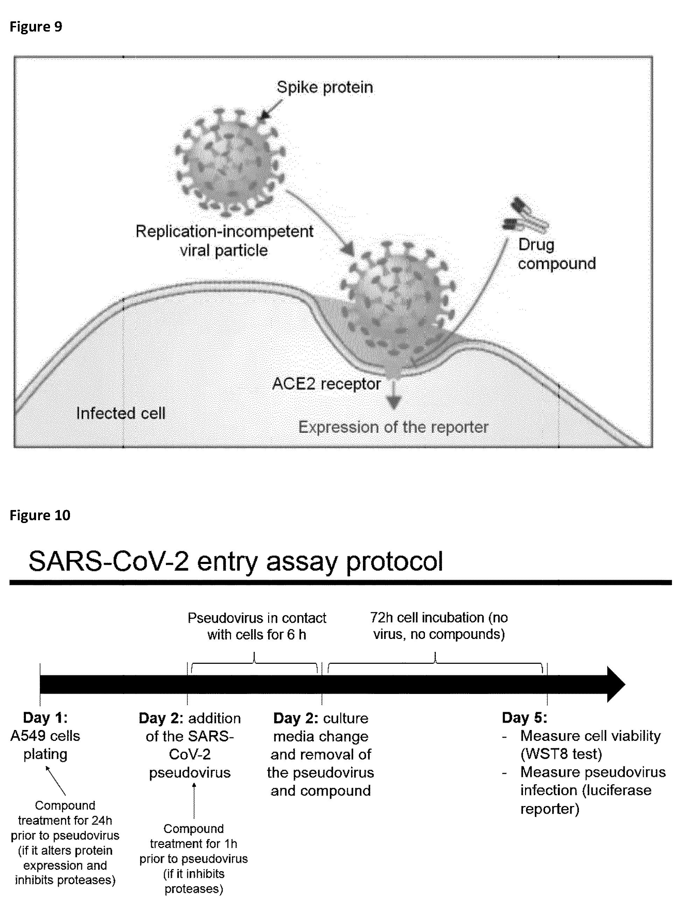

Figure 9 ACE-2 is the host cell receptor responsible for mediating infection by SARS-CoV-2, the novel coronavirus responsible for coronavirus disease 2019 (COVID-19). Treatment with a drug compound (AAT) disrupts the interaction between virus and receptor. (Adapted from https://www.rndsystems.com/resources/articles/ace-2-sars-receptor-identified).
**Figure 10****:** SARS-CoV-2 pseudoviral entry assay protocol.
Figure 11 a) b) Effect of plasma derived and recombinant AAT on SARS-CoV-2 pseudovirus entry in ACE2 overexpressing A549 human lung alveolar basal epithelium cells. Most pronounced inhibitory effect (93.4%) is observed at 200uM for Recombinant AAT 1 produced in CHO cells using vector pXC17.4 (Lonza Biologics Plc) c) Effect of other serine protease inhibitors on SARS-CoV-2 pseudovirus entry in ACE2 overexpressing A549 human lung alveolar basal epithelium cells d) IFN-beta-1a at 10ng/mL in combination with 25uM plasma-derived AAT and recombinant AAT produced in CHO cells (Recombinant AAT 1, Recombinant AAT 2) show an additive inhibitory effect in a SARS-CoV-2 pseudoviral entry assay.

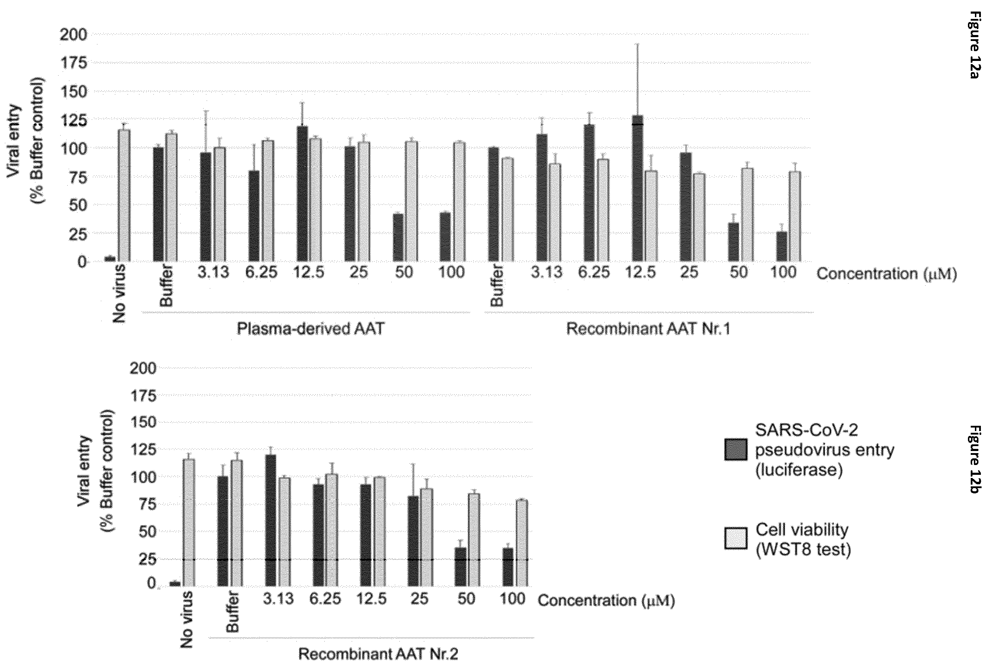

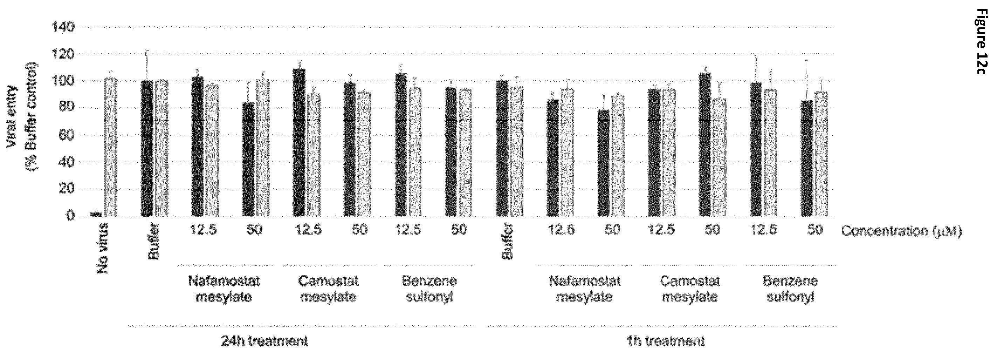

Figure 12 a) b) Effect of plasma derived and recombinant AAT on SARS-CoV-2 pseudovirus entry in ACE2 overexpressing HeLa human cervical cancer cells c) Effect of other serine protease inhibitors on SARS-CoV-2 pseudovirus entry in ACE2 overexpressing HeLa human cervical cancer cells.

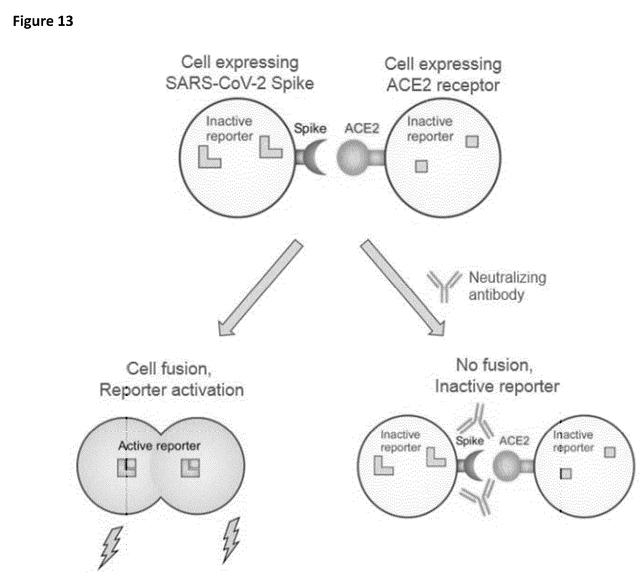

**Figure 13** Schematic illustration of the SARS-CoV-2 spike fusion assay.
**Figure 14** SARS-CoV-2 spike fusion assay in HeLa human cervical cancer cells overexpressing either ACE2 and small bit luciferase or SARS-CoV-2 spike and large bit luciferase. Recombinant AAT (rhAAT) produced in CHO cells (Recombinant AAT 1 and Recombinant AAT 2) shows a better inhibitory effect on SARS-CoV-2 spike mediated cell fusion compared to plasma derived AAT.

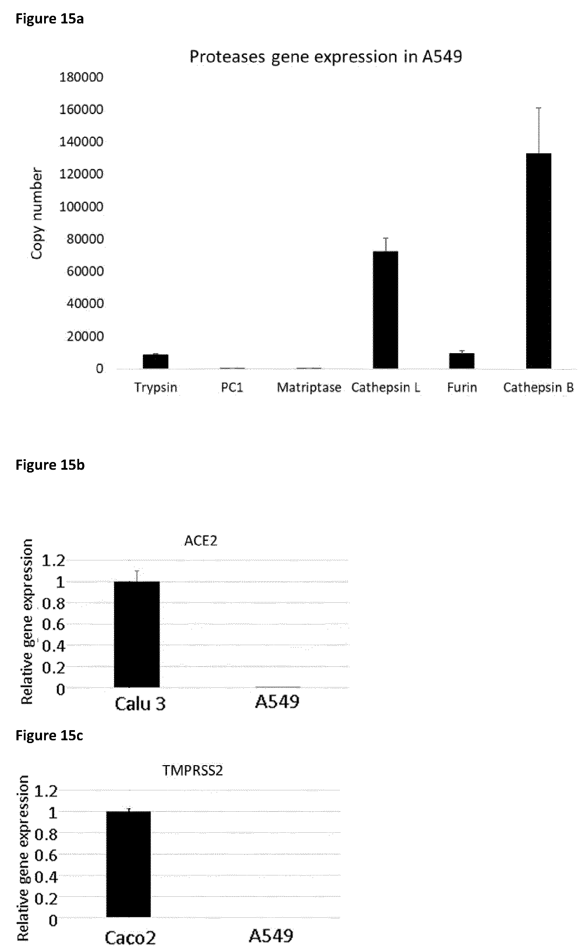

**Figure 15 a)** qPCR analysis of spike protein priming protease gene expression in human lung cell line b) qPCR analysis of ACE2 gene expression in human lung cell line (Calu 3 and A549), shows no endogenous expression of ACE2 in the A549 cell line c) qPCR analysis of TMPRSS2 gene expression in human lung cell line (A549) versus a coloreactal cell line (Caco2), shows no endogenous expression of TMPRSS2 in the A549 cell line d) e) Relative quantative qPCR analysis of spike protein priming protease gene expression in various human cell lines (tissues). Evidently, cells originating from the lungs display the highest copy number for Trypsin and Cathepsin B, in Calu3 and A549 cells, respectively. Notably, cells derived from neural tissue (SH-SY5Y) also display a realtively high copy number for both Trypsin and Cathepsin B.
**Figure 16 a)** AAT and rhAAT inhibits cathepsin B protease activity b) AAT and rhAAT inhibits cathepsin L protease activity, with the most pronounced inhibitory effect observed for recombinant AAT produced in HEK293 cells with vector pcDNA3.1(+) (Recombinant AAT 4) c) AAT and rhAAT inhibits trypsin protease activity, with the most pronounced inhibitory effect observed for recombinant AAT produced in HEK293 cells with vector pcDNA3.1(+) (Recombinant AAT 4) d) AAT and rhAAT inhibits furin protease activity, with the most pronounced inhibitory effect observed for recombinant AAT produced in HEK293 cells with vector pcDNA3.1(+) (Recombinant AAT 4) e) AAT and rhAAT inhibits PC1 protease activity f) AAT and rhAAT inhibits matriptase protease activity g) AAT and rhAAT inhibits TMPRSS2 protease activity h) AAT and rhAAT inhibits elastase protease activity, with the most pronounced inhibitory effect observed for recombinant AAT produced in CHO cells with vector pXC17.4 (Recombinant AAT 1) i) AAT and rhAAT inhibits neutrophil elastase protease activity, with the most pronounced inhibitory effect observed for recombinant AAT produced in HEK293 cells with vector pcDNA3.1(+) (Recombinant AAT 4).
**Figure 17** Standard curve for binding of a) plasma derived AAT and b) recombinant AAT measured using Octet method. Different binding affinity is observed between the plasma derived AAT and the recombinant AAT produced in CHO cells with vectors PL136 and 137.

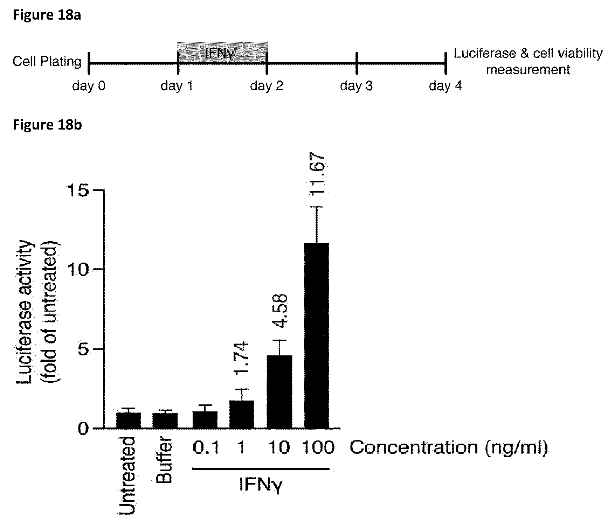

**Figure 18 a)** Schematic illustration of the experimental timeline b) IFNγ-mediated microglial activation (inflammation).
**Figure 19 a)** Schematic illustration of the experimental timeline b) AAT decreases IFNγ-mediated microglial activation (inflammation). Effect is observed for plasma derived AAT, recombinant AAT (rhAA) and for cells transduced with a gene expressing AAT (gene therapy).
**Figure 20a)** Schematic illustration of the experimental timeline b) IFNβ improves the anti-inflammatory effect obtained with AAT.

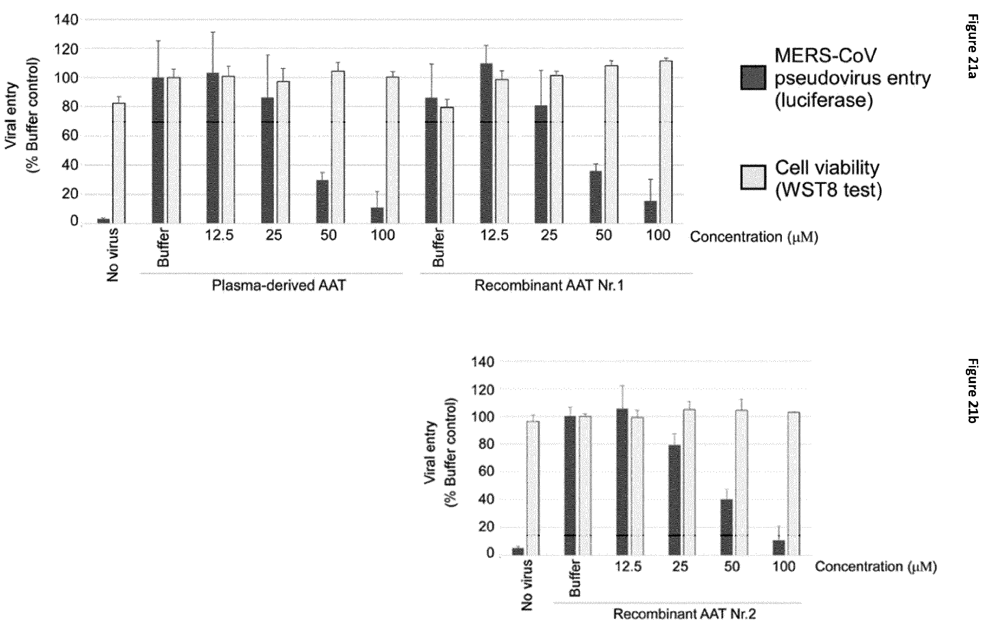

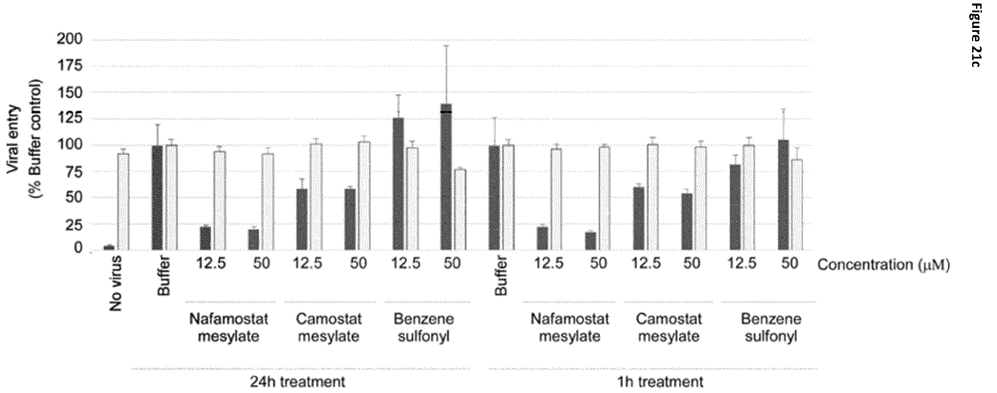

**Figure 21 a) b)** Effect of plasma derived and recombinant AAT on MERS-CoV pseudovirus entry in Caco2 human colorectal adenocarcinoma cells c) Effect of other serine protease inhibitors on MERS-CoV pseudovirus entry in Caco2 human colorectal adenocarcinoma cells.

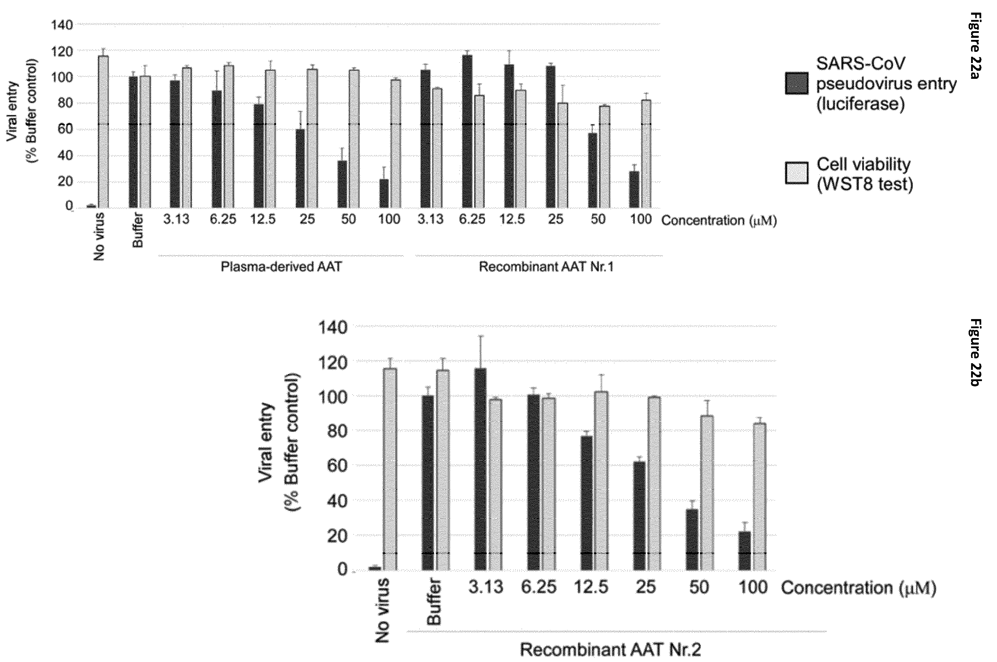

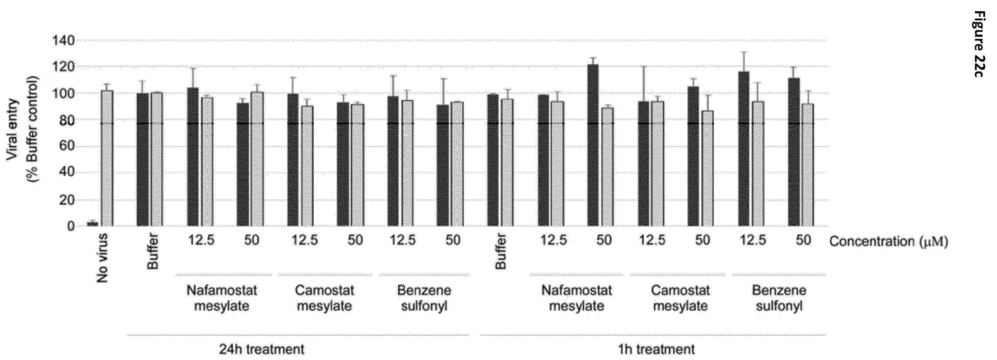

**Figure 22 a) b)** Effect of plasma derived and recombinant AAT on SARS-CoV pseudovirus entry in ACE2 overexpressing A549 human lung alveolar basal epithelium cells c) Effect of other protease inhibitors on SARS-CoV pseudovirus entry in ACE2 overexpressing A549 human lung alveolar basal epithelium cells.

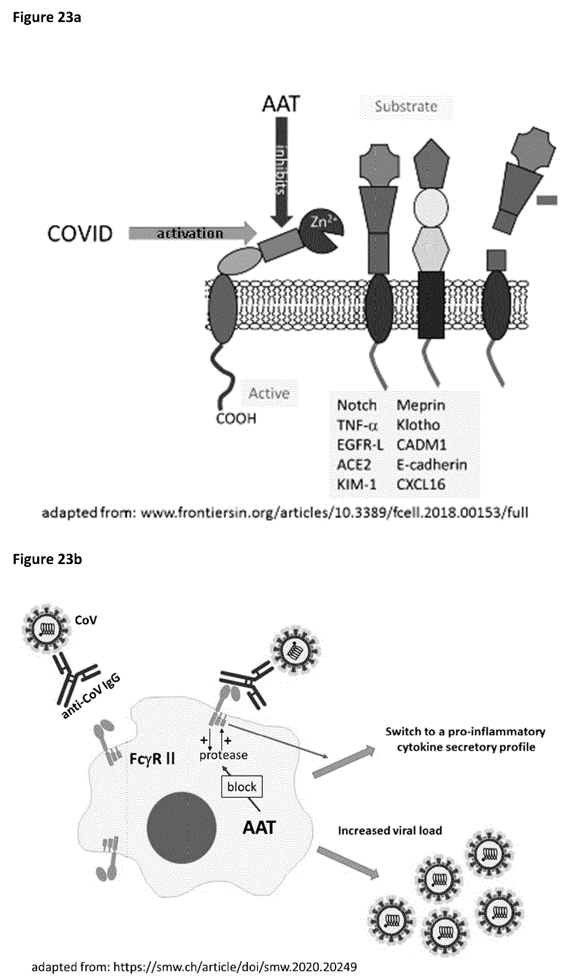

**Figure 23 a)** impact of AAT on ADAM17 activation and protein shedding b) impact of AAT on activation of monocyte/macrophages by spike protein/IgG immune complexes.
**Figure 24** Differences in molecular weight of AAT from different sources.

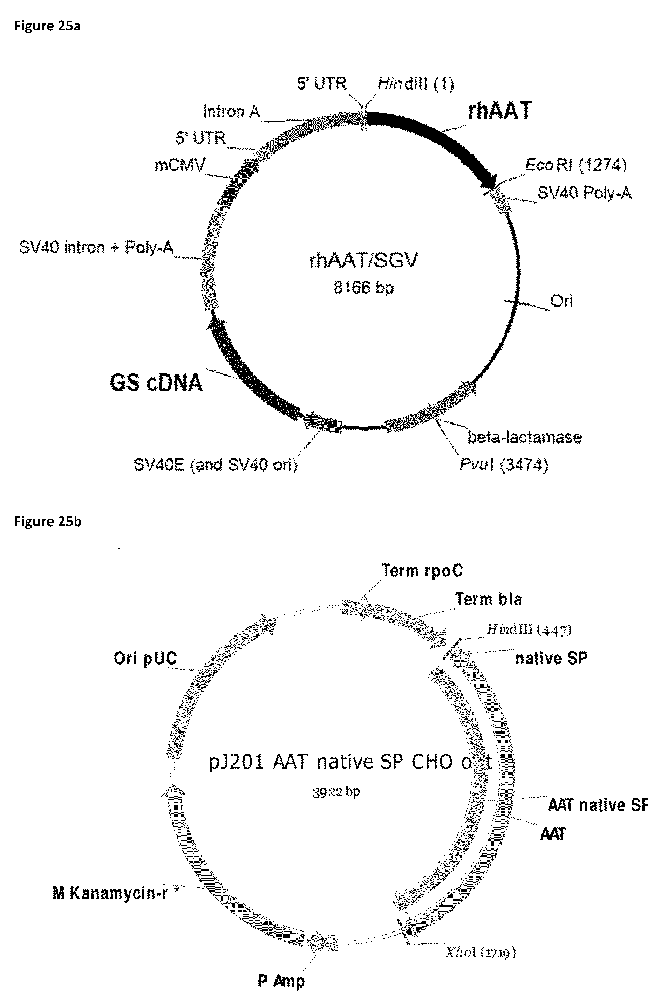

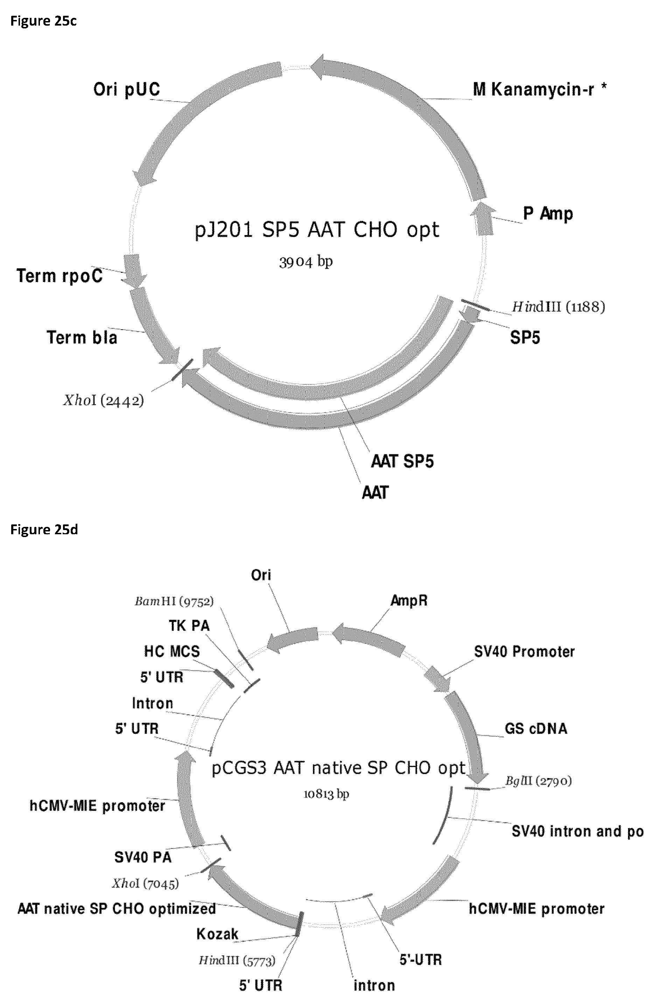

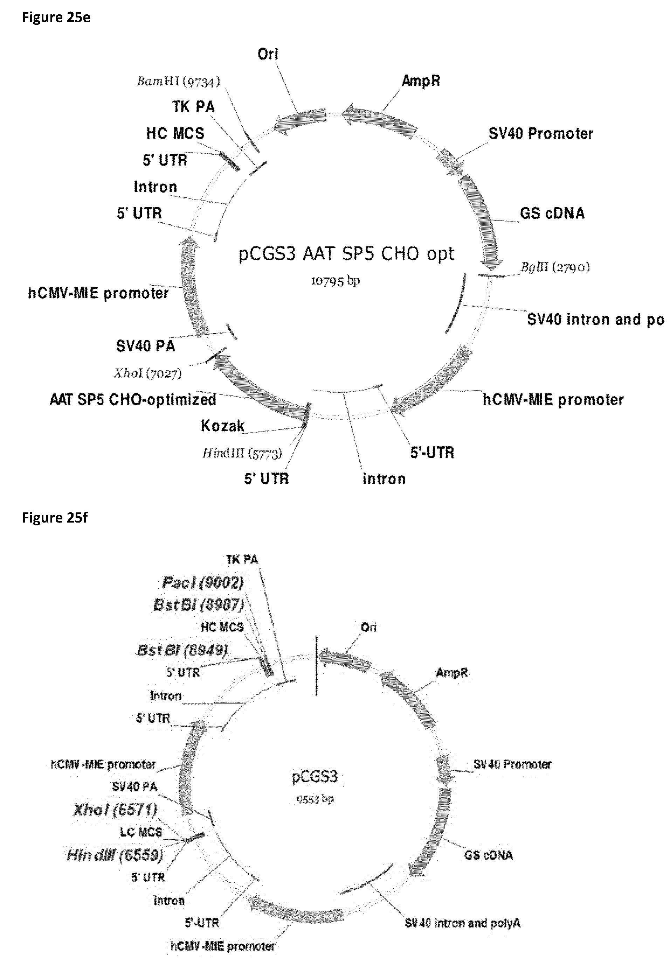

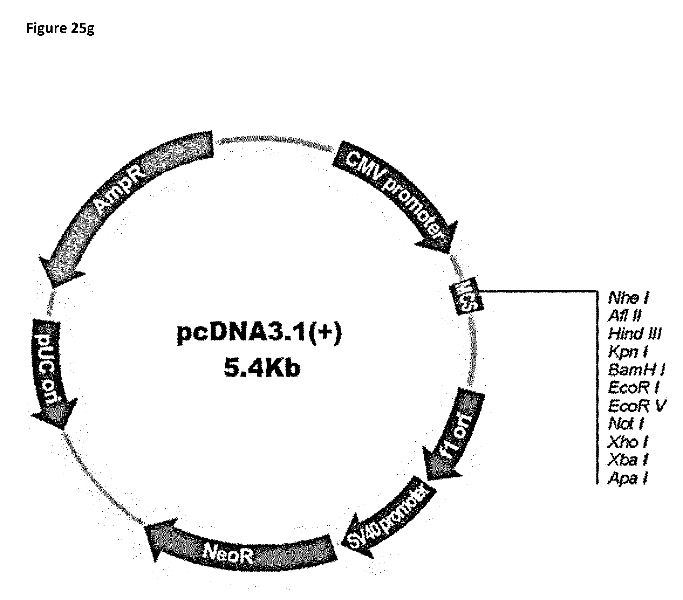

**Figure 25 a)** Vector map for pXC-17.4 (Lonza Biologics) b) Vector map for pJ201_AAT native_SP (Merck) c) Vector map for pJ201_AAT_SP5 (Merck) d) Vector map for PL136 (Merck) e) Vector map for PL137 (Merck) f) Vector map for empty pCGS3 vector g) Vector map for pcDNA3.1(+) (GenScript).

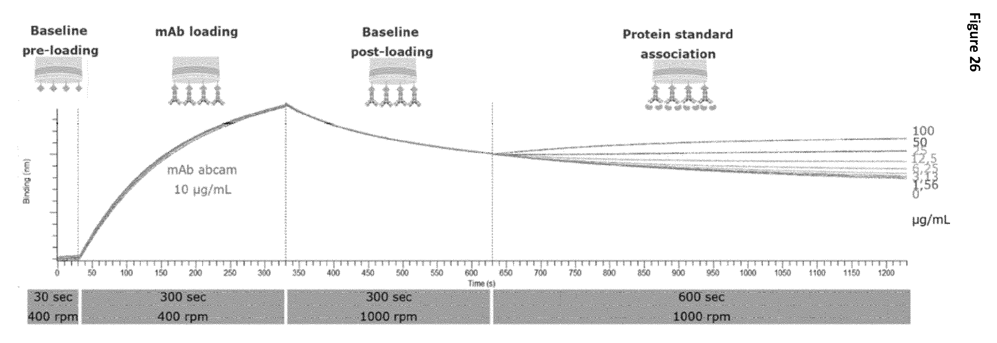

**Figure 26** Octet method developed for the detection of AAT affinity.

All Figures: "Respikam" refers to Plasma derived AAT (clinical-grade); "Sigma" refers to Plasma derived AAT; "Recombinant AAT 1" refers to AAT produced in CHO by pXC-17.4 (GS System, Lonza), "Recombinant AAT 2" refers to AAT produced in CHO by PL136/PL137 (Merck), "Recombinant AAT 3" refers to AAT produced in HEK293T; "Recombinant AAT 4" refers to AAT produced in HEK293 by pcDNA3.1(+) (GenScript).

### EXAMPLES

### Materials & Methods

### Treatment of SARS-CoV-2 infection by Alphal-Antitrypsin (AAT)

The impact of AAT on SARS-CoV-2 infection is shown on two levels:
1. First, the protease-dependent entry of SARS-CoV-2 into cells
2. Second the overshooting inflammation in severe SARS-CoV-2 disease

Importantly, this represents three stages of the COVID-19 (i.e. the disease caused by SARS-CoV-2):
1. First stage COVID-19 (disease caused by SARS-CoV-2, flue-like symptoms, usually disappearing within one week) does not require treatment.
2. Second stage disease, typically characterized by viral pneumonia, requires hospital and an antiviral treatment (i.e. inhibition of SARS-CoV-2 entry into cells) is of major interest at this stage to prevent further progression.
3. Third stage COVID disease is characterized by acute respiratory distress syndrome (ARDS) and severe systemic inflammation; this stage requires an anti-inflammatory and antiviral treatment.

According to our analysis, AAT is useful for the treatment of stage II and stage III COVID-19 whereas prophylactic administration of AAT is envisaged in order to prevent the development of stage I into stage II and III.

### Example 1 - Entry of SARS-CoV-2 into cells

The entry of SARS-CoV-2 is mediated by the binding of the viral spike protein with the host cell Angiotensin converting enzyme 2 (ACE2) receptor. To recapitulate *in-vitro* this biological event, lentivectors coding for a reporter (GFP or luciferase) and expressing the spike protein on its surface are generated. The cellular entry of the lentivector is mediated by the SARS-CoV-2 mechanism, the efficiency of which is measured by the expression of the reporter (GFP or luciferase) in the target cells (Ou, X., Liu, Y., Lei, X. et al. Characterization of spike glycoprotein of SARS-CoV-2 on virus entry and its immune cross-reactivity with SARS-CoV. Nat Commun 11, 1620, 2020). AAT is added to quantify inhibition on viral entry by reading out the GFP and/or luciferase signals.

The transmembrane Serine Protease TMPRSS2 is crucial for SARS-CoV-2 infection (Toshio Hirano, Masaaki Murakami COVID-19: A New Virus, but a Familiar Receptor and Cytokine Release Syndrome Immunity, 22 April 2020) and for Hepatitis C infection (Esumi M, Ishibashi M, Yamaguchi H, Nakajima S, Tai Y, Kikuta S, Sugitani M, Takayama T, Tahara M, Takeda M, WakitaT- Trans-membrane serine protease TMPRSS2 activates hepatitis C virus infection. Hepatology. 2015 Feb; 61(2):437-46). The hepatitis C infection can be blocked by AAT (presumably through TMPRSS2 inhibition-Esumi et al., 2020), SARS-CoV-2 (as well as some highly pathogenic forms of influenza virus) has a sequence that allows cleavage by the protease furin. This cleavage site is not present in the SARS and the MERS coronaviruses, suggesting that it is important for pathogenicity of SARS-CoV-2 (B. Coutard, C. Valle, X. de Lamballerie, B. Canard, N.G. Seidah, E. Decroly, The spike glycoprotein of the new coronavirus 2019-nCoV contains a furin-like cleavage site absent in CoV of the same clade, Antiviral Research, Volume 176, 2020). Alpha1-antitrypsin Portland has a strong and selective activity on furin (Jean F, Stella K, Thomas L, et al. alpha1-Antitrypsin Portland, a bioengineered serpin highly selective for furin: application as an antipathogenic agent. Proc Natl Acad Sci USA. 1998;95(13):7293-7298. doi:10.1073/pnas.95.13.7293). It is, therefore, envisaged that wild type AAT (plasma derived) as well as recombinant (produced in mammalian cells, e.g. CHO and/or HEK cells) would display furin activity.

### Experiments

### 1.1. Impact of AAT on cellular cleavage of recombinant spike protein

Recombinant spike protein is added to relevant cell types (any ACE2 expressing cells and known in the art) and proteolytic cleavage is assessed by Western blotting. Any ACE2 expressing cell-lines can be used. Multiple target cell lines express various amount of the ACE2 receptor, include but are not limited to; Calu-3, SH-SY5Y, HEK, HT1080, A549, MRC 5, Huh7, Vero81, VeroE6, Hela, RS and LLCMK2.

### 1.2. Impact of AAT on entry of pseudoviruses

Pseudoviruses (i.e. pseudotyped viral vectors) that use the SARS-CoV-2 spike protein as viral attachment/fusion protein correctly depict the mechanisms of SARS-CoV-2 into cells. This entry is dependent on two proteolytic steps which are inhibited by the protease inhibitor AAT. Therefore, the entry of pseudo-viruses in a variety of different cell lines, preferably ACE2 expressing cell-lines are investigated.

### 1.3. Impact of AAT on cellular infection by active SARS-CoV-2 (live virus tests):

AAT is tested on live SARS-CoV-2 virus manipulated under biosecurity level 3 in accordance with OFSP recommendations. The virus is inoculated *in-vitro* on cells treated with AAT, or any fragment, variant and isoform thereof as multiple parameters are measured to assess efficiency including reduced cytotoxicity and viral titer. More specifically, cellular infection with SARS-CoV-2 is assessed by immunofluorescence with viral proteins as well as quantification of cell death. Since SARS-CoV-2 infect neural tissue (Helms J, Kremer S, Merdji H, et al. Neurologic Features in Severe SARS-CoV-2 Infection [published online ahead of print, 2020 Apr 15]. N Engl J Med. 2020; Pleasure SJ, Green AJ, Josephson SA. The Spectrum of Neurologic Disease in the Severe Acute Respiratory Syndrome Coronavirus 2 Pandemic Infection: Neurologists Move to the Frontlines. JAMA Neurol. Published online April 10, 2020), infection of neural tissue (e.g. Minibrain^{™}) as well as neuroblastoma cells (SH-SY5Y) will be performed as co-treatment with AAT and is expected to modify viral cytotoxicity or tropism.

**Example** 2 - **Overshooting inflammation** in **severe SARS-CoV-2 disease also known as "Cytokine** Storm", Fu Y, Cheng Y, Wu Y. Understanding SARS-CoV-2-Mediated Inflammatory Responses: From Mechanisms to Potential Therapeutic Tools [published online ahead of print, 2020 Mar 3]. Virol Sin. 2020;1-6. doi:10.1007/s12250-020-00207-4):

| **Mechanism of SARS-CoV-2 induced inflammation⁹** | **Activity of AAT** |
|---|---|
| Inflammation Caused by Rapid Viral | inhibition of viral entry |
| Replication and Cellular Damage | |
| Inflammation Caused by Virus-Induced ACE2 Downregulation and Shedding | inhibition of ADAM17 |
| Inflammatory Responses Induced by Anti-Spike IgG (Anti-S-IgG) | Proteolysis was shown to enhance also monocyte FcyRJI-mediated functions, such as antibody-dependent cellular cytotoxicity and induction of TNF-cu release. |

### Experiments

2.1. SARS-CoV-2 activation of ADAM17 (TACE): ADAM17 activation part of SARS-CoV-2 pathology (see, e.g. Vanesa Palau, Marta Riera, Maria José Soler, ADAM17 inhibition may exert a protective effect on COVID-19, Nephrology Dialysis Transplantation, gfaa093, https://doi.org/10.1093/ndt/gfaa093; https://clinicalaffairs.umn.edu/umn-research/regulation-sars-cov-2-receptor-ace2-adam17; Haga S, Yamamoto N, Nakai-Murakami C, et al. Modulation of TNF-alpha-converting enzyme by the spike protein of SARS-CoV and ACE2 induces TNF-alpha production and facilitates viral entry. Proc Natl Acad Sci US A. 2008;105(22):7809-7814. doi:10.1073/pnas.0711241105). AAT inhibits ADAM17 (see, e.g. Bergin DA, Reeves EP, Meleady P, et al. α-1 Antitrypsin regulates human neutrophil chemotaxis induced by soluble immune complexes and IL-8. J Clin Invest. 2010;120(12):4236-4250. doi:10.1172/JCI41196; Serban KA, Petrusca DN, Mikosz A, et al. Alpha-1 antitrypsin supplementation improves alveolar macrophages efferocytosis and phagocytosis following cigarette smoke exposure. PLoS One. 2017;12(4):e0176073. Published 2017 Apr 27. doi:10.1371/journal.pone.0176073).

Investigate the impact of AAT on ADAM17 activation and protein shedding (in particular ACE2) after exposure of cells to (Figure 23a):
i) spike protein
ii) pseudovirus
iii) SARS-CoV-2 (live virus).

Most relevant cells to be used are any ACE2 expressing cells (as listed above).

2.2. Activation of Fc receptors by SARS-CoV-2/IgG immune complexes (antibody-dependent enhancement see e.g. F. Negro, Swiss Med Wkly. 2020;150:w20249 "Is antibody-dependent enhancement playing a role in COVID-19 pathogenesis?"). Proteolysis through trypsin-like proteases enhances monocyte FcyRJI-mediated functions, such induction of TNF release (J M Debets, J G Van de Winkel, J L Ceuppens, I E Dieteren, W A Buurman in The Journal of Immunology February 15, 1990, 144 (4) 1304-1310).

Investigate impact of AAT on activation of monocyte/macrophages by spike protein/IgG immune complexes (Figure 23b).

### Example 3 - Additional experiments in support of AAT efficacy in decreasing viral proliferation

### 3. Viral polymerase assay

The SARS-CoV-2 viral polymerase is a key element in the replication of the viral genetic material. A cell-line cell line expressing the multi-subunit viral polymerase (constituted of at least the 3 core subunits NSP7, NSP8 and NSP12) is transfected to also overexpress a luciferase reporter which can only be activated by the viral polymerase activity.

The luciferase reporter signal is proportional to the viral polymerase activity. AAT is tested to assess viral polymerase inhibition activity.

Upon entry in the host cell, SARS-CoV-2 will use the host cell machinery to enhance its own replication. This will induce a toxic response on the host cell, essentially mediated by the NSP1 viral protein.

To mimic this biological event a cell-line expressing the viral NSP1 protein controlled by a tetracycline-inducible promoter is generated. Following the cells' treatment with tetracycline the toxic NSP1 protein will be expressed. Addition of AAT is presumed to inhibit this toxic effect mediated by the viral protein.

### Experiment 4

### Introduction

The recent pandemic caused by the newly emerged SARS-CoV-2 Coronavirus took the world by surprise. As opposed to previous Coronavirus outbreaks such as SARS and MERS, the new virus is not characterized by a particularly high case-fatality rate, but rather by an unprecedented epidemiological success. And indeed, the virus has so far largely resisted to all attempts of eradication, despite stringent measures taken in many countries around the globe. SARS-CoV-2 is an RNA virus, but Coronaviruses differ from other RNA viruses in several respects. They have a large genome and a sophisticated life cycle which is still poorly understood. Also, coronaviruses have a proof-reading machinery, which confers a relatively stable genome, as compared to other types of RNA viruses. And indeed, given the size of the pandemic, so far relatively few mutations leading to amino acid changes have been reported. The coronavirus S protein, also known as spike, is the dominant protein of the viral envelop. It mediates the attachment of the virus to the host cell surface receptors and subsequent fusion between the viral and host cell membranes to facilitate viral entry into the host cell. It is a trimeric class I fusion protein which exists in a metastable pre-fusion conformation divided in two main subunits, namely S1 and S2. In order to attach to the host cell surface receptors, the receptor binding domain (RBD) contained within the S1 domain undertakes a hinge-like conformational change, defining two different states [1]. This conformational change is required for proteolytic processing and/or fusion of the S2 domain with the host cell membrane. It is now thought that a variety of proteases including PC family of proteases, trypsin-like proteases, and cathepsins are able to cleave the spike protein [2].

The spike protein D614G mutation has received attention as it could potentially alter viral attachment, fusogenicity, and/or immunogenicity. The D614G mutation has been suggested to increase infectivity (ref), transmissibility [3] and/or case fatality rate [4]. More recent studies directly demonstrate that the G614 variant indeed enhances viral entry into cells [5, 6].

SARS-CoV-2 employs a multi-subunit replication/transcription machinery [7]. A set of non-structural proteins (nsp) produced as cleavage products of the ORF1a and ORF1b viral polyproteins assemble to facilitate viral replication and transcription, making this machinery a potential target for therapeutic intervention against COVID-19 viral infections [8]. RNA-dependent RNA polymerase (RdRp, also known as nsp12) is a key player in the synthesis of viral RNA. Recently resolved crystal structure of SARS-CoV-2 nsp12 in complex with its nsp7 and nsp8 co-factors underlines the central role these non-structural proteins have in the replication and transcription cycle of the COVID-19 virus [8, 9]. The P314L mutation of the RdRp has received less attention, however it has been suggested that this mutation might alter viral proof reading and thereby lead to an increased down-stream mutation rate [10].

In this study, we investigate the emergence of a SARS-CoV-2 variant with concomitant mutations in the S protein and the RdR-polymerase. Both mutations are in strategically relevant sites of the respective proteins and hence candidates to alter the biology of the virus. The G624/L323 variant, but not the individual G624 or L323 mutants, is epidemiologically highly successful and over the last months has largely replaced the original D624/P323 variant.

### Methods:

### Cell lines and culture

Human cell lines used in the study were HEK 293T/17 (human kidney, ATC C#CRL-11268), A549, HeLa and HMC3-MHCII^{Luc} cells. HMC3-MHCII^{Luc} cell line coding for luciferase reporter under major histocompatibility complex II promoter (MHCII) has been described as a valuable tool to study human microglial activation by and was obtained from Prof. Karl-Heinz Krause at the University of Geneva. HMC3-MHCII^{Luc} cells were transduced with a lentiviral vector to obtain an AAT-producing HMC3-MHCII^{Luc};Ubi^{AAT} cell line (See Lentivirus production). Cells were maintained in Dulbecco modified Eagle medium (DMEM) supplemented with 10% (v/v) fetal bovine serum (FBS), 100 µg/ml of penicillin and streptomycin, 2 mM 1-glutamine, 1 mM sodium pyruvate and 1% non-essential amino acids. HMC3 cell lines were not added non-essential amino acids. Cultureds were maintained at 37°C in a 5% CO2 atmosphere.

### Spike fusion assay.

Suited concentration of treatments was pre-diluted in culture media. On the top of this mix was added the HeLa cell line stably overexpressing the human ACE2 receptor and the Smallbit split-luciferase fragment together with the HeLa cell line stably overexpressing the SARS-CoV-2 spike protein and the Largebit split-luciferase fragment, and incubated at 27°C for 24h. Interaction between ACE2 and SARS-CoV-2 spike mediates the fusion of both cell line, thus interactions between the largebit and smallbit split luciferase fragments which leads to a functional luciferase. Light emission was measured with the Nanoglo kit (Promega) according to manufacturer instructions.

### Generation of cell lines expressing ACE2 and TMPRSS2.

Hela and A549 cells were plated at a density of 2E05 cells per well in a 12 well plate. The day after cells were co-transduced either with ACE2/puromycin and/or TMPRSS2/blasticidin lentiviruses. Four day after transduction, cells were selected with blasticidin amd puromycin at 5 µg/ml. Cells were maintained as polyclonal population.

### Plasmids

ACE2 and TMPRSS2 cDNA ORFs were purchased from GenSript and were cloned into pCDH-CMV-MCS-EF1α-Puro (SEQ ID NO: 13) and pCDH-CMV-MCS-EF1α-Blast (SEQ ID NO: 14) lentivectors respectively using standard cloning methods. pCG1_SCoV2-S plasmid (SEQ ID NO: *15)* encoding SARS-CoV-2-Spike protein was provided by Prof. Dr. Stefan Pöhlmann (University Göttingen, Göttingen, Germany). G614 Spike was cloned using site-directed mutagenesis with the following primers: 5'-GCGTGAACTGTACCGAAGTG-3' (SEQ ID NO: 16) and 5'-CCTGGTACAGCACTGCC-3' (SEQ ID NO: 17). C-terminal 19 amino acids truncated version of the SARS-CoV-2-Spike was generated by PCR amplification using primers 5'-AGCGAATTCGGATCCGCC-3' (SEQ ID NO: 18) and 5'-ACAGTCGACTCTAGATTAGCAGCAGCTGCCACAG-3' (SEQ ID NO: 19) followed by cloning into pCG1 plasmid.

### AAT purification produced in HEK293T cells

Supernatant from HEK293T cells overexpressing human AAT-His tagged was sampled and incubated overnight at 4°C under shaking with Ni Sepharose excel histidine-tagged protein purification resin (Cytiva). Volume ratio supernatant:resine is 200:1. Then supernatant was discarded, and beads were washed once with phosphate-buffered saline (PBS). Beads were washed once times with PBS supplemented by 10% (v/v) elution buffer (PBS supplemented with 500 mM imidazole, pH 7.5), and once with PBS supplemented with 20% elution buffer. Finally purified AAT-His-tagged was eluted in 100% elution buffer. Imidazole was removed by dialysis to obtain AAT-His in PBS buffer.

### Lentivirus production

For recombinant-lentivirus production, plasmids were transfected in HEK293T cells using the calcium phosphate method. Briefly, 4.5x10⁶ cells were plated in a 10-cm dish and transfected 16h later with 15 µg of either ACE2, TMPRSS2, AAT or empty-expressing lentiviral vectors, 10 µg of packaging plasmid (psPAX2, gift from Didier Trono [Addgene plasmid 12260]) and 5 µg of envelope (pMD2G, gift from Didier Trono [Addgene plasmid 12259]). The medium was changed 8h post-transfection. After 48h, the viral supernatants were collected and filtered using 45 µm PVDF filters and stored at -80°C.

### Coronavirus spike pseudotyped Lentiviral production

Spike-pseudotyped lentivirus were produced by co-transfection 293T cells with psPAX2, pCDH-CMV-Gluc-EF1α- GFP, and plasmids encoding either SARS-CoV-2 Spike D614 Full length (FL) (SEQ ID NO: 15), Spike G614 Full length (FL) (SEQ ID NO: 20), Spike D614 DeltaCter (SEQ ID NO: 21), Spike G614 DeltaCter (SEQ ID NO: 22), SARS-CoV spike (Sino Biological Europe GmbH, Catalog Number: VG40150-G-N), MERS-CoV spike (Sino Biological Europe GmbH, Catalog Number: VG40069-G-N) or empty vector (SEQ ID NO: 23) by using the calcium phosphate method as described above.

### Coronavirus spike pseudotyped Lentiviral Infectivity Assays

HeLa stably overexpressing the human ACE2 receptor, A549 cells stably overexpressing the human ACE2 receptor or Caco2 cells were seeded into 96-well plates. 3 hours after plating, compound treatment was performed, or omitted depending on the experimental setup. 24h later cells were transduced with coronavirus pseudovirus for 6h. Then culture media was changed and cells were kept at 37°C. After three-day incubation, Gaussia luciferase activity was measured by the addition of 10uL cell supernatant to 50uL of phosphate-buffered saline [PBS] supplemented with 4 µM coelenterazine and immediately measuring luminescence in a luminometer (Glomax; Promega). In parallel the cells were trypsinized and resuspended in PBS supplemented with 10% FBS and were analysed by flow cytometry using Attune NxT Flow Cytometer(thermofisher Scientific). Data was analysed using FlowJo 11 (FlowJo, LLC, Ashland, OR).

### Cell free assay (spike protein priming protease inhibition)

The reaction was performed in 100 ul for trypsin (Sigma-Aldrich), 75 ul for cathepsin B (R&D Systems) and 50 ul for PC1 (R&D Systems), matriptase (R&D Systems), furin (NEB), cathepsin L (R&D Systems) and TMPRSS2 (creative biomart). Buffer is composed of PBS pH 7.4 for trypsin (diluted to 6.4nM) ; 25 mM MES, pH 5 for cathepsin B (diluted to 1.76ng/ul) ; 25 mM MES, 5 mM CaCl2, 1% Brij-35, pH 6 for PC1 (diluted to 1.76ng/ul); 50 mM Tris, 50 mM NaCl, 0.01% Tween 20, pH 9 for matriptase (diluted to 1.76 ng/ul); 100 mM HEPES, 1 mM CaCl2, 0.5% Triton X100, 1 mM 2-Mercaptoethanol, pH 7.5 for furin ( diluted to 10U/m1) ; 0.005% Brij-35, 1 mM EDTA, 5 mM DTT, 50 mM MES, pH 6 for cathepsin L (diluted to 1.76ng/ul); 50 mM TrisHCl, 150 mM NaCl, 0.01% tween 20, pH 8 for TMPRSS2 (diluted to 0.5uM). Unless specified all reagents were provided by Sigma Aldrich. For trypsin, cathepsin B, PC1, matriptase, furin, cathepsin L assays, SARS-CoV-2 peptide composed of the sequence TNSPRRARSVA with modification MCA/Lys (DNP) FRET pair (Biomatik) was used. For TMPRSS2 assay, peptide composed of the sequence Boc-QAR-AMC (R&D Systems) was used. For elastase ELA1 from pig pancreas, assay kit (E-12056) was purchased from molecular probes (Sigma-Aldrich) and for neutrophil elastase ELA2, assay kit (BML-AK497) was purchased from Enzo, tests were performed according to manufacturer instructions. Fluorescence or absorbance was measured every minute for 45 minutes in plate reader. Vmax was determined for each condition as fluorescence unit per minute and enzymatic activity is expressed in percentage of untreated condition.

### Gene expression analysis

Total RNA was isolated using RNeasy Micro Kit (Qiagen) according to the manufacturer's instructions. 500ng of total RNA from each sample were reverse transcripted with Primescript kit from Takara in a total volume of 10 µl, at 37°C for 15min. The relative mRNA levels were evaluated by quantitative RT-qPCR using SYBR Green PCR kit (Applied Biosystems) by 2 delta Ct method. GAPDH was used as internal control.

### Gene copy number estimation

For copy number, linearization of each specific plasmid was performed using restriction enzyme digestion. The product of digestion was cleaned using PCR clean up kit (GeneJET) according to manufacturer instructions.Then optical density was measured and copy number determined. A serial dilution of 1/10 from digested plasmid was used for qPCR.

Plasmids for furin, cathepsinL, matriptase (stl4), trypsin (PRSS1), PC1 (PCSK1) were provided by GenScript and plasmid for cathepsin B was provided from Sino Biological.

### Primers

| **Gene** | | **Oligo sequence (5' to 3')** |
|---|---|---|
| Furin | Forward | GGAACATGACAGCTGCAACT (SEQ ID NO: 32) |
| Furin | Reverse | TCGTCACGATCTGCTTCTCA (SEQ ID NO: 33) |
| Cathepsin L | Forward | TAGAGGCACAGTGGACCAAG (SEQ ID NO: 34) |
| Cathepsin L | Reverse | ATGGCCATTGTGAAGCTOTG (SEQ ID NO: 35) |
| Cathepsin B | Forward | TCTCTGACCGGATCTGCATC (SEQ ID NO: 36) |
| Cathepsin B | Reverse | TCACAGGGAGGGATGGAGTA (SEQ ID NO: 37) |
| PC1 | Forward | GCGTGCCTGAGAAGAAAGAG (SEQ ID NO: 38) |
| PC1 | Reverse | ATCCCGTTCTCTTTCAGCCA (SEQ ID NO: 39) |
| Trypsin | Forward | AAGTGTGAAGCCTCCTACCC (SEQ ID NO: 40) |
| Trypsin | Reverse | GGTGTAGACTCCAGGCTTGT (SEQ ID NO: 41) |
| Matriptase | Forward | CCCAACAACCAGCATGTGAA (SEQ ID NO: 42) |
| Matriptase | Reverse | ACTGGAGTCGTAGGAGAGGT (SEQ ID NO: 43) |
| Matriptase | Forward | AGAACGTCCTGCTCATCACA (SEQ ID NO: 44) |
| Matriptase | Reverse | TGTGCAGTCAATGTTGGGTG (SEQ ID NO: 45) |

### Results: Impact of the S protein D614G mutation on cell transduction with pseudotype lentivectors

Our aim was to establish cell lines that consistently express the viral receptor ACE2, as well as relevant proteases able cleave the spike protein. As seen in Figure 3A and B, neither Hela cells, nor A549 cells expressed relevant amounts of ACE2 mRNA. Similarly, mRNA levels of TMPRSS2 were almost undetectable. Both cell types show a low to intermediate gene expression of furin, but relatively high level gene expression of cathepsin L (a protease able to cleave spike protein from both, SARS-CoV-1 and SARS-CoV-2). In order to establish cell lines permissive for spike protein pseudotyped lentivectors, we therefore transduced both Hela cells and A549 cells either individually, or combined with ACE2 (SEQ ID NO: 24) and TMPRSS2(SEQ ID NO: 25).

As expected, exposure of wild type (mock-transduced) Hela cells or A549 cells to the above described lentivectors did not lead to GFP or luciferase expression (Figure 3c-f). Similarly, overexpression of TMPRSS2 by itself did not lead to significant transduction rate. Using the D614 pseudotype lentivector, there was a small, yet clearly detectable transduction of the ACE2 and ACE2/TMPRSS2-expressing Hela cells, but only a very small transduction of A549 cells. This changed markedly when the G614 pseudotype vector was used. There was a much stronger transduction of both ACE2-expressing lines. Note however that transduction of ACE2-expressing HeLa cells was much stronger than the one of ACE2-expressing A549 cells. Conversely, the overexpression of TMRPSS2 in addition to ACE2, lead to strong additional enhancement of transduction, which was not the case for Hela cells. Thus, most likely Hela cells already strongly express a spike cleaving protease, and the overexpression of TMRPSS2 is not required for efficient transduction. In contrast, in A549 cells, proteolytic cleavage appears to be a rate-limiting step, and overexpression of TMRPSS2 therefore enhances transduction.

### Discussion (HeLa versus A549 cell-line susceptibility to viral infection)

The increased susceptibility of one cell-line (HeLa, cervical, female, Afro-american origin) to SARS-CoV-2 pseudoviral infection is a particularly interesting observation versus another cell-line (A549, lungs, male, Caucasian origin) as it underlies the concept that characterization of patient populations is key to determining a therapeutic strategy tailored towards achieving maximal impact for patients either diagnosed with COVID-19 or otherwise for prophylactic purposes. Although race in itself might present a secondary role for consideration, genetic predisposition for either low endogenous AAT or higher levels of chronic inflammation (higher IFN-y and/or cathepsin L) for instance require higher doses and/or more regular administration of the active therapeutic substance (AAT).

### Experiment 4

The aim was to detect the endogenous levels of ACE2 and TMPRSS2 gene expression in different cell lines.

Method: Total RNA was extracted from Calu3, CaCo2, VeroE6, HepG2, A549, SH-SY5Y, HEK293-117T, HeLa, cell lines using RNeasy Micro Kit (Qiagen). Complementary DNA was synthesized from total RNA using PrimeScript^{™} RT Reagent kit (Takara). The real-time PCR measurement of cDNAs was performed using PowerUp SYBR^{™} Green Master Mix (Applied Biosystems) and normalized to the expression of GAPDH as control housekeeping gene. The primers are listed below:
ACE2 forward: cattggagcaagtgttggatctt (SEQ ID NO: 26)
ACE2 reverse: gagctaatgcatgccattctca (SEQ ID NO: 27)
TMPRSS2 forward: cacggactggatttatcgacaa (SEQ ID NO: 28)
TMPRSS2 reverse: cgtcaaggacgaagaccatgt (SEQ ID NO: 29)
GAPDH forward: gcacaagaggaagagagagacc (SEQ ID NO: 30)
GAPDH reverse: aggggagattcagtgtggtg (SEQ ID NO: 31)

The results are shown in Figure 3.

### Experiment 5

The aim was to determine whether Interferon gamma treatment induces ACE2 expression in A549 cell line.

Protocol: A549 cells were seeded in 12 well plate and the addition of interferon gamma was performed 24 hours post cell splitting. The treatment lasted for 48 hours and we performed RNA extraction, reverse transcriptase reaction and qPCR as previously described (cf. method section of Experiment 4).

The results are shown in Figure 4.

### Experiment 6

The aim was to determine whether interferon gamma treatment induces ACE2 expression in HeLa cells expressing the SARS-CoV-2 fusion protein.

Protocol: HeLa Spike cells were seeded in 12 well plate and the addition of interferon gamma was performed 24 hours post cell splitting. The treatment lasted for 48 hours and we performed RNA extraction, reverse transcriptase reaction and qPCR as previously described (cf. method section of Experiment 4).

The results are shown in Figure 5.

### Experiment 7: SARS-CoV-2 viral entry assay

The results are shown in Figure 7 and 8. Done with pseudolentivector expressing the surface protein SARS-CoV-2 spike harboring the D614G mutation, coding for a luciferase reporter (Figure 7 and 8).

The pseudolentivector is added on A549 cells overexpressing constitutively the ACE2 receptor (Figure 7) or both ACE2 and TMPRSS2 (Figure 8 ). Compound treatment is either performed 24h (panels A&B) or 1h (panels C&D) prior to addition of the pseudolentivector. Further schematic information on the pseudoviral assay protocol is provided in Figures 9 and 10.

### Example 4

Viral entry inhibition test with A549 human lung alveolar basal epithelium cells overexpressing ACE2 (Figure 11 and 22) or Caco2 human colorectal adenocarcinoma (Figure 21) were treated for 24h with AAT from multiple sources (Figure 11a,11b, 21a, 21b, 22a, 22b) or with inhibitor compounds (Figure 11c, 21c,22c). Then the lentivector coding for the luciferase reporter and expressing the SARS-CoV-2 spike protein with the D614G mutation (Figure 11), the SARS-CoV spike protein (Figure 22) or the MERS-CoV spike protein (Figure 21) was added for 6h. Finally, the culture media was changed, and cells were incubated for 3 additional days prior to measurements. Viral entry was measured by the lentivirus-mediated luciferase signal (Figure 11,21, 22 dark grey) and normalized with WST8-cell viability displayed as light grey. For all conditions the concentrations of DMSO / PBS have been normalized (buffer). All conditions were performed in triplicate, error bars represent standard deviation. Please refer to material and method section for technical information.

### Example 5

Viral entry inhibition test with HeLa human cervical cancer cells overexpressing ACE2 were treated for 24h with AAT from multiple sources (Figure 12a, 12b) or with inhibitor compounds (Figure 12c). Then the lentivector coding for the luciferase reporter and expressing the SARS-CoV-2 spike protein with the D614G mutation was added for 6h. Finally, the culture media was changed, and cells were incubated for 3 additional days prior to measurements. Viral entry was measured by the lentivirus-mediated luciferase signal (dark grey) and normalized with WST8-cell viability displayed as light grey. For all conditions the concentrations of DMSO/PBS have been normalized (buffer). All conditions were performed in triplicate, error bars represent standard deviation. Please refer to material and method section for technical information.

### Example 6

SARS-CoV-2 spike fusion assay. The principle of the test is depicted in Figure 13. HeLa human cervical cancer cells overexpressing either ACE2 and small bit luciferase or SARS-CoV-2 spike and large bit luciferase were mixed at equal number with the suited concentration of treatment. 24h later the cell fusion was measured by the amount of luciferase reporter signal. Luciferase signal are observed in function of the buffer control. Sera was diluted at final concentration of 1/16. For all conditions the concentrations of Sera / PBS have been normalized (buffer). All conditions were performed in triplicate, error bars represent standard deviation (Figure 14). Please refer to material and method section for technical information.

### Example 7

Quantitative PCR analysis was performed to determine gene copy number of proteases in A549 cell. Copy number was calculated using linear plasmid for each gene as a control (Figure 15a). Please refer to material and method section for technical information.

### Example 8

Quantitative PCR analysis was performed to determine relative gene expression of proteases ACE2 (Figure 15 b) and TMPRSS2 (Figure 15 c) in A549 cells. Calu3 and Caco2 cell lines were used as reference for respectively ACE2 and TMPRSS2 expression. Gapdh gene was used to normalize. Error bars indicate the standard deviation. Please refer to material and method section for technical information.

### Example 9

SARS-CoV-2 peptide 50uM, cathepsin B recombinant protein 1.76ng/ul and AAT at luM were incubated together for 45 min. Fluorescence was measured every minute in a UV spectrophotometer (excitation at 330nm and emission detection at 390nm) (Figure 16a). Vmax value was determined with the slope of the trendline. Experiment has been performed in duplicate. Error bars indicate the standard deviation. Please refer to material and method section for technical information.

### Example 10

SARS-CoV-2 peptide 50uM, cathepsin L recombinant protein 1.76ng/ul and AAT at 10 uM, were incubated together for 45 min. Fluorescence was measured every minute in a UV spectrophotometer (excitation at 330nm and emission detection at 390nm) (Figure 16 b). Vmax value was determined with the slope of the trendline. Experiment has been performed in duplicate. Error bars indicate the standard deviation. Please refer to material and method section for technical information.

### Example 11

SARS-CoV-2 peptide 50uM, trypsin recombinant protein 6.4nM and two concentrations of AAT (0.1 uM and 1 uM) were incubated together for 45 min. Fluorescence was measured every minute in a UV spectrophotometer (excitation at 330nm and emission detection at 390nm)(Figure 16 c). Vmax value was determined with the slope of the trendline. Experiment has been performed in duplicate. Error bars indicate the standard deviation. Please refer to material and method section for technical information.

### Example 12

SARS-CoV-2 peptide 50uM, furin recombinant protein 10U/ml and AAT at 1 uM were incubated together for 45 min. Fluorescence was measured every minute in a UV spectrophotometer (excitation at 330nm and emission detection at 390nm) (Figure 16d). Vmax value was determined with the slope of the trendline. Experiment has been performed in duplicate. Error bars indicate the standard deviation. Please refer to material and method section for technical information.

### Example 13

SARS-CoV-2 peptide 50uM, PCI recombinant protein 1.76ng/ul and several concentrations of AAT (1 uM, 10 uM, 40 uM and 100 uM) were incubated together for 45 min. Fluorescence was measured every minute in a UV spectrophotometer (excitation at 330nm and emission detection at 390nm) (Figure 16 e). Vmax value was determined with the slope of the trendline. Experiment has been performed in duplicate. Error bars indicate the standard deviation. Please refer to material and method section for technical information.

### Example 14

SARS-CoV-2 peptide 50uM, matriptase recombinant protein 1.76ng/ul and several concentrations of AAT (1 uM, 10 uM, 40 uM and 100 uM) were incubated together for 45 min. Fluorescence was measured every minute in a UV spectrophotometer (excitation at 330nm and emission detection at 390nm) (Figure 16 f). Vmax value was determined with the slope of the trendline. Experiment has been performed in duplicate. Error bars indicate the standard deviation. Please refer to material and method section for technical information.

### Example 15

AAT inhibits TMPRSS2 protease activity. Boc-QAR-AMC peptide 30 uM, TMPRSS2 recombinant protein 0.5 uM and AAT at 5 uM were incubated together for 45 min. Fluorescence was measured every minute in a UV spectrophotometer (excitation at 380nm and emission detection at 460nm) (Figure16g). Vmax value was determined with the slope of the trendline. Experiment has been performed in duplicate. Error bars indicate the standard deviation. Please refer to material and method section for technical information.

### Example 16

DQ^{™} elastin substrate 25ug/mL, elastase (ELA1) from pig pancreas 0.25U/mL and several concentrations of AAT (l0 nM, 50nM and 100 nM) were incubated together for 45 min. Fluorescence was measured every minute in a fluorescence reader (excitation at 485nm and emission detection at 530nm) (Figure 16 h). Vmax value was determined with the slope of the trendline. It was performed in duplicate. Error bars indicate the standard deviation. Please refer to material and method section for technical information.

### Example 17

Substrate MeOSuc-AAPV-pNA 100 uM, purified human neutrophil elastase (ELA2) 2.2uU/ul and several concentrations of AAT (1 nM and 10 nM) were incubated together for 45 min. Absorbance was measured every minute in a spectrophotometer reader (A405nm) (Figure 16i. Vmax value was determined with the slope of the trendline during 20 min. It was performed in duplicate. Error bars indicate the standard deviation. Please refer to material and method section for technical information.

### Example 18

Titer measurement was done using an Octet RED (Sartorius, Octet RED96) method, Protein A biosensors (Sartorius, Ref 18-5010; 18-5012), Mouse Mc anti hAAT IgG2A (abcam, ab116604), neutralization buffer (MBD; 0,02% Tween 20, 150mM, NaCl,1mg/mL BSA, PBS1X) and regeneration buffer (MBD; 10mM Glycine- HCL (pH2)) binding as illustrated in Figure 26. Comparison between plasma derived AAT (Sigma-Aldrich, SRP6312) and recombinant AAT produced in CHO by PL136/PL137 pool (Figure 17).

| **Acquisition parameters** | | | |
|---|---|---|---|
| **Step** | **Matrix** | **Duration (sec)** | **Shaking speed (rpm)** |
| **Sensor pre-conditioning** | Regeneration buffer | 5 *3 cycles | 1000 |
| | Neutralization buffer | 5 *3 cycles | 1000 |
| **Baseline pre-loading** | Neutralization buffer | 30 | 400 |
| **mAb loading** | mAb at 10 µg/mL in neutralization buffer | 300 | 400 |
| **Baseline post-loading** | Neutralization buffer | 300 | 1000 |
| **Protein standard association** | Range from 1,56 to 100 µg/mL in neutralization buffer | 600 | 1000 |
| **Sensor regeneration** | Regeneration buffer | 5 *3 cycles | 1000 |
| | Neutralization buffer | 5 *3 cycles | 1000 |

| **Analysis parameters** | | | |
|---|---|---|---|
| **Standard curve equation** | Dose Response - 5-PL unweighted | | |
| **Binding rate equation** | R Equilibrium | | |

### Example 19

Human microglial cells HMC3-MHCII^{Luc} cells were plated at day 0, activated with IFNγ at day1 until day 2 and measurement of the luciferase activity and cell viability were done at day 4 (Figure 18a). Activation was measured by the activity of MHCII-driven luciferase and normalized to cell viability. Luciferase activity for all conditions is represented as fold of the untreated cells control (Figure 18 b). All conditions were performed in triplicate, error bars represent standard deviation

### Example 20

Human microglial HMC3-MHCII^{Luc} and HM:C3-MHCII^{Luc};Ubi^{AAT} cells were plated at day 0, activated with IFNγ at day1 until day 2 and measurement of the luciferase activity and cell viability were measured at day 4. Plasma-derived AAT was applied from day 0 to day 4 on HMC3-MHCII^{Luc} cells (Figure 19a). Activation was measured by the activity of MHCH-driven luciferase and normalized to cell viability. Luciferase activity for all conditions is represented as percentage of IFNy-activation control (Figure 19 b). All conditions were performed in triplicate, error bars represent standard deviation

### Example 21

Human microglial HMC3-MHCII^{Luc} cells were plated at day 0, exposed to IFNγ and/or IFNβ at day 1 until day 2 and measurement of the luciferase activity and cell viability were done at day 4. Plasma-derived AAT was applied from day 0 to day 4 (Figure 20 a). Activation was measured by the activity of MHCII-driven luciferase and normalized to cell viability. Luciferase activity for all conditions is represented as percentage of IFNγ-activation control (Figure 20 b). Bars for plasma-derived AAT (1, 10, 25 µM) are repetition of Example 20 for comparison purpose. All conditions were performed in triplicate, error bars represent standard deviation.

### Example 22

Coomassie Figure legend: SDS-PAGE coomassie-stained with AAT from multiple sources.educed samples were prepared for analysis by mixing with NuPage 4x LDS sample buffer (Life Technologies) and NuPage 10x sample reducing agent (Life Technologies), and incubated at 70° C, 10 min. For non-reduced samples, the reducing agent and heat incubation were omitted. 5ug of protein was loaded per lane. Samples were electrophoresed on 4-20% Mini- PROTEAN^{®} Precast Gels (BioRad) with TGS buffer. Then gels were fixed for 30 minutes at room temperature in fixation buffer (50% Methanol + 10% acetic acid + 40% H2O). Coomassie blue (Sigma Aldrich) was added at final concentration of 0.25% and incubated for additional 15 minutes. Finally gels were destained overnight in multiple washes of fixation buffer, and imaged on a G:Box imager (Syngene)(Figure 24).

### Example 23

Expression of recombinant human AAT in HEK 293 cells. Based on the SEQ ID NO: 46 for HEK293 or SEQ ID NO: 47 for HEK293T. For HEK293 an rhAAT expression clone containing SERPINA1_OHu22141C_pcDNA3.1(+) plasmid was generated from the vector in Figure 25g. The plasmid-containing clone was sequence verified and a sufficient amount of plasmid for transfection of HEK 293 cells (ATCC, Catalog# CRL-1573) was prepared. HEK293 cells were transfected with the plasmid and rhAAT expression and secretion into the culture broth in 3 sets of 6-well plates was confirmed using a commercially available ELISA kit (Thermo Fischer Scientific, Catalog# EH411RBX5). Mock transfected cells as well as cells transfected with pCMV-Td Tomato and EGFP fluorescent markers was used as a control.

### Critical materials and reagents used for generating rhAAT HEK 293 cells

| **Materials and Reagents** | **Supplier** | **Catalog** # | **Amount** | **Purpose** |
|---|---|---|---|---|
| pcDNA^{™}3.1(+) | Thermo Fischer Scientific | V79020 | - | Expression vector |
| HEK-293 | ATCC | CRL-1573 | - | Host cells |
| HyClone^{™} Dulbecco's Modified Eagles Medium | Thermo Fischer Scientific | SH3024301 | 500mL | Growth medium |
| Gibco^{™} Fetal Bovine Serum, certified, US | Thermo Fischer Scientific | 16-000-044 | 500mL | Nutrient supplement |
| Gibco^{™} Penicillin-Streptomycin (10,000 U/mL) | Thermo Fischer Scientific | 15-140-122 | 100mL | Antibiotic supplement |
| Gibco^{™} Versene Solution | Thermo Fischer Scientific | 15-040-066 | 100mL | Cell dissociation reagent |
| Gibco^{™} DPBS, no calcium, no magnesium | Thermo Fischer Scientific | 14-190-250 | 500 mL | Cell wash/dilution buffer |
| Gibco^{™} Geneticin^{™} Selective Antibiotic (G418 Sulfate) (50 mg/mL) | Thermo Fischer Scientific | 10-131-035 | 20 mL | Antibiotic solution (selection agent) |
| Lipofectamine^{™} LTX Reagent with PLUS^{™} Reagent | Thermo Fischer Scientific | 15338100 | 1.0 mL | Transfection facilitating agent |
| Human Serpin A3/Alpha-1-Antichymotrypsin ELISA Kit | Thermo Fischer Scientific | EH411RBX5 | 1 | A1AT identity/quantitation |
| rhAAT Select Resin | | | 50mL | |
| Pellicon 10kDa membrane | Millipore | | | |
| Pellicon 10kDa membrane | Millipore | | | |

### PROCEDURE

Production of rhAAT in HEK 293 cells was executed in two stages:
1. Preparation of rhAAT expressing cells.
2. Production of rhAAT.

### Preparation of rhAAT expressing cells

Preparation of rhAAT expressing cells included the following activities:
- Construction and validation of expression-ready plasmid
- Transfection of the host cells
- Generation of rhAAT-expressing (stable) pools
- Clone selection

### Construction and validation of expression-ready plasmid

Based on the sequence presented in SEQ ID NO: 46, a rhAAT expression clone containing SERPINA1_OHu22141C_pcDNA3.1(+) plasmid was prepared by Genscript Biotech Corporation (Piscataway, NJ).

The plasmid-containing clone was shipped to RTI International (Research Triangle Park, NC) for sequence verification and preparation of a sufficient amount of plasmid for transfection of HEK 293 cells.

### Transient transfection of the host cells

HEK293 cells were transfected with SERPINA1_OHu22141C_pcDNA3.1(+) plasmid and rhAAT expression and secretion into the culture broth in 3 sets of 6-well plates was confirmed using a commercially available ELISA kit. Mock transfected cells as well as cells transfected with pCMV-Td Tomato and EGFP fluorescent markers was used as a control.

### Amounts of reagents and plasmid DNA per experiment per well

| **Plasmids** | **Plas mid (ng/ µL)** | **Plasmid (µL)** | **Volume / well (µL)** | **# of wells** | **Total volume (µL)** | **Opti-Mem (µL)** | **Plasmid (µL)** | **PLUS (µL)** | **L T X (µ L)** |
|---|---|---|---|---|---|---|---|---|---|
| pcDNA3.1 SERPINA1 | 1000 | 2.5 | 500 | 8 | 4000 | 3900 | 20.0 | 20.0 | 60 |
| Mock | 0 | 0 | 500 | 4 | 2000 | 1960 | 0.0 | 10.0 | 30 |
| EGFP | 308 | 8.1 | 500 | 2 | 1000 | 964 | 16.2 | 5.0 | 15 |
| pCMV-Td Tomato | 500 | 5.0 | 500 | 1 | 500 | 485 | 5.0 | 2.5 | 7.5 |

### Generation of rhAAT-expressing (stable) pools

Upon confirmation of successful transient transfection, generation of stable pools was performed in T-150 by antibiotic selection. The cells were maintained for up to 2 weeks under antibiotic selection to eliminate cells without plasmid. Following which, the culture supernatant was tested for protein expression using ELISA.

Pooled cells were frozen at 80°C prior to the transfer for further cell expansion and rhAAT production in shake flasks.

### Clone selection

Cells were harvested diluted and transferred to 10-cm2 dishes for isolating single clones. 3-6 clones from 50-96 colonies, depending on the behavior of the transgene, were identified for subsequent confirmation rhAAT expression by ELISA.

### Production of rhAAT

Production of rhAAT includes cell expansion and rhAAT production in cell stacks, purification of rhAAT by affinity chromatography, product concentration and formulation (buffer exchange) utilizing Amicon Ultra 15, 10kDa tubes.

### Cell expansion and rhAAT production in Cell Stacks

Recombinant anchorage dependent HEK-293 cells were grown in culture treated T-flasks and Cell Staks bottles in Dulbecco's Modified Eagles Medium (DMEM, HyClone) and supplemented with 10% fetal bovine serum (FBS, Gibco), Penicillin-Streptomycin (Gibco), and Geneticin Selective antibiotic G418 Sulfate (Gibco). The cultures were incubated in a humidified incubator at 37.0°C and 5% CO₂ and rotating at 2.5 rpm when grown in roller bottles and subsequently harvested.

### Purification of rhAAT by affinity chromatography

Clarified culture supernatant was tested for pH and Conductivity and titrated to 7.4 ± 0.1 using 1M Tris, pH 7.4 and ±5 mS/cm of the equilibration buffer with 1M NaCl, respectively, as required. Following the pH and conductivity adjustment, the clarified supernatant was filterd through 0.2µm filter prior to purification.

### Affinity chromatography

The affinity chromatography column packed with rhAAT Select Resin was rinsed with 3CV of Purified Water and sanitized with 3CV 0.5 M NaOH (with a 30min hold after 2CV), followed by another rinse with 3CV of Purified Water and equilibration with 10CV 0f 20mM Tris, 150mM NaCl pH 7.4 buffer. The rhAAT Select column was loaded with the maximum volume of the clarified supernatant calculated based on rhAAT titer at the end of shake flask culture, volume of the clarified supernatant and the maximum column load capacity of 10mg/mL resin. If required, in order to avoid overloading the resin, the clarified supernatant was processed through the rhAAT Select column in multiple cycles. Loaded protein was washed with 4CV of 20mM Tris, 150mM NaCl pH 7.4 buffer and rhAAT eluted with 4CV 0f 20mM Tris, 2 M MgCl pH 7.4 buffer into PETG collection bottles. The column was stripped with 4CV of PBS, pH 2.0 buffer and if required, equilibrated with 3CV of 20mM Tris, 150mM NaCl pH 7.4 buffer prior to performing additional cycles.

### Example 24

Construction of the vectors PL136 (pCGS3_AAT_native_SP; Figure 25d) (original signal peptide into HindIII/XhoI restriction sites (LC MCS)) and PL137 (pCGS3_AAT_SP5, Figure 25e) (The singal peptide of SEQ ID NO:1 was optimized by replacing amino acids 1-24 with an ATUM^{SM} (Newark, California) optimized signal peptide).

Coding sequences were codon optimized for expression in Chinese Hamster Ovary cells, using DNA2.0 proprietary algorithm (GeneGPS^{®} Expression OptimizationTechnology, https://www.dna20.com/services/genegps). A canonical kozak sequence (GCCGCCACC) was added in front of the start codon. HindIII and XhoI restriction sites were added 5' and 3' of the synthetized coding sequences.

The resulting DNA fragments were cloned into vector pJ201. The complete plasmid map and sequence of clone pJ201_AAT_native_SP (Figure 25b) and pJ201 _AAT_SP5 (Figure 25c) are provided.

pCGS3_AAT_native_SP and pCGS3_AAT_SP5 were obtained according to the following cloning scheme (empty pCGS3 vector Figure 25f):

| **Vector** | **Insert** | **Construct name** |
|---|---|---|
| pCGS3/ HindIII-XhoI | pJ201_AAT_native_SP/ HindIII-XhoI | pCGS3_AAT_native_SP |
| pCGS3/ HindIII-XhoI | pJ201_AAT_SP5/ HindIII-XhoI | pCGS3_AAT_SP5 |

### Fragments preparation:

**pCGS3/HindIII-XhoI**

| | |
|---|---|
| **MQ-H20** | **33 µl** |
| **pCGS3 (1 µg/µl)** | **1 µl** |
| **10X CutSmart buffer** | **4 µl** |
| **HindIII-HF** | **1 µl** |
| **XhoI** | **1 µl** |
| **total** | **40 µl** |

**pJ201_AAT**_**native_SP/HindIII-XhoI**

| | |
|---|---|
| **MQ-H20** | **9 µl** |
| **pJ201_AAT_native_SP (50 ng/µl)** | **25 µl** |
| **10X CutSmart buffer** | **4 µl** |
| **HindIII-HF** | **1 µl** |
| **XhoI** | **1 µl** |
| **total** | **40 µl** |

**pJ201_AAT_SP5/ HindIII-XhoI**

| | |
|---|---|
| **MQ-H20** | **9 µl** |
| **pJ201_AAT_SP5 (50 ng/µl)** | **25 µl** |
| **10X CutSmart buffer** | **4 µl** |
| **HindIII-HF** | **1 µl** |
| **XhoI** | **1 µl** |
| **total** | **40 µl** |

All digestions were incubated for 2 hour at 37°C. Fragments of interest were isolated by agarose gel electrophoresis (1.2% agarose gel). Fragments of expected size were excised, gel purified and used for the following ligations:

### Ligations

### pCGS3_AAT_native_SP_A

| | |
|---|---|
| **MQ-H20** | **4 µl** |
| **pCGS3/ HindIII-XhoI (10 ng/µl)** | **2 µl** |
| **pJ201_AAT_native_SP/ HindIII-XhoI (10 ng/µl)** | **2 µl** |
| **10X ligase buffer** | **1 µl** |
| **T4 DNA ligase** | **1 µl** |
| **total** | **10 µl** |

### pCGS3_AAT_SP5_A

| | |
|---|---|
| **MQ-H20** | **4 µl** |
| **pCGS3/ HindIII-XhoI (10 ng/µl)** | **2 µl** |
| **pJ201_AAT_SP5/HindIII-XhoI (10 ng/µl)** | **2 µl** |
| **10X ligase buffer** | **1 µl** |
| **T4 DNA ligase** | **1 µl** |
| **total** | **10 µl** |

The ligation mixtures (1 µl) were transformed into E. coli Stabl2 cells from Invitrogen. The transformed E. coli cells were cultivated on LB Agar Lennox, Animal Free containing 50 mg/l Carbenicillin. E. coli colonies were transferred into 4 ml LB Broth Lennox, Animal Free containing 50 mg/l Ampicillin and incubated over night at 33°C in a shaker incubator at 300 rpm. Plasmid DNA was isolated from the E. coli cultures using a Qiagen BioRobot 9600 and the Nucleo Spin Robot-8 Plasmid kit from Macherey-Nagel. DNA was then sequenced using chains specific sequencing primers covering the cloning sites.

The sequences of the following clones were confirmed to be identical to the expected ones PL136 (pCGS3_AAT_native_SP; Figure 25d) and PL137 (pCGS3_AAT_SP5, Figure 25e). AAT obtained from the PL136 in CHO and PL137 in CHO were pooled.

### Example 25

### Gene Synthesis and Single Gene Construction

The rhAAT single gene vector was constructed by sub-cloning the product gene into the vector pXC-17.4 (Lonza) (Figure 25a).

### DNA Amplification

1µL of vector DNA was used to transform One Shot^{®} Stbl3 Chemically Competent E. coli cells (Life Technologies, C7373-03) using the heat-shock method according to manufacturer's instructions. Cells were spread onto ampicillin-containing (50 µg/ml) LB agar plates (LB Broth Base, Select APS^{™} and Bacto-Agar, both Becton Dickinson, 292438 and 214010 respectively) and incubated overnight at 37°C until bacterial colonies were evident.

For Giga preps, single bacterial cultures were used to inoculate a starter culture which was subsequently used to inoculate 1.0 L Plasmid Plus Medium (Thomson, 446300) containing 50 µg ampicillin and incubated at 37°C overnight with shaking. Vector DNA was isolated using the QIAGEN Gigaprep system (Qiagen, 12291). In all instances, DNA concentration was measured using a Nanodrop 1000 spectrophotometer (Thermo-Scientific) and adjusted to 1mg/mL. DNA quality was assessed by measuring the absorbance ratio at 260 and 280 nm.

### Routine Culture of CHOK1SV GS-KO Cells

CHOK1SV GS-KO cells were cultured in CD-CHO media (Life Technologies, 10743-029) supplemented with 6mM L-glutamine (Life Technologies, 25030-123). Cells were incubated in a shaking incubator at 36.5°C, 5% CO2, 85% humidity, 140 rpm. Cells were routinely sub-cultured every 3-4 days, seeding at 0.2x106 cells/ml and were propagated in order to have sufficient cells available for transfection. Cells were discarded by passage 20.

Stable recombinant CHOK1SV GS-KO cells were cultured in CD-CHO media supplemented with 50 µM MSX (L-Methionine Sulfoximine, Sigma-Aldrich, M5379) and SP4. Cells were incubated in a shaking incubator at 36.5°C, 5% CO2, 85% humidity, 140 rpm. Cells were routinely sub-cultured every 3-4 days, seeding at 0.2x 106 cells/ml and were propagated in order to have sufficient cells available for the large scale fed-batch overgrowth.

### Stable Pooled Transfection of CHOK1SV GS-KO Cells

Single gene vector DNA plasmid was prepared for transfection by linearising with PvuI followed by ethanol precipitation and resuspension in EB buffer to a final concentration of 400 µg/ml. Transfection was carried out via electroporation using the Gene Pulse XCell. For each transfection, viable cells were resuspended in pre-warmed CD-CHO media to 1.43x 107 cells/ml. 100 µl linearised DNA at a concentration of 400 µg/ml was aliquotted into a 0.4 cm gap electroporation cuvette and 700 µl cell suspension added. Three cuvettes of cells and DNA were electroporated at 300 V, 900 µF and immediately recovered to 30 ml pre-warmed CD-CHO supplemented with 10 ml/L SP4 (Lonza, BESP1076E) to generate a stable pool. The transfectants were incubated in a shaking incubator at 36.5°C, 5% CO2, 85% humidity, 140 rpm.

A total of 3 stable pool transfectants were established. 24 h post-transfection the cultures were centrifuged and resuspended into pre-warmed CD-CHO supplemented with 50 µM MSX and 10 ml/L SP4. Cell growth and viability were periodically checked post- transfection.

When the viable cell density was >0.6x 106 cells/ml, the transfectant cultures were suitable to progress. Cells were seeded at 0.2x 106 cells/ml in a final volume of 100 ml in CD-CHO medium supplemented with 50 µM MSX/ 10 ml/L SP4, in a 500 ml vented Erlenmeyer flask (Fisher Scientific (Coming), 10352742) and incubated in a shaking incubator at 36.5°C, 5% CO2, 85% humidity, 140 rpm. Cell cultures were monitored and expanded once cultures had adapted to exponential growth. Cultures were expanded to 200 ml culture volume in 500 ml vented Erlenmeyer flasks at a concentration of 0.2x 106 cells/ml in CD- CHO supplemented with 50 µM MSX/10 ml/L SP4 and incubated under the conditions described above (see Section 0). Cultures were then expanded to the appropriate production volume.

### Abridged Fed-Batch Overgrow

Cells were propagated to production volume of 2 L per product by seeding the appropriate culture volume at 0.2x 106 cells/mL in Lonza's CM42 base media supplemented with 4 mL/L SPE from the established stable pools. The production volumes were established in 5 L (Generon, 931116) shake flasks and incubated in a shaking incubator at 36.5°C, 5% CO2, 85% humidity, and 140 rpm. Cell count and viability were monitored on day 4, before feeding was initiated and periodically until the culture was harvested on day 11. The bolus feeds were administered on day 4 and 8 consisting of a mixture of Lonza's proprietary feeds.

### Harvesting and Concentrating of Production Culture

The culture was harvested by centrifugation at 3000 rpm for 10 min and filtered using a 0.22 µm PES membrane to obtain clarified supernatant.

### Alpha-1 anti-trypsin Affinity Chromatography

The clarified cell supernatant was purified using an in-house packed 50 ml Alpha-1 anti- trypsin Select column (GE Healthcare). The column was equilibrated pre- and post- product application with 20mM Tris, 150mM NaCl, pH 7.4 and elution was carried out with 20mM Tris, 2 M MgCl2, pH 7.4. The procedure was carried out on an AKTA Purifier at 10 ml/min. Product-containing fractions from the affinity chromatography eluate were pooled and buffer exchanged into 50mM Tris, 75mM NaCl, pH 8.0 by Tangential Flow Filtration (TFF) using Spectrum's KrosFlo TFF system with a 10 kDa MWCO (D02-E010-05-N).

### Anion Exchange Chromatography

The pooled and buffer exchanged fractions were further purified using 25 ml CaptoQ column (5x 5ml columns connected in tandem) (GE Healthcare) on an AKTA purifier at 10 ml/min. The column was equilibrated pre- and post- product application with 50mM Tris, 75mM NaCl, pH 8.0. The product was eluted over a 10 CV linear gradient to 50mM Tris, 1 M NaCl, pH 8.0. Product-containing fractions from the anion exchange chromatography eluate were pooled, diluted 1 mg/ml and quality analysed.

### SE-HPLC

Duplicate samples were analysed by SE-HPLC on an Agilent 1200 series HPLC system, using a Zorbax GF-250 9.4mM ID x 25 cm column (Agilent). 80 µL aliquots of 1 mg/mL samples (or stock concentration if samples are < 1 mg/mL) were injected and run in 50mM sodium phosphate, 150mM sodium chloride, 500mM arginine, pH 6.0 at 1 mL/min for 15 minutes. Soluble aggregate levels were analysed using Empower software. Signals arising from buffer constituents were analysed by blank buffer injection and are omitted in the data analysis unless indicated otherwise.

### Product concentration

The purified product was concentrated to a target of 25mg/mL using Vivaspin Turbo-15 Centrifugal Filter Units 30KDa Molecular Weight Cut-Off (Sartorius, VS1 ST02).

### REFERENCES

1. Wrapp, D., et al., Cryo-EM structure of the 2019-nCoV spike in the prefusion conformation. Science, 2020. 367(6483): p. 1260-1263.
2. Jaimes, J.A., J.K. Millet, and G.R. Whittaker, Proteolytic Cleavage of the SARS-CoV-2 Spike Protein and the Role of the Novel S1/S2 Site. iScience, 2020. 23(6): p. 101212.
3. Korber, B., et al., Spike mutation pipeline reveals the emergence of a more transmissible form of SARS-CoV-2. bioRxiv, 2020: p. 2020.04.29.069054.
4. Becerra-Flores, M. and T. Cardozo, SARS-CoV-2 viral spike G614 mutation exhibits higher case fatality rate. Int J Clin Pract, 2020.
5. Li, Q., et al., The Impact of Mutations in SARS-CoV-2 Spike on Viral Infectivity and Antigenicity. Cell, 2020.
6. Ogawa, J., et al., The D614G mutation in the SARS-CoV-2 Spike protein increases infectivity in an ACE2 receptor dependent manner. bioRxiv, 2020.
7. Goodfellow, I. Goodfellow, and S.I. Taube, Calicivirus Replication and Reverse Genetics Viral Gastroenteritis : Molecular Epidemiology and Pathogenesis. 2016: p. 355-378.
8. Yin, W., et al., Structural basis for inhibition of the RNA-dependent RNA polymerase from SARS-CoV-2 by remdesivir. Science, 2020.
9. Gao, Y., et al., Structure of the RNA-dependent RNA polymerase from COVID-19 virus. Science, 2020. 368(6492): p. 779-782.
10. Pachetti, M., et al., Emerging SARS-CoV-2 mutation hot spots include a novel RNA-dependent-RNA polymerase variant. J Transl Med, 2020. 18(1): p. 179.

## Claims

1. A composition for use in the treatment and/or prevention of a disease or syndrome caused by a coronavirus infection in a subject in need thereof, the composition comprising a therapeutically effective amount of an alphal-antitrypsin (AAT) protein, a variant, an isoform and/or a fragment thereof,
wherein the variant is selected from the group comprising short cyclic peptides derived from the C-terminal sequence as set forth in SEQ ID NO: 2, and the short cyclic peptide is selected from the group comprising Cyclo-(CPFVFLM)-SH, Cyclo-(CPFVFLE)-SH, Cyclo-(CPFVFLR)-SH, and Cyclo-(CPEVFLM)-SH, or any combination thereof, and wherein the isoform of the AAT protein is a splice variant resulting from alternative splicing of an AAT mRNA.

2. The composition for use according to claim 1, wherein the subject in need thereof has at least one selected from the group consisting of:
1. a lower level of endogenous alpha-antitrypsin (AAT) prior to the virus infection or during the virus infection compared to at least one subject during the virus infection, which is asymptomatic or has mild symptoms, wherein preferably the lower level of endogenous AAT prior to a virus infection or during the virus infection is caused by AAT-deficiency,
2. a higher level of at least one spike protein priming protease prior to the virus infection or during the virus infection compared to at least one subject during the virus infection, which is asymptomatic or has mild symptoms, wherein preferably the higher level of the at least one spike protein priming protease is caused by age and/or a genetic predisposition,
3. a higher level of angiotensin converting enzyme 2 (ACE2 receptor) in a subject prior to the virus infection or during the virus infection compared to at least one subject during the virus infection, which is asymptomatic or has mild symptoms, wherein preferably the higher level of ACE2 receptor is caused by at least one selected from the group of an infection, inflammation, age and a genetic predisposition, and
4. a higher level of interferon-gamma (IFN- γ ) in a subject prior to a respiratory virus infection or during a respiratory virus infection compared to at least one subject during a respiratory virus infection, which is asymptomatic or has mild symptoms, wherein preferably the higher level of IFN-γ is caused by at least one selected from the group of an infection, inflammation, age and a genetic predisposition.

3. The composition for use according to claim 2, wherein the spike protein priming protease is at least one selected from the group consisting of transmembrane protease serine subtype 2 (TMPRSS2), transmembrane protease subtype 6 (TMPRSS6), cathepsin L, cathepsin B, proprotein convertase 1 (PC1), trypsin, elastase, neutrophil elastase, matriptase and furin, preferably cathepsin L and furin.

4. The composition for use according to any of the claims 2 or 3, wherein the subject in need thereof has
i) a lower level of endogenous AAT, and
ii) a higher level of at least one spike protein priming protease and/or a higher level of ACE2 receptor and/or a higher level of IFN-γ,
wherein preferably the higher level of IFN-γ is caused by at least one disease or condition selected from the group consisting of AAT-deficiency, a liver disease such as a non-alcoholic fatty liver disease, diabetes, obesity and a cardiovascular condition.

5. The composition for use according to any one of the preceding claims, wherein the alphal-antitrypsin (AAT) protein is human plasma-extracted.

6. The composition for use according to any one of the claims 1 to 5, wherein the alpha1-antitrypsin (AAT) protein is recombinant alphal-antitrypsin (rhAAT).

7. The composition for use according to any one of the claims 1 to 6, wherein the alpha1-antitrypsin protein is as set forth in SEQ ID NO: 1.

8. The composition for use according to claim 6, wherein the alphal-antitrypsin fragment is a C-terminal sequence fragment, or any combination thereof.

9. The composition for use according to any of the previous claims, wherein the composition further comprises a pharmaceutically acceptable excipient or carrier and/or wherein the composition further comprises a nucleoside analogue, a protease inhibitor, an immune-suppressor, preferably sarilumab or tocilizumab, an antibiotic, an antibody directed against structural components of the virus, or fragment thereof, preferably passive immunotherapy, interferon beta, preferably interferon beta-1a, and/or a vaccine.

10. The composition for use according to any one of the preceding claims wherein said composition is administered by intravenous injection, intravenous infusion, infusion with a dosator pump, inhalation nasal-spray, eye-drops, skin-patches, slow-release formulations, *ex vivo* gene therapy or ex vivo cell-therapy, preferably by intravenous injection.

11. A method for determining the susceptibility of a subject of interest for treatment and/or prevention of a disease or syndrome caused by a coronavirus infection using a composition comprising a therapeutically effective amount of an alphal-antitrypsin (AAT) protein, the variant, the isoform and/or the fragment thereof as defined in any of claims 1 to 10, said method comprises the steps of:
a) determining the level of at least one of the group comprising endogenous alpha1-antitrypsin, at least one spike protein priming protease, ACE2 receptor and interferon-gamma in a sample of the subject of interest prior to the virus infection or during the virus infection,
b) determining the level of at least one of the group comprising endogenous alpha1-antitrypsin, at least one spike protein priming protease, ACE2 receptor and interferon-gamma in a sample of at least one reference subject during a coronavirus infection, wherein the reference subject is asymptomatic or has mild symptoms,
c) comparing the level of interest determined in step a) to the reference level determined in step b),
wherein the subject of interest is more susceptible for treatment and/or prevention of the virus infection if the subject of interest has at least one selected from the group consisting of:
1. a lower level of interest of endogenous alpha-antitrypsin (AAT) compared to the reference level of endogenous AAT,
2. a higher level of interest of at least one spike protein priming protease compared to the reference level of at least one spike protein priming protease,
3. a higher level of interest of angiotensin converting enzyme 2 (ACE2 receptor) compared to the reference level of the ACE2 receptor and
4. a higher level of interest of interferon-gamma (IFN- γ ) compared to the reference level of IFN- γ .

12. A method for determining the therapeutically effective amount of alphal-antitrypsin (AAT) for an effective treatment and/or prevention of a disease or syndrome caused by a coronavirus infection using the composition according to any of the claims 1 to 10, said method comprises the steps of:
a) determining the level of endogenous alphal-antitrypsin in a sample of a subject of interest prior to the virus infection or during the virus infection,
b) determining the amount of AAT in the composition, which is required to achieve a level of AAT in the subject of at least 10 µM, preferably at least 20 µM, more preferably at least 50 µM, even more preferably at least 100 µM, and most preferably at least 200 µM.

13. The composition for use according to any one of the claims 1 to 10, or the method according to claim 11 or 12, wherein the coronavirus is selected from the group consisting of MERS-CoV, SARS-CoV-1, SARS-CoV-2, alpha Coronavirus 299E, alpha Coronavirus NL63, beta Coronavirus OC43, and beta Coronavirus HKU1; preferably the coronavirus is selected from the group consisting of MERS-CoV, SARS-CoV-1, and SARS-CoV-2, more preferably the virus is SARS-CoV-2.

14. The composition for use according to any one of the claims 1 to 10, or the method according to claims 11 to 13, wherein the disease or syndrome is a respiratory syndrome or a severe acute respiratory syndrome or the disease or syndrome is an inflammatory disease or syndrome of the nervous system.

## Patentansprüche

1. Zusammensetzung zur Verwendung bei der Behandlung und/oder Vorbeugung einer Erkrankung oder eines Syndroms, die/das durch eine Coronavirus-Infektion bei einem Individuum verursacht wird, das diese benötigt, wobei die Zusammensetzung eine therapeutisch wirksame Menge Alpha 1-Antitrypsin (AAT)-Protein, einer Variante, einer Isoform und/oder einem Fragment davon umfasst,
wobei die Variante ausgewählt ist aus der Gruppe umfassend kurze zyklische Peptide, die von der in SEQ ID NO:2 dargestellten C-terminalen Sequenz abstammen, und das kurze zyklische Peptid ausgewählt ist aus der Gruppe umfassend Cyclo-(CPFVFLM)-SH, Cyclo-(CPFVFLE)-SH, Cyclo-(CPFVFLR)-SH und Cyclo-(CPEVFLM)-SH, oder eine beliebige Kombination davon, und wobei die Isoform des AAT-Proteins eine Splice-Variante ist, die aus dem alternativen Splicing einer AAT-mRNA resultiert.

2. Zusammensetzung zur Verwendung nach Anspruch 1, wobei das Individuum, das diese benötigt, mindestens eines hat ausgewählt aus der Gruppe bestehend aus:
1. einem niedrigeren Spiegel an endogenem Alpha-Antitrypsin (AAT) vor der Virusinfektion oder während der Virusinfektion im Vergleich zu mindestens einem Individuum während der Virusinfektion, die asymptomatisch ist oder milde Symptome aufweist, wobei bevorzugt der niedrigere Spiegel an endogenem Alpha-Antitrypsin (AAT) vor der Virusinfektion oder während der Virusinfektion durch einen Mangel an AAT verursacht wird,
2. einem höheren Spiegel mindestens einer Spike-Protein-Priming-Protease vor der Virusinfektion oder während der Virusinfektion im Vergleich zu mindestens einem Individuum während der Virusinfektion, die asymptomatisch ist oder milde Symptome aufweist, wobei bevorzugt der höhere Spiegel der mindestens einen Spike-Protein-Priming-Protease durch Alter und/oder eine genetische Prädisposition verursacht wird,
3. einem höheren Spiegel an Angiotensin-konvertierendem Enzym 2 (ACE2-Rezeptor) bei einem Individuum vor der Virusinfektion oder während der Virusinfektion im Vergleich zu mindestens einem Individuum während der Virusinfektion, die asymptomatisch ist oder milde Symptome aufweist, wobei bevorzugt der höhere Spiegel an ACE2-Rezeptor durch mindestens eines ausgewählt aus der Gruppe von einer Infektion, Entzündung, Alter und einer genetischen Prädisposition verursacht wird, und
4. einem höheren Spiegel an Interferon-gamma (IFN-γ) bei einem Individuum vor einer respiratorischen Virusinfektion oder während einer respiratorischen Virusinfektion im Vergleich zu mindestens einem Individuum während einer respiratorischen Virusinfektion, die asymptomatisch ist oder milde Symptome aufweist, wobei bevorzugt der höhere Spiegel an IFN-γ durch mindestens eines ausgewählt aus der Gruppe von einer Infektion, Entzündung, Alter und einer genetischen Prädisposition verursacht wird.

3. Zusammensetzung zur Verwendung nach Anspruch 2, wobei die Spike-Protein-Priming-Protease mindestens eine ausgewählt aus der Gruppe bestehend aus transmembraner Protease-Serin-Subtyp 2 (TMPRSS2), transmembraner Protease-Subtyp 6 (TMPRSS6), Cathepsin L, Cathepsin B, Proprotein-Convertase 1 (PC1), Trypsin, Elastase, neutrophile Elastase, Matriptase und Furin, bevorzugt Cathepsin L und Furin ist.

4. Zusammensetzung zur Verwendung nach einem der Ansprüche 2 oder 3, wobei das Individuum, das diese benötigt,
i) einen niedrigeren Spiegel an AAT hat, und
ii) einen höheren Spiegel an mindestens einer Spike-Protein-Priming-Protease und/oder einen höheren Spiegel eines ACE2-Rezeptors und/oder einen höheren Spiegel an IFN-γ hat,
wobei bevorzugt der höhere Spiegel an IFN-γ durch mindestens eine Erkrankung oder einem Leiden verursacht wird, die/der ausgewählt ist aus der Gruppe bestehend aus einem Mangel an AAT, einer Lebererkrankung wie eine nichtalkoholischer Fettleber, Diabetes, Adipositas und einem kardiovaskulären Leiden.

5. Zusammensetzung zur Verwendung nach einem der vorangehenden Ansprüche, wobei das Alpha 1-Antitrypsin (AAT)-Protein aus humanem Plasma extrahiert ist.

6. Zusammensetzung zur Verwendung nach einem der Ansprüche 1 bis 5, wobei das Alpha 1-Antitrypsin (AAT)-Protein rekombinantes Alpha 1-Antitrypsin (rhAAT) ist.

7. Zusammensetzung zur Verwendung nach einem der Ansprüche 1 bis 6, wobei das Alpha 1-Antitrypsin-Protein wie in SEQ ID NO:1 dargestellt ist.

8. Zusammensetzung zur Verwendung nach Anspruch 6, wobei das Alpha 1-Antitrypsin-Fragment ein C-terminales Sequenzfragment oder eine beliebige Kombination davon ist.

9. Zusammensetzung zur Verwendung nach einem der vorangehenden Ansprüche, wobei die Zusammensetzung des Weiteren einen pharmazeutisch verträglichen Exzipienten oder Träger umfasst und/oder wobei die Zusammensetzung des Weiteren ein Nucleosid-Analogon, einen Protease-Inhibitor, ein Immunsuppressivum, bevorzugt Sarilumab oder Tocilizumab, ein Antibiotikum, einen gegen die strukturellen Bestandteile des Virus gerichteten Antikörper oder ein Fragment davon, bevorzugt passive Immuntherapie, Interferon-beta, bevorzugt Interferon-beta-1a und/oder einen Impfstoff umfasst.

10. Zusammensetzung zur Verwendung nach einem der vorangehenden Ansprüche, wobei die Zusammensetzung durch intravenöse Injektion, intravenöse Infusion, Infusion mit einer Dosierpumpe, Inhalation, Nasenspray, Augentropfen, Hautpflaster, Formulierungen mit langsamer Freisetzung, ex vivo-Gentherapie oder ex vivo-Zelltherapie, bevorzugt durch intravenöse Injektion verabreicht wird.

11. Verfahren zum Bestimmen der Empfänglichkeit eines Individuums von Interesse zur Behandlung und/oder Vorbeugung einer Erkrankung oder eines Syndroms, die/das durch eine Coronavirus-Infektion verursacht wird, unter Verwendung einer Zusammensetzung, die eine therapeutisch wirksame Menge Alpha 1-Antitrypsin (AAT)-Protein, der Variante, der Isoform und/oder des Fragments davon umfasst, wie nach einem der Ansprüche 1 bis 10 definiert, wobei das Verfahren die Schritte umfasst:
a) Bestimmen des Spiegels von mindestens einem aus der Gruppe umfassend endogenes Alpha 1-Antitrypsin, mindestens eine Spike-Protein-Priming-Protease, ACE2-Rezeptor und Interferon-gamma in einer Probe des Individuums von Interesse vor einer Virusinfektion oder während einer Virusinfektion,
b) Bestimmen des Spiegels von mindestens einem aus der Gruppe umfassend endogenes Alpha 1-Antitrypsin, mindestens eine Spike-Protein-Priming-Protease, ACE2-Rezeptor und Interferon-gamma in einer Probe mindestens eines Referenz-Individuums während einer Coronavirus-Infektion, wobei das Referenz-Individuum asymptomatisch ist oder milde Symptome aufweist,
c) Vergleichen des in Schritt a) bestimmten Spiegels von Interesse mit dem in Schritt b) bestimmten Referenz-Spiegel,
wobei das Individuum von Interesse empfänglicher für eine Behandlung und/oder Vorbeugung der Virusinfektion ist, wenn das Individuum von Interesse mindestens eines aufweist, ausgewählt aus der Gruppe bestehend aus:
1. einen niedrigeren Spiegel von Interesse an endogenem Alpha-Antitrypsin (AAT) im Vergleich zum Referenz-Spiegel an endogenem AAT,
2. einen höheren Spiegel von Interesse von mindestens einer Spike-Protein-Priming-Protease im Vergleich zum Referenz-Spiegel mindestens einer Spike-Protein-Priming-Protease,
3. einen höheren Spiegel von Interesse an Angiotensin-konvertierendem Enzym 2 (ACE2-Rezeptor) im Vergleich zu dem Referenz-Spiegel an ACE2-Rezeptor, und
4. einen höheren Spiegel von Interesse an Interferon-gamma (IFN-γ) im Vergleich zu dem Referenz-Spiegel an IFN-γ.

12. Verfahren zum Bestimmen der therapeutisch wirksamen Menge Alpha 1-Antitrypsin (AAT) für eine wirksame Behandlung und/oder Vorbeugung einer Erkrankung oder eines Syndroms, die/das durch eine Coronavirus-Infektion verursacht wird, unter Verwendung der Zusammensetzung nach einem der Ansprüche 1 bis 10, wobei das Verfahren die Schritte umfasst:
a) Bestimmen des Spiegels an endogenem Alpha 1-Antitrypsin in einer Probe eines Individuums von Interesse vor einer Virusinfektion oder während einer Virusinfektion,
b) Bestimmen der Menge an AAT in der Zusammensetzung, die erforderlich ist, um einen Spiegel an AAT in dem Individuum von mindestens 10 µM, bevorzugt mindestens 20 µM, weiter bevorzugt mindestens 50 µM, noch weiter bevorzugt mindestens 100 µM und am weitesten bevorzugt mindestens 200 µM zu erreichen.

13. Zusammensetzung zur Verwendung nach einem der Ansprüche 1 bis 10 oder das Verfahren nach Anspruch 11 oder 12, wobei das Coronavirus ausgewählt ist aus der Gruppe bestehend aus MERS-CoV, SARS-CoV-1, SARS-CoV-2, Alpha-Coronavirus 299E, Alpha-Coronavirus NL63, Beta-Coronavirus OC43 und Beta-Coronavirus HKU1; bevorzugt ist das Coronavirus ausgewählt aus der Gruppe bestehend aus MERS-CoV, SARS-CoV-1 und SARS-CoV-2, weiter bevorzugt ist das Virus SARS-CoV-2.

14. Zusammensetzung zur Verwendung nach einem der Ansprüche 1 bis 10 oder das Verfahren nach den Ansprüchen 11 bis 13, wobei die Erkrankung oder das Syndrom ein respiratorisches Syndrom oder ein schweres, akutes respiratorisches Syndrom ist oder die Erkrankung oder das Syndrom eine entzündliche Erkrankung oder ein entzündliches Syndrom des Nervensystems ist.

## Revendications

1. Composition pour utilisation dans le traitement et/ou la prévention d'une maladie ou d'un syndrome causé(e) par une infection par le coronavirus chez un sujet en ayant besoin, la composition comprenant une quantité thérapeutiquement efficace d'une protéine alpha1-antitrypsine (AAT), d'un variant, d'une isoforme et/ou d'un fragment correspondant,
dans laquelle le variant est choisi dans le groupe comprenant des peptides cycliques courts dérivés de la séquence C-terminale tels que décrits dans SEQ ID NO: 2, et le peptide cyclique court est choisi dans le groupe comprenant Cyclo-(CPFVFLM)-SH, Cyclo-(CPFVFLE)-SH, Cyclo-(CPFVFLR)-SH, et Cyclo-(CPEVFLM)-SH, ou toute combinaison correspondante, et dans laquelle l'isoforme de la protéine AAT est un variant d'épissage provenant d'un épissage alternatif d'un ARNm AAT.

2. Composition pour utilisation selon la revendication 1, dans laquelle le sujet nécessitant celle-ci a au moins l'un choisi dans le groupe constitué par :
1. un niveau plus bas d'alpha-antitrypsine endogène (AAT) avant l'infection par le virus ou pendant l'infection par le virus par rapport à au moins un sujet au cours de l'infection par le virus, qui est asymptomatique ou présente des symptômes légers, de préférence le niveau plus bas d'AAT endogène avant une infection par le virus ou pendant l'infection par le virus étant causé par un déficit en AAT,
2. un niveau plus élevé d'au moins une protéase d'amorçage de protéine spike avant l'infection par le virus ou pendant l'infection par le virus par rapport à au moins un sujet au cours de l'infection par le virus, qui est asymptomatique ou présente des symptômes légers, de préférence le niveau plus élevé d'au moins une protéase d'amorçage de protéine spike étant causé par l'âge et/ou une prédisposition génétique,
3. un niveau plus élevé d'enzyme 2 de conversion de l'angiotensine (récepteur ACE2) chez un sujet avant l'infection virale ou pendant l'infection virale par rapport à au moins un sujet pendant l'infection virale, qui est asymptomatique ou présente des symptômes légers, de préférence le niveau plus élevé de récepteur ACE2 étant causé par au moins l'un choisi dans le groupe d'une infection, une inflammation, l'âge et une prédisposition génétique, et
4. un niveau plus élevé d'interféron gamma (IFN-γ) chez un sujet avant une infection par un virus respiratoire ou pendant une infection par un virus respiratoire par rapport à au moins un sujet pendant une infection par un virus respiratoire, qui est asymptomatique ou présente des symptômes légers, de préférence le niveau plus élevé d'IFN-γ étant causé par au moins l'un choisi dans le groupe d'une infection, une inflammation, l'âge et une prédisposition génétique.

3. Composition pour utilisation selon la revendication 2, dans laquelle la protéase d'amorçage de protéine spike est au moins une protéase choisie dans le groupe constitué par la protéase transmembranaire à sérine de sous-type 2 (TMPRSS2), la protéase transmembranaire de sous-type 6 (TMPRSS6), la cathepsine L, la cathepsine B, la proprotéine convertase 1 (PC1), la trypsine, l'élastase, l'élastase des neutrophiles, la matriptase et la furine, de préférence la cathepsine L et la furine.

4. Composition pour utilisation selon l'une quelconque des revendications 2 et 3, dans laquelle le sujet en ayant besoin a
i) un niveau plus bas d'AAT endogène, et
ii) un niveau plus élevé d'au moins une protéase d'amorçage de protéine spike et/ou un niveau plus élevé de récepteur ACE2 et/ou un niveau plus élevé d'IFN-γ,
dans laquelle de préférence, le niveau plus élevé d'IFN-γ est causé par au moins une maladie ou affection choisie dans le groupe constitué par une carence en AAT, une maladie du foie telle qu'une stéatose hépatique non alcoolique, le diabète, l'obésité et une affection cardiovasculaire.

5. Composition pour utilisation selon l'une quelconque des revendications précédentes, dans laquelle la protéine alpha1-antitrypsine (AAT) est extraite de plasma humain.

6. Composition pour utilisation selon l'une quelconque des revendications 1 à 5, dans laquelle la protéine alpha1-antitrypsine (AAT) est une alpha1-antitrypsine recombinante (rhAAT).

7. Composition pour utilisation selon l'une quelconque des revendications 1 à 6, dans laquelle la protéine alpha1-antitrypsine est telle que définie dans SEQ ID NO: 1.

8. Composition pour utilisation selon la revendication 6, dans laquelle le fragment d'alpha1-antitrypsine est un fragment de séquence C-terminale, ou toute combinaison correspondante.

9. Composition pour utilisation selon l'une quelconque des revendications précédentes, dans laquelle la composition comprend en outre un excipient ou support pharmaceutiquement acceptable et/ou dans laquelle la composition comprend en outre un analogue nucléosidique, un inhibiteur de protéase, un immunosuppresseur, de préférence le sarilumab ou le tocilizumab, un antibiotique, un anticorps dirigé contre des composants structuraux du virus, ou un fragment correspondant, de préférence l'immunothérapie passive, l'interféron bêta, de préférence l'interféron bêta-1a, et/ou un vaccin.

10. Composition pour utilisation selon l'une quelconque des revendications précédentes, dans laquelle ladite composition est administrée par injection intraveineuse, perfusion intraveineuse, perfusion avec une pompe doseuse, inhalation par pulvérisation nasale, gouttes oculaires, patchs cutanés, formulations à libération prolongée, thérapie génique *ex vivo* ou thérapie cellulaire *ex vivo,* de préférence par injection intraveineuse.

11. Procédé pour déterminer la susceptibilité d'un sujet d'intérêt pour le traitement et/ou la prévention d'une maladie ou d'un syndrome causé(e) par une infection par le coronavirus au moyen d'une composition comprenant une quantité thérapeutiquement efficace d'une protéine alpha1-antitrypsine (AAT), le variant, l'isoforme et/ou le fragment correspondant tels que définis dans l'une quelconque des revendications 1 à 10, ledit procédé comprenant les étapes de :
a) détermination du niveau d'au moins l'un du groupe comprenant l'alpha1-antitrypsine endogène, au moins une protéase d'amorçage de protéine spike, un récepteur ACE2 et l'interféron gamma dans un échantillon du sujet d'intérêt avant l'infection par le virus ou pendant l'infection par le virus,
b) détermination du niveau d'au moins l'un du groupe comprenant l'alpha1-antitrypsine endogène, au moins une protéase d'amorçage de protéine spike, un récepteur ACE2 et l'interféron gamma dans un échantillon d'au moins un sujet de référence pendant une infection par le coronavirus, le sujet de référence étant asymptomatique ou présentant des symptômes légers,
c) comparaison du niveau d'intérêt déterminé à l'étape a) avec le niveau de référence déterminé à l'étape b),
dans lequel le sujet d'intérêt est plus susceptible pour un traitement et/ou une prévention de l'infection par le virus si le sujet d'intérêt a au moins l'un choisi dans le groupe constitué par :
1. un niveau d'intérêt plus bas d'alpha-antitrypsine endogène (AAT) par rapport au niveau de référence d'AAT endogène,
2. un niveau d'intérêt plus élevé d'au moins une protéase d'amorçage de protéine spike par rapport au niveau de référence d'au moins une protéase d'amorçage de protéine spike,
3. un niveau d'intérêt plus élevé d'enzyme 2 de conversion de l'angiotensine (récepteur ACE2) par rapport au niveau de référence du récepteur ACE2 et
4. un niveau d'intérêt plus élevé d'interféron gamma (IFN-γ) par rapport au niveau de référence de l'IFN-γ.

12. Procédé de détermination de la quantité thérapeutiquement efficace d'alpha1-antitrypsine (AAT) pour un traitement et/ou une prévention efficace d'une maladie ou d'un syndrome causé(e) par une infection par le coronavirus au moyen de la composition selon l'une quelconque des revendications 1 à 10, ledit procédé comprenant les étapes de :
a) détermination du niveau d'alpha1-antitrypsine endogène dans un échantillon d'un sujet d'intérêt avant l'infection par le virus ou pendant l'infection par le virus,
b) la détermination de la quantité d'AAT dans la composition, nécessaire pour atteindre un niveau d'AAT chez le sujet d'au moins 10 µM, de préférence d'au moins 20 µM, plus préférablement d'au moins 50 µM, encore plus préférablement d'au moins 100 µM, et le plus préférablement d'au moins 200 µM.

13. Composition pour utilisation selon l'une quelconque des revendications 1 à 10, ou procédé selon la revendication 11 ou 12, dans laquelle/lequel le coronavirus est choisi dans le groupe constitué par MERS-CoV, SARS-CoV-1, SARS-CoV-2, coronavirus alpha 299E, coronavirus alpha NL63, coronavirus bêta OC43 et coronavirus bêta HKU1 ; de préférence, le coronavirus est choisi dans le groupe constitué par MERS-CoV, SARS-CoV-1 et SARS-CoV-2, et plus préférablement le virus est SARS-CoV-2.

14. Composition pour utilisation selon l'une quelconque des revendications 1 à 10, ou procédé selon les revendications 11 à 13, dans laquelle/lequel la maladie ou le syndrome est un syndrome respiratoire ou un syndrome respiratoire aigu grave ou la maladie ou le syndrome est une maladie inflammatoire ou un syndrome du système nerveux.
